# EUROPEAN PATENT APPLICATION

(11) **EP 2 727 942 A1**
(43) Date of publication of application: **07.05.2014**
(21) Application number: 12007505.6
(22) Date of filing: 05.11.2012
(51) Int. Cl.: C07K 16/28, C07K 16/32, C07K 16/00

(54) **Bispecific antibodies against human EGFR, HER2, and HER3**

(71) Applicant: MAB Discovery GmbH, 82061 Neuried (DE)
(72) Inventor: Fischer, Stephan, 82362 Weilheim (DE); Renninger, Stephanie, 81379 München (DE)
(74) Representative: Schreiner, Siegfried

(57) **Abstract**

A monoclonal antibody, characterized in specifically binding to human EGFR and HER2, to EGFR and HER3 or to HER2 and HER3 is useful for the manufacture of a pharmaceutical composition and for the treatment of breast, colon, lung, or pancreatic cancer.

## Description

The present invention relates to a method for the production of bispecific antibodies against EGFR and HER2, against EGFR and HER3 or against HER2 and HER3, such antibodies, compositions and uses thereof.

### Background of the invention

Human Epidermal growth factor receptor (EGFR, HER1), its sequence and functions are described by UniProt P00533 (EC=2.7.10.1), human HER2, its sequence and functions are described by UniProt P04626 (Receptor tyrosine-protein kinase erbB-2, EC=2.7.10.1), human HER3, its sequence and functions are described by UniProt P21860 (Receptor tyrosine-protein kinase erbB-3, EC=2.7.10.1). Fendry BM. et al., Cancer Res. 50 (1990 1550-1558 generated by immunization in mice with EGFR an anti-EGFR antibody which moderately binds also to p185^{HER2}. Bostrom J. et al., Science 323 (2009) 1610-1614 generated a repertoire of Herceptin^{®} antibody variants with mutations in the light chain (LC). Fabs thereof were generated which bind also VEGF. Bostrom J. et al. PLOS & (2011) e17887 discusses the thermodynamic background of mulitispecificity of antibodies. Schaefer G. et al., Cancer Cell 20 (2011) 472 generated an antibody binding to HER3/EGFR by phage display. WO9964461 relates to bispecific antibodies against Apo2 and DR4. Immunization of mice was performed with mixed antigens. WO9939729 relates to the use of an HER2, HER3 and/or HER4 activating ligand which is a heregulin (HRG) polypeptide. W02008027236 relates to a method of making a bispecific antibody against VEGF and HER2 by diversifying VL. WO2007146959 relates to dimeric and oligomeric structures of HER extracellular domains and HER/Fc molecules. W02008140493 relates to anti-EGFR family member antibodies and heterogenic bispecific antibodies comprising anti-EGFR family member antibodies. WO2010129304 relates to methods for making heterogenic molecules, such as bispecific antibodies. WO2010108127 relates to a heterogenic bispecific antibody comprising an antigen-binding domain that specifically binds to EGFR and HER3 wherein the toxicity of the antibody is less than the toxicity of an EGFR antagonist. W02011056997 relates to a method of affinity maturation of an antibody. W02011060206 relates to an antibody against HER-3 and a combination thereof with other antibodies of the HER family. WO2011140254 relates to a combination

### Method for the production of bispecific antibodies against human EGFR, HER2, and HER3

The present invention relates to a method for the production of bispecific antibodies against EGFR and HER2, against EGFR and HER3 or against HER2 and HER3, such antibodies, compositions and uses thereof.

### Background of the invention

Human Epidermal growth factor receptor (EGFR, HER1), its sequence and functions are described by UniProt P00533 (EC=2.7.10.1), human HER2, its sequence and functions are described by UniProt P04626 (Receptor tyrosine-protein kinase erbB-2, EC=2.7.10.1), human HER3, its sequence and functions are described by UniProt P21860 (Receptor tyrosine-protein kinase erbB-3, EC=2.7.10.1). Fendry BM. et al., Cancer Res. 50 (1990 1550-1558 generated by immunization in mice with EGFR an anti-EGFR antibody which moderately binds also to p185^{HER2}. Bostrom J. et al., Science 323 (2009) 1610-1614 generated a repertoire of Herceptin^{®} antibody variants with mutations in the light chain (LC). Fabs thereof were generated which bind also VEGF. Bostrom J. et al. PLOS & (2011) e17887 discusses the thermodynamic background of mulitispecificity of antibodies. Schaefer G. et al., Cancer Cell 20 (2011) 472 generated an antibody binding to HER3/EGFR by phage display. W09964461 relates to bispecific antibodies against Apo2 and DR4. Immunization of mice was performed with mixed antigens. WO9939729 relates to the use of an HER2, HER3 and/or HER4 activating ligand which is a heregulin (HRG) polypeptide. WO2008027236 relates to a method of making a bispecific antibody against VEGF and HER2 by diversifying VL. WO2007146959 relates to dimeric and oligomeric structures of HER extracellular domains and HER/Fc molecules. WO2008140493 relates to anti-EGFR family member antibodies and heterogenic bispecific antibodies comprising anti-EGFR family member antibodies. WO2010129304 relates to methods for making heterogenic molecules, such as bispecific antibodies. WO2010108127 relates to a heterogenic bispecific antibody comprising an antigen-binding domain that specifically binds to EGFR and HER3 wherein the toxicity of the antibody is less than the toxicity of an EGFR antagonist. WO2011056997 relates to a method of affinity maturation of an antibody. WO2011060206 relates to an antibody against HER-3 and a combination thereof with other antibodies of the HER family. W02011140254 relates to a combination of anti-EGFR antibodies. WO2012007167 relates to a heterogenic bispecific antibody against HER2 and LewisY.

### Summary of the Invention

The invention relates to a method for the production of monoclonal bispecific antibodies against human EGFR and HER2, against EGFR and HER3 or against HER2 and HER3 and such antibodies. EGFR, HER2 and HER3 are therapeutic targets and the invention provides also compositions, B cells, methods of use, and methods of production of the antibodies according to the invention.

The invention provides a method for the production of a bispecific antibody specifically binding to EGFR and HER2, to EGFR and HER3 or to HER2 and HER3, characterized in performing in the order specified the steps of
i) immunizing a rabbit with the three antigens EGFR, HER2 and HER3 or the two antigens EGFR and HER2, EGFR and HER3, or HER2 and HER3, respectively,
ii) isolating B cells from said rabbit,
iii) isolating B cells from said B cells isolated in step ii) which bind to one or two antigens used in step i) for immunization,
iv) isolating single B cells from said B cells isolated in step iii),
v) selecting one of said single B cells which comprises mRNA encoding a polypeptide comprising a VH region and/or a polypeptide comprising a VL region of an antibody which binds specifically to EGFR and HER2, or
   selecting one of said single B cells which comprises mRNA encoding a polypeptide comprising a VH region and/or a polypeptide comprising a VL region of an antibody which binds specifically to EGFR and HER3 , or
   selecting one of said single B cells which comprises mRNA encoding a polypeptide comprising a VH region and/or a polypeptide comprising a VL region of an antibody which binds specifically to HER2 and HER3, and
vi) producing the antibody expressed by said selected B cell.

Preferably the EGFR, HER2, and/or HER3 antigen used for immunization is/are fusion polypeptides consisting of said antigen and a human Fc polypeptide. Preferably in step i) CFA is used as adjuvant. Preferably in step i) CFA and IFA are used together as adjuvants. Preferably in step ii) B cells are isolated from the blood of the rabbit. B cells are isolated preferably as PBMCs and depleted from macrophages. The antigens used for isolating B cells in step iii) can be the EGFR, HER2, and/or HER3 proteins, fragments thereof, preferably the extracellular domain or parts thereof, cells presenting the antigens on their surface or the like.

Preferably in step iv) single B cells, secreting immunoglobulin, preferably IgG, are separated, preferably by FACS. Preferably the single B cell is then treated with a feeder cell before performing step v). In step v) one of said antibody producing single rabbit B cells is selected.

### Antibodies against HER2 and HER3

Preferably in step v) a single B cell is selected which comprises mRNA encoding a VH region of an antibody specifically binding to HER2 and HER3, which is at least 90% identical to a VH region of SEQ ID NO:1+n and mRNA encoding a VL region of an antibody specifically binding to HER2 and HER3, which is at least 90% identical to a VL region of SEQ ID NO:3+n, wherein n is a number selected from the group of 0 and 1.

Preferably the method according to the invention is characterized in selecting in step v) a single B cell which comprises mRNA encoding a VH region of an antibody specifically binding to HER2 and HER3 of SEQ ID NO:1+n and mRNA encoding a VL region of an antibody specifically binding to HER2 and HER3 of SEQ ID NO:3+n, wherein n is a number selected from the group of 0 and 1.

The invention comprises a monoclonal antibody, characterized in specifically binding to human HER2 and HER3. Preferably the antibody is characterized in being a monoclonal rabbit antibody.

The heavy chain variable (VH) region of an antibody specifically binding to HER2 and HER3 is preferably characterized in that said VH region is at least 90% identical to a VH region selected from the group consisting of VH regions of SEQ ID NO:1 and 2.

The light chain variable (VL) region of an antibody specifically binding to HER2 and HER3 is preferably characterized in that said VL region is at least 90% identical to a VL region selected from the group consisting of VL regions of SEQ ID NO:3 and 4.

The antibody according to the invention is preferably characterized in that its VH region is at least 90% identical to a VH region of SEQ ID NO:1+n and its VL region is at least 90% identical to a VL region of SEQ ID NO:3+n, wherein n is a number selected from the group consisting of 0 and 1.

The heavy chain variable (VH) region of an antibody according to the invention is preferably characterized in that said VH region is selected from the group consisting of VH regions of SEQ ID NO:1 and 2.

The light chain variable (VL) region of an antibody according to the invention is preferably characterized in that said VL region is selected from the group consisting of VL regions of SEQ ID NO:3 and 4.

The antibody according to the invention is preferably characterized in that its VH region is selected from the group consisting of VH regions of SEQ ID NO:1+n and its VL region is selected from the group consisting of VL regions of SEQ ID NO:3+n, wherein n is a number selected from the group consisting of 0 and 1. Antibody 47 is characterized by said variable region sequences wherein n=0 and antibody 87 by n=1.

The antibody according to the invention is preferably characterized in comprising a VH region and a VL region comprising the respective CDR1, CDR2 and CDR3 regions of antibody 47 or 87.

The antibody according to the invention is preferably characterized in that the antibody comprises a VH region selected from the group of VH regions comprising a CDR1H region of SEQ ID NO:5+n, a CDR2H region of SEQ ID NO:7+n and aCDR3H region of SEQ ID NO:9+n, wherein n is a number selected from the group consisting of 0 and 1.

The antibody according to the invention is preferably characterized in that the antibody comprises a VL region selected from the group of VL regions comprising a CDR1L region of SEQ ID NO:11+n, a CDR2L region of SEQ ID NO:13+n and aCDR3L region of SEQ ID NO:15+n, wherein n is a number selected from the group consisting of 0 and 1.

The antibody according to the invention is preferably characterized in that the antibody comprises a VH region selected from the group of VH regions comprising a CDR1H region of SEQ ID NO:5+n, a CDR2H region of SEQ ID NO:7+n and aCDR3H region of SEQ ID NO:9+n and in that the antibody comprises a VL region selected from the group of VL regions comprising a CDR1L region of SEQ ID NO:11+n, a CDR2L region of SEQ ID NO:13+n and aCDR3L region of SEQ ID NO:15+n, wherein n is a number selected from the group consisting of 0 and 1.

### Antibodies against EGFR and HER2

Preferably the method according to the invention is characterized in selecting in step v) a single B cell is selected which comprises mRNA encoding a VH region of an antibody specifically binding to EGFR and HER2, which is at least 90% identical to a VH region of SEQ ID NO:17+n and mRNA encoding a VL region of an antibody specifically binding to EGFR and HER2, which is at least 90% identical to a VL region of SEQ ID NO:33+n, wherein n is a number selected from the group of 0, 1, 2, 3, 4, 5, 6, 7, 8, 9, 10, 11, 12, 13, 14, and 15.

Preferably the method according to the invention is characterized in selecting in step v) a single B cell which comprises mRNA encoding a VH region of an antibody specifically binding to EGFR and HER2 of SEQ ID NO:17+n and mRNA encoding a VL region of an antibody specifically binding to EGFR and HER2 of SEQ ID NO:33+n, wherein n is a number selected from the group of 0, 1, 2, 3, 4, 5, 6, 7, 8, 9, 10, 11, 12, 13, 14, and 15.

The invention comprises a monoclonal antibody, characterized in specifically binding to human EGFR and HER2. Preferably the antibody is a monoclonal rabbit antibody. Preferably the antibody binds to EGFR and/or HER2 at an antibody concentration of 300 µmol/l or more with an OD value of 1.0 or more, preferably 1.5 or more, preferably 2.0.

The VH region of an antibody specifically binding to EGFR and HER2 is preferably characterized in that said VH region is at least 90% identical to a VH region selected from the group consisting of VH regions of SEQ ID NO:17+n, wherein n is a number selected from the group of 0, 1, 2, 3, 4, 5, 6, 7, 8, 9, 10, 11, 12, 13, 14, and 15.

The VL region of an antibody specifically binding to EGFR and HER2 is preferably characterized in that said VL region is at least 90% identical to a VL region selected from the group consisting of VL regions of SEQ ID NO:33+n, wherein n is a number selected from the group of 0, 1, 2, 3, 4, 5, 6, 7, 8, 9,10,11,12,13,14, and 15.

The antibody according to the invention is preferably characterized in that its VH region is at least 90% identical to a VH region of SEQ ID NO:17+n and its VL region is at least 90% identical to a VL region of SEQ ID NO:33+n, wherein n is a number selected from the group of 0, 1, 2, 3, 4, 5, 6, 7, 8, 9, 10, 11, 12, 13, 14, and 15.

The VH region of an antibody according to the invention is preferably characterized in that said VH region is selected from the group consisting of VH regions of SEQ ID NO:17+n, wherein n is a number selected from the group of 0, 1, 2, 3, 4, 5, 6, 7, 8, 9, 10, 11, 12, 13, 14, and 15.

The light chain variable (VL) region of an antibody according to the invention is preferably characterized in that said VL region is selected from the group consisting of VL regions of SEQ ID NO:33+n, wherein n is a number selected from the group of 0, 1, 2, 3, 4, 5, 6, 7, 8, 9, 10, 11, 12, 13, 14, and 15.

The antibody according to the invention is preferably characterized in that its VH region is selected from the group consisting of VH regions of SEQ ID NO:17+n and its VL region is selected from the group consisting of VL regions of SEQ ID NO:33+n, wherein n is a number selected from the group of 0, 1, 2, 3, 4, 5, 6, 7, 8, 9, 10, 11, 12, 13, 14, and 15. Antibody 1 is characterized by said variable region sequences wherein n=0, antibody 6 by n=1, antibody 9 by n=2, antibody 15 by n=3, antibody 18 by n=4, antibody 20 by n=5, antibody 21 by n=6, antibody 34 by n=7, antibody 45 by n=8, antibody 68 by n=9, antibody 72 by n=10, antibody 74 by n=11, antibody 77 by n=12 antibody 78 by n=13, antibody 79 by n=14, and antibody 88 by n=15.

The antibody according to the invention is preferably characterized in comprising a VH region and a VL region comprising the respective CDR1, CDR2 and CDR3 regions of an antibody selected of the group consisting of antibodies 1, 6, 9, 15, 18, 20, 21, 34, 45, 68, 72, 74, 77, 78, 79, and 88.

The antibody according to the invention is preferably characterized in that the antibody comprises a VH region selected from the group of VH regions comprising a CDR1H region of SEQ ID NO:49+n, a CDR2H region of SEQ ID NO:65+n and aCDR3H region of SEQ ID NO:81+n, wherein n is a number selected from the group of 0, 1, 2, 3, 4, 5, 6, 7, 8, 9, 10, 11, 12, 13, 14, and 15.

The antibody according to the invention is preferably characterized in that the antibody comprises a VL region selected from the group of VL regions comprising a CDR1L region of SEQ ID NO:97+n, a CDR2L region of SEQ ID NO:113+n and aCDR3L region of SEQ ID NO:129+n, wherein n is a number selected from the group consisting of 0, 1, 2, and 3.

The antibody according to the invention is preferably characterized in that the antibody comprises a VH region selected from the group of VH regions comprising a CDR1H region of SEQ ID NO:49+n, a CDR2H region of SEQ ID NO:65+n and aCDR3H region of SEQ ID NO:81+n and in that the antibody comprises a VL region selected from the group of VL regions comprising a CDR1L region of SEQ ID NO:97+n, a CDR2L region of SEQ ID NO:113+n and aCDR3L region of SEQ ID NO:129+n, wherein n is a number selected from the group of 0, 1, 2, 3, 4, 5, 6, 7, 8, 9, 10, 11, 12, 13, 14, and 15.

### Antibodies against EGFR and HER3

Preferably the method according to the invention is characterized in selecting in step v) a single B cell is selected which comprises mRNA encoding a VH region of an antibody specifically binding to EGFR and HER3, which is at least 90% identical to a VH region of SEQ ID NO:145+n and mRNA encoding a VL region of an antibody specifically binding to EGFR and HER3, which is at least 90% identical to a VL region of SEQ ID NO:161+n, wherein n is a number selected from the group of 0, 1, 2, 3, 4, 5, 6, 7, 8, 9, 10, 11, 12, 13, 14, and 15.

Preferably the method according to the invention is characterized in selecting in step v) a single B cell which comprises mRNA encoding a VH region of an antibody specifically binding to EGFR and HER3 of SEQ ID NO:145+n and mRNA encoding a VL region of an antibody specifically binding to EGFR and HER3 of SEQ ID NO:161+n, wherein n is a number selected from the group of 0, 1, 2, 3, 4, 5, 6, 7, 8, 9, 10, 11, 12, 13, 14, and 15.

The invention comprises a monoclonal antibody, characterized in specifically binding to human EGFR and HER3. Preferably the antibody is a monoclonal rabbit antibody.

The VH region of an antibody specifically binding to EGFR and HER3 is preferably characterized in that said VH region is at least 90% identical to a VH region selected from the group consisting of VH regions of SEQ ID NO:145+n, wherein n is a number selected from the group of 0, 1, 2, 3, 4, 5, 6, 7, 8, 9, 10, 11, 12, 13, 14, and 15..

The light chain variable (VL) region of an antibody specifically binding to EGFR and HER3 is preferably characterized in that said VL region is at least 90% identical to a VL region selected from the group consisting of VL regions of SEQ ID NO:161+n, wherein n is a number selected from the group of 0, 1, 2, 3, 4, 5, 6, 7, 8, 9, 10, 11, 12, 13, 14, and 15..

The antibody according to the invention is preferably characterized in that its VH region is at least 90% identical to a VH region of SEQ ID NO:145+n and its VL region is at least 90% identical to a VL region of SEQ ID NO:161+n, wherein n is a number selected from the group of 0, 1, 2, 3, 4, 5, 6, 7, 8, 9, 10, 11, 12, 13, 14, and 15.

The VH region of an antibody according to the invention is preferably characterized in that said VH region is selected from the group consisting of VH regions of SEQ ID NO:145+n, wherein n is a number selected from the group of 0, 1, 2, 3, 4, 5, 6, 7, 8, 9, 10, 11, 12, 13, 14, and 15.

The light chain variable (VL) region of an antibody according to the invention is preferably characterized in that said VL region is selected from the group consisting of VL regions of SEQ ID NO:161+n, wherein n is a number selected from the group of 0, 1, 2, 3, 4, 5, 6, 7, 8, 9, 10, 11, 12, 13, 14, and 15.

The antibody according to the invention is preferably characterized in that its VH region is selected from the group consisting of VH regions of SEQ ID NO:145+n and its VL region is selected from the group consisting of VL regions of SEQ ID NO:161+n, wherein n is a number selected from the group of 0, 1, 2, 3, 4, 5, 6, 7, 8, 9, 10, 11, 12, 13, 14, and 15.. Antibody 11 is characterized by said variable region sequences wherein n=0, antibody 22 by n=1, antibody 23 by n=2, antibody 24 by n=3, antibody 28 by n=4, antibody 32 by n=5, antibody 52 by n=6, antibody 53 by n=7, antibody 55 by n=8, antibody 56 by n=9, antibody 60 by n=10, antibody 62 by n=11, antibody 64 by n=12, antibody 69 by n=13 antibody 71 by n=14, and antibody 75 by n=15.

The antibody according to the invention is preferably characterized in comprising a VH region and a VL region comprising the respective CDR1, CDR2 and CDR3 regions of an antibody selected of the group consisting of antibodies 11, 22, 23, 24, 28, 32, 52, 53, 55, 56, 60, 62, 64, 69, 71, and 75.

The antibody according to the invention is preferably characterized in that the antibody comprises a VH region selected from the group of VH regions comprising a CDR1H region of SEQ ID NO:177+n, a CDR2H region of SEQ ID NO:193+n and aCDR3H region of SEQ ID NO:209+n, wherein n is a number selected from the group of 0, 1, 2, 3, 4, 5, 6, 7, 8, 9, 10, 11, 12, 13, 14, and 15.

The antibody according to the invention is preferably characterized in that the antibody comprises a VL region selected from the group of VL regions comprising a CDR1L region of SEQ ID NO:225+n, a CDR2L region of SEQ ID NO:241+n and aCDR3L region of SEQ ID NO:257+n, wherein n is a number selected from the group of 0, 1, 2, 3, 4, 5, 6, 7, 8, 9, 10, 11, 12, 13, 14, and 15.

The antibody according to the invention is preferably characterized in that the antibody comprises a VH region selected from the group of VH regions comprising a CDR1H region of SEQ ID NO:177+n, a CDR2H region of SEQ ID NO:193+n and aCDR3H region of SEQ ID NO:209+n and in that the antibody comprises a VL region selected from the group of VL regions comprising a CDR1L region of SEQ ID NO:225+n, a CDR2L region of SEQ ID NO:241+n and aCDR3L region of SEQ ID NO:257+n, wherein n is a number selected from the group of 0, 1, 2, 3, 4, 5, 6, 7, 8, 9, 10, 11, 12, 13, 14, and 15.

The invention also comprises a rabbit B cell, characterized in comprising mRNA encoding a polypeptide comprising a VH region and/or a polypeptide comprising a VL region of an antibody according to the invention. The invention also comprises a method for the production of an antibody according to the invention, characterized in isolating from a rabbit B cell comprising mRNA encoding a polypeptide comprising a VH region and/or a polypeptide comprising a VL region of an antibody according to the invention an antibody and selecting said antibody if it binds specifically to EGFR and HER2, to EGFR and HER3 or to HER2 and HER3.

Preferably the anti-EGFR and HER2, EGFR and HER3 or HER2 and HER3 antibodies of the present invention are antagonistic antibodies.

The antibody according to the invention is preferably characterized in being a humanized or chimeric version of said antibody. Preferably, the antibody according to the invention is an antibody comprising antigen binding sequences from a rabbit donor grafted to a heterologous non-human, human, or humanized sequence (e.g., framework and/or constant domain sequences). Preferably an antibody of the invention has rabbit V regions or rabbit CDR regions and a human C region and/or framework. Preferably, the rabbit light chain V region or a human framework region comprising rabbit light chain CDRs is fused to a human kappa light chain constant region. Preferably, the rabbit heavy chain V region or a human framework region comprising rabbit heavy chain CDRs is fused to a human constant region, preferably IgG1.

The invention also provides a pharmaceutical composition characterized by comprising an antibody according to the invention. The invention also provides the use of an antibody according to the invention for the manufacture of a pharmaceutical composition. The invention also provides an antibody according to the invention for the treatment of cancer. The invention also provides an antibody according to the invention for the treatment of breast, colon, lung, or pancreatic cancer. The invention also provides the use of an antibody according to the invention for manufacture of a medicament for the treatment of cancer. The invention also provides the use of an antibody according to the invention for manufacture of a medicament for the treatment of breast, colon, lung, or pancreatic cancer. The invention also provides an antibody according to the invention for use in the treatment of cancer, preferably in the treatment breast, colon, lung, or pancreatic cancer.

The invention also provides a nucleic acid encoding an antibody according to the invention. The invention also provides an expression vector characterized in comprising a nucleic acid according to the invention for the expression of an antibody according to the invention in a prokaryotic or eukaryotic host cell. The invention also provides a prokaryotic or eukaryotic host cell comprising a nucleic acid according to the invention. The invention also provides a method of producing an antibody that specifically binds to EGFR and HER2, to EGFR and HER3 or to HER2 and HER3 characterized by expressing a nucleic acid according to the invention in a prokaryotic or eukaryotic host cell and recovering said antibody from said cell or the cell culture supernatant.

The invention also provides a method for inhibiting EGFR and/or HER2 in a cell expressing EGFR and/or HER2, comprising contacting the cell with an antibody specifically binding to EGFR and HER2 according to the invention. The invention also provides a method for inhibiting EGFR and/or HER3 activity in a cell expressing EGFR and/or HER3, comprising contacting the cell with an antibody specifically binding to EGFR and HER3 according to the invention. The invention also provides a method for inhibiting HER2 and/or HER3 activity in a cell expressing HER2 and/or HER3, comprising contacting the cell with an antibody specifically binding to HER2 and HER3 according to the invention.

### Detailed Description of the Invention

The term "rabbit" according to the invention means animals of the family Leporidae, preferably of genus Oryctolagus.

The term "antibody" encompasses the various forms of antibody structures including, but not being limited to, whole antibodies and antibody fragments. The antibody according to the invention is in its primary form produced by a B-cell of a rabbit and binds in this form specifically to human EGFR and HER2, to EGFR and HER3, or to HER2 and HER3. Therefore the antibody according to the invention binds specifically to human EGFR and HER2, to EGFR and HER3 or to HER2 and HER3 based on its antigen-binding portion, preferably its VH region comprising three VH CDRs and/or its VL region comprising three VL CDRs. The term "VL (or VH) region" has the same meaning as VL (or VH) domain. The antibody according to the invention is in its primary form a mature antibody, which may be different from a simple germline antibody. Without being bound by theory, it is believed that binding of the antigen to a germline antibody might lead to significant structural rearrangements, whereas the unbound state of a matured antibody might be closer to its bond state. Therefore the mature form of the antibody has probably a more rigid structure than the germline form. The germline antibody might be therefore more conformational flexible, resulting in a slower binding rate (see e.g. Wedemayer GJ et al., Science. 1997 Jun 13; 276 (5319):1665-9; Structural insights into the evolution of an antibody combining site). The presumably lower flexible structure of the mature antibody may improve the physicochemical properties of the antibody according to the invention, as being e.g. solubility or low aggregation, leading to improved therapeutic properties. The antibody according to the invention as identified from a rabbit B cell is an antibody having variable regions of natural origin. "Natural origin" means according to the invention, that such an antibody has variable regions which are identical in their amino acid sequences to the sequences of variable regions naturally occurring in rabbits. The antibody according to the invention can be further modified and is preferably a rabbit antibody, a humanized antibody, a chimeric antibody, a fragment thereof, or a further genetically engineered and recombinant produced antibody as long as the characteristic properties according to the invention are retained. The antibody can be bound to a further agent, e.g. as being an immunoconjugate.

The term "rabbit monoclonal antibody "means a monoclonal antibody produced by immunizing a rabbit and isolated from a B cell of said rabbit. Preferably the antibody according to the invention is characterized in that it comprises at the C terminal end of the VL region of SEQ NO.: 273, 274, 275, 276 or 277 and/or at the C terminal end of the VL region SEQ ID NO:278, 279 or 280.

An "immunoconjugate" means an antibody conjugated to one or more cytotoxic agents, such as a chemotherapeutic agent, a drug, a growth inhibitory agent, a toxin, another antibody or a radioactive isotope.

"Antibody fragments" comprise a portion of a full length antibody, preferably the variable regions thereof, or at least the antigen binding site thereof. Examples of antibody fragments include diabodies, Fab fragments, and single-chain antibody molecules. scFv antibodies are, e.g., described in Huston, J.S., Methods in Enzymol. 203 (1991) 46-88.

The terms "monoclonal antibody" or "monoclonal antibody composition" as used herein refer to a preparation of antibody molecules of a single amino acid composition. The term "chimeric antibody" refers to a monoclonal antibody comprising a variable region, i.e., binding region, from rabbit and at least a portion of a constant region derived from a different source or species, usually prepared by recombinant DNA techniques. According to the invention chimeric antibodies comprising a rabbit variable region and a human constant region and humanized rabbit antibodies are especially preferred. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art (see, e.g., Morrison, S.L., et al, Proc. Natl. Acad Sci. USA 81 (1984) 6851-6855; US 5,202,238 and US 5,204,244).

The term "humanized antibody" or "humanized version of an antibody" refers to antibodies in which a human variable region has been modified to comprise the CDRs of an antibody according to the invention. In a preferred embodiment, the CDRs of the VH and VL are grafted into the framework region of human antibody to prepare the "humanized antibody." See e.g. Riechmann, L., et al, Nature 332 (1988) 323-327; and Neuberger, M.S., et al, Nature 314 (1985) 268-270. The heavy and light chain variable framework regions can be derived from the same or different human antibody sequences. The human antibody sequences can be the sequences of naturally occurring human antibodies. Human heavy and light chain variable framework regions are listed e.g. in Lefranc, M.-P., Current Protocols in Immunology (2000) - Appendix IP A.1P.1-A.1P.37 and are accessible via IMGT, the international ImMunoGeneTics information system^{®} (http://imgt.cines.fr) or via http://vbase.mrc-cpe.cam.ac.uk.

The term "recombinant antibody", as used herein, is intended to include all antibodies according to the invention that are prepared by recombinant means, such as antibodies from a host cell such as a NS0 or CHO cell using a recombinant expression vector transfected into a host cell. Such recombinant human antibodies have variable and constant regions in a rearranged form.

The term "antibody specifically binding to human EGFR and HER2", as used herein, refer to an antibody which binds specifically to human EGFR and HER2, but not to HER3. The term "antibody specifically binding to human EGFR and HER3", as used herein, refer to an antibody which binds specifically to human EGFR and HER3, but not to HER2. The term "antibody specifically binding to human HER2 and HER3", as used herein, refer to an antibody which binds specifically to human HER2 and HER3, but not to EGFR.

The term "specifically binding ", as used herein, refer to binding of the antibody to the respective antigen, measured by ELISA, wherein said ELISA comprises coating EGFR, HER2, and HER3 protein respectively to a solid support, adding said antibody under conditions to allow the formation of an immune complex with the respective HER protein, detecting said immune complex by measuring the Optical Density values (OD) using a secondary antibody binding to an antibody according to the invention and using a peroxidase-mediated color development. Specific binding is found if at an antibody concentration of 300 µmol/l or more an OD value of 0.3 or more, preferably 1.0 or more, preferably 1.5 or more, preferably 2.0 or more (measured against same solvent without antibody at 450nm) is found. Preferably the antibody against EGFR and HER2 according to the invention binds to EGFR and HER2 in a ratio of 1:10 to 10:1, the antibody against EGFR and HER3 according to the invention binds to EGFR and HER3 in a ratio of 1:10 to 10:1 and the antibody against HER2 and HER3 according to the invention binds to HER2 and HER3 in a ratio of 1:10 to 10:1 measured by ELISA wherein said ELISA comprises: coating EGFR, HER2 and HER3 protein respectively to a solid support, adding said antibody under conditions to allow the formation of an immune complex with the respective HER protein, detecting said immune complex by measuring the Optical Density using a secondary antibody binding to said antibody and a peroxidase-mediated color development, and calculating said binding ratios using said Optical Density values. The antibodies according to the invention do not bind specifically to other human antigens like human Fc, human IL12R, human HER4, HGFR, Notch-1, CD44, IGF-1R, P-Cadherin, EpoR, or DLL4. Therefore for these and other such human antigens OD values will be 0.1 or lower (e.g. 0.0).

The antibody according to the invention comprises a VH region and a VL region or parts thereof, which are together sufficient for the specific binding to EGFR, HER2, and/or HER3 according to the invention. Therefore the antibody according to the invention is different to antibodies which are sometimes called "bispecific, multispecific etc. antibodies" in the state of the art and which comprise according to the state of the art at least a variable heavy and a variable light chain region from a first antibody and a variable heavy and a variable light chain region from a second antibody wherein each of those antibodies binds to a different antigen or epitope or which comprise several single-domains of nanobodies (such antibodies of the state of the art are described e.g. by Kontermann R., MAbs. 2012 Mar 1;4(2); and Caravella J, Lugovskoy, A.Curr Opin Chem Biol. 2010 Aug;14(4):520-8).

The terms "EGFR, HER2, and HER3" or "antigens" or "HER proteins", as used herein, refer to Human Epidermal growth factor receptor (EGFR, HER1), its sequence and functions are described by UniProt P00533 (EC=2.7.10.1), human HER2, its sequence and functions are described by UniProt P04626 (Receptor tyrosine-protein kinase erbB-2, EC=2.7.10.1), and human HER3, its sequence and functions are described by UniProt P21860 (Receptor tyrosine-protein kinase erbB-3, EC=2.7.10.1).

The "variable domain (or region) of an antibody according to the invention" (variable domain of a light chain (VL), variable domain of a heavy chain (VH)) as used herein denotes each of the pair of light and heavy chain domains which are involved directly in binding the antibody to the antigen. The variable light and heavy chain domains have the same general structure and each domain comprises four framework (FR) regions whose sequences are widely conserved, connected by three complementary determining regions, CDRs. The term "antigen-binding portion of an antibody" when used herein refer to the amino acid residues of an antibody which are responsible for antigen-binding. The antigen-binding portion of an antibody comprises preferably amino acid residues from the "complementary determining regions" or "CDRs". The CDR sequences are defined according to Kabat et al, Sequences of Proteins of Immunological Interest, 5th Ed. Public Health Service, National Institutes of Health, Bethesda, Md. (1991). Using this numbering system, the actual linear amino acid sequence may contain fewer or additional amino acids corresponding to a shortening of, or insertion into, a FR or CDR of the variable domain. For example, a heavy chain variable domain may include a single amino acid insert (residue 52a according to Kabat) after residue 52 of H2 and inserted residues (e.g. residues 82a, 82b, and 82c, etc. according to Kabat) after heavy chain FR residue 82. The CDR2 sequence of the light chain of an antibody according to the invention preferably consists of the first three amino acids of its CDRL2 sequence SEQ ID NO: 13, 14, 113 to 128 or 241 to 256, lacking one amino acid at the C-terminus. For example CDR2 sequence (SEQ ID NO:241) of the light chain of antibody 11 is lacking amino acid "T". The Kabat numbering of residues may be determined for a given antibody by alignment at regions of homology of the sequence of the antibody with a "standard" Kabat numbered sequence. The variable domain of the heavy chain of an antibody according to the invention is composed of a single immunoglobulin domain and is about 110 to 120 amino acids long. The variable domain of the light chain of an antibody according to the invention is composed of a single immunoglobulin domain and is about 110 to 120 amino acids long.

In one embodiment the antibody according to the invention comprises a Fc part derived from human origin and preferably all other parts of the human constant regions. As used herein the term "Fc part derived from human origin" denotes a Fc part which is either a Fc part of a human antibody of the subclass IgG1, IgG2, IgG3 or IgG4, e.g. a Fc part from human IgG1 subclass, a mutated Fc part from human IgG1 subclass (preferably with a mutation on L234A + L235A), a Fc part from human IgG4 subclass or a mutated Fc part from human IgG4 subclass (preferably with a mutation on S228P). In one embodiment the antibody according to the invention is of human IgG1 subclass. Human constant chains are well known in the state of the art and e.g. described by Kabat, E.A., (see e.g. Johnson, G. and Wu, T.T., Nucleic Acids Res. 28 (2000) 214-218).

In one embodiment the antibody according to the invention comprises a heavy chain variable region (VH) sequence having at least 90%, 91%, 92%>, 93%>, 94%, 95%, 96%, 97%, 98%, 99%, or 100% sequence identity to an amino acid sequence selected from the group of SEQ ID NO:1+n, wherein n is a number from 0 to 1, selected from the group of SEQ ID NO:17+n, wherein n is a number from 0 to 15 or SEQ ID NO:17+n, or selected from the group of SEQ ID NO:145+n wherein n is a number from 0 to 15. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, whereby the antibody retains the ability to bind specifically according to the invention to EGFR and HER2, to EGFR and HER3 or to HER2 and HER3. In certain embodiments, a total of 1 to 10 amino acids have been substituted, inserted and/or deleted in each of such sequence. In certain embodiments, substitutions, insertions, or deletions occur in regions outside the CDRs (i.e., in the FRs).

In one embodiment the antibody according to the invention comprises a light chain variable region (VL) having at least 90%, 91%, 92%, 93%, 94%, 95%, 96%o, 97%, 98%, 99%, or 100% sequence identity to the amino acid sequence selected from the group of SEQ ID NO:3+n, wherein n is a number from 0 to 1, selected from the group of SEQ ID NO:33+n, wherein n is a number from 0 to 15 or SEQ ID NO:17+n, or selected from the group of SEQ ID NO:161+n wherein n is a number from 0 to 15. In certain embodiments, a VL sequence having at least 90%, 91%, 92%, 93%, 94%, 95%, 96%, 97%, 98%, or 99%o identity contains substitutions (e.g., conservative substitutions), insertions, or deletions relative to the reference sequence, whereby the antibody retains the ability to bind specifically to EGFR,HER2 and HER3. In certain embodiments, a total of 1 to 10 amino acids have been substituted, inserted and/or deleted in each of such sequence. In certain embodiments, the substitutions, insertions, or deletions occur in regions outside the CDRs (i.e., in the FRs).

Identity or homology with respect to the sequence is defined herein as the percentage of amino acid residues in the candidate sequence that are identical with the parent sequence, after aligning the sequences and introducing gaps, if necessary, to achieve the maximum percent sequence identity. None of N-terminal, C-terminal, or internal extensions, deletions, or insertions into the antibody sequence shall be construed as affecting sequence identity or homology. The variant retains the ability to bind specifically to EGFR and HER2, to EGFR and HER3 or to HER2 and HER3 and preferably has properties, which are superior to those of the parent antibody. For example, the variant may have improved binding to the targets. Exemplary "parent" antibodies comprises the CDR regions of antibodies 47, 1 or 11 and are preferably used for the preparation of such variants. Preferably, the parent antibody has a human framework region and, if present, has human antibody constant domains. For example, the parent antibody may be a humanized antibody. An "affinity matured" antibody is one with one or more alterations in one or more CDRs thereof which result in an improvement in the affinity of the antibody for antigen, compared to a parent antibody which does not possess those alteration(s). Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al., Proc Nat. Acad. Sci, USA 91 :3809-3813 (1994); Schier et al., Gene 169: 147-155 (1995); Yelton et al., J. Immunol. 1 55 : 1994-2004 (1995); Jackson et al., J. Immunol. 1 54(7):3310-9 ( 1995); and Hawkins et al., J. Mol. Biol. 226:889-896 (1992) and WO2010108127. "Percent (%) amino acid sequence identity" with respect to a peptide or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software.

The number "n" according to the invention is meant to be identical for the same antibody, its heavy and light chains and CDR regions. That means that e.g. for antibody 47(n=0) the heavy chain sequence comprises SEQ ID NO:1, the light chain sequence comprises SEQ ID NO: 3, and the heavy chain CDR1H sequence comprises SEQ ID NO:5. Sequence numbers and values of "n" of variable chains and CDRs of antibodies according to the invention are shown in tables 1 to 3.

**Table 1**

| **Antibody HER2/3** | **SEQ ID NO. of antibody region** | | | | | | | | |
|---|---|---|---|---|---|---|---|---|---|
| | **VH** | **VL** | **CDR1H** | **CDR2H** | **CDR3H** | **CDRL1** | **CDRL2** | **CDRL3** | **n** |
| 47 | 1 | 3 | 5 | 7 | 9 | 11 | 13 | 15 | 0 |
| 87 | 2 | 4 | 6 | 8 | 10 | 12 | 14 | 16 | 1 |

**Table 2**

| **Antibody EGFR/HER2** | **SEQ ID NO. of antibody region** | | | | | | | | |
|---|---|---|---|---|---|---|---|---|---|
| | **VH** | **VL** | **CDR1H** | **CDR2H** | **CDR3H** | **CDRL1** | **CDRL2** | **CDRL3** | **n** |
| 1 | 17 | 33 | 49 | 65 | 81 | 97 | 113 | 129 | 0 |
| 6 | 18 | 34 | 50 | 66 | 82 | 98 | 114 | 130 | 1 |
| 9 | 19 | 35 | 51 | 67 | 83 | 99 | 115 | 131 | 2 |
| 15 | 20 | 36 | 52 | 68 | 84 | 100 | 116 | 132 | 3 |
| 18 | 21 | 37 | 53 | 69 | 85 | 101 | 117 | 133 | 4 |
| 20 | 22 | 38 | 54 | 70 | 86 | 102 | 118 | 134 | 5 |
| 21 | 23 | 39 | 55 | 71 | 87 | 103 | 119 | 135 | 6 |
| 34 | 24 | 40 | 56 | 72 | 88 | 104 | 120 | 136 | 7 |
| 45 | 25 | 41 | 57 | 73 | 89 | 105 | 121 | 137 | 8 |
| 68 | 26 | 42 | 58 | 74 | 90 | 106 | 122 | 138 | 9 |
| 72 | 27 | 43 | 59 | 75 | 91 | 107 | 123 | 139 | 10 |
| 74 | 28 | 44 | 60 | 76 | 92 | 108 | 124 | 140 | 11 |
| 77 | 29 | 45 | 61 | 77 | 93 | 109 | 125 | 141 | 12 |
| 78 | 30 | 46 | 62 | 78 | 94 | 110 | 126 | 142 | 13 |
| 79 | 31 | 47 | 63 | 79 | 95 | 111 | 127 | 143 | 14 |
| 88 | 32 | 48 | 64 | 80 | 96 | 112 | 128 | 144 | 15 |

**Table 3**

| **Antibody EGFR/HER3** | **SEQ ID NO. of antibody region** | | | | | | | | |
|---|---|---|---|---|---|---|---|---|---|
| | **VH** | **VL** | **CDR1H** | **CDR2H** | **CDR3H** | **CDRL1** | **CDRL2** | **CDRL3** | **n** |
| 11 | 145 | 161 | 177 | 193 | 209 | 225 | 241 | 257 | 0 |
| 22 | 146 | 162 | 178 | 194 | 210 | 226 | 242 | 258 | 1 |
| 23 | 147 | 163 | 179 | 195 | 211 | 227 | 243 | 259 | 2 |
| 24 | 148 | 164 | 180 | 196 | 212 | 228 | 244 | 260 | 3 |
| 28 | 149 | 165 | 181 | 197 | 213 | 229 | 245 | 261 | 4 |
| 32 | 150 | 166 | 182 | 198 | 214 | 230 | 246 | 262 | 5 |
| 52 | 151 | 167 | 183 | 199 | 215 | 231 | 247 | 263 | 6 |
| 53 | 152 | 168 | 184 | 200 | 216 | 232 | 248 | 264 | 7 |
| 55 | 153 | 169 | 185 | 201 | 217 | 233 | 249 | 265 | 8 |
| 56 | 154 | 170 | 186 | 202 | 218 | 234 | 250 | 266 | 9 |
| 60 | 155 | 171 | 187 | 203 | 219 | 235 | 251 | 267 | 10 |
| 62 | 156 | 172 | 188 | 204 | 220 | 236 | 252 | 268 | 11 |
| 64 | 157 | 173 | 189 | 205 | 221 | 237 | 253 | 269 | 12 |
| 69 | 158 | 174 | 190 | 206 | 222 | 238 | 254 | 270 | 13 |
| 71 | 159 | 175 | 191 | 207 | 223 | 239 | 255 | 271 | 14 |
| 75 | 160 | 176 | 192 | 208 | 224 | 240 | 256 | 272 | 15 |

Cell-mediated effector functions like ADCC of antibodies according to the invention can be enhanced by engineering their oligosaccharide component as described in Umana, P., et al, Nature Biotechnol. 17 (1999) 176-180, and US 6,602,684, WO 2005/044859, WO 2004/065540, WO2007/031875. The term "antibody-dependent cellular cytotoxicity (ADCC)" refers to lysis of human target cells by an antibody according to the invention in the presence of effector cells.

The antibodies according to the invention are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression nucleic acids encoding light and heavy chains of an antibody according to the invention or fragments thereof are inserted into expression vectors by standard methods. Expression is performed in appropriate prokaryotic or eukaryotic host cells, such as CHO cells, NSO cells, SP2/0 cells, HEK293 cells, COS cells, yeast, or E. coli cells, and the antibody is recovered from the cells (from the supernatant or after cells lysis). Recombinant production of antibodies is well-known in the state of the art and described, for example, in the review articles of Makrides, S.C., Protein Expr. Purif. 17 (1999) 183-202; Geisse, S., et al, Protein Expr. Purif. 8 (1996) 271-282; Kaufman, R.J., Mol. Biotechnol. 16 (2000) 151-161; Werner, R.G., Drug Res. 48 (1998) 870-880. The antibodies may be present in whole cells, in a cell lysate, or in a partially purified, or pure form. Purification is performed in order to eliminate other cellular components or other contaminants, e.g., other cellular nucleic acids or proteins, by standard techniques, including, column chromatography and others well known in the art (see Ausubel, F., et al, ed. Current Protocols in Molecular Biology, Greene Publishing and Wiley Interscience, New York (1987)). Expression in NSO cells is described by, e.g., Barnes, L.M., et al, Cytotechnology 32 (2000) 109-123; Barnes, L.M., et al, Biotech. Bioeng. 73 (2001) 261-270. Transient expression is described by, e.g.,Durocher, Y., et al, Nucl. Acids. Res. 30 (2002) E9. Cloning of variable domains is described by Orlandi, R., et al, Proc. Natl. Acad. Sci. USA 86 (1989) 3833- 3837; Carter, P., et al, Proc. Natl. Acad. Sci. USA 89 (1992) 4285-4289; Norderhaug, L., et al, J. Immunol. Methods 204 (1997) 77-87. A preferred transient expression system (HEK 293) is described by Schlaeger, E.-J. and Christensen, K., in Cytotechnology 30 (1999) 71-83, and by Schlaeger, E.-J., in J. Immunol. Methods 194 (1996) 191-199. Monoclonal antibodies are suitably separated from the culture medium by conventional immunoglobulin purification procedures such as, for example, protein A-Sepharose, hydroxylapatite chromatography or affinity chromatography.

DNA and RNA encoding the monoclonal antibodies are sequenced using conventional procedures. RT PCR is preferably used.

Antibodies obtained from said cell lines are preferred embodiments of the invention. Amino acid sequence variants of an antibody are prepared by introducing nucleotide changes into the antibody encoding DNA, or by peptide synthesis. Any cysteine residue not involved in maintaining the proper conformation of the antibody may also be substituted, generally with serine, to improve the oxidative stability of the molecule and to prevent aberrant crosslinking. Conversely, cysteine bond(s) may be added to the antibody to improve its stability (particularly where the antibody is an antibody fragment such as an Fv fragment).

The heavy and light chain variable domains according to the invention are combined with sequences of promoter, translation initiation, constant region, 3' untranslated region, polyadenylation, and transcription termination to form expression vector constructs. The heavy and light chain expression constructs can be combined into a single vector, co-transfected, serially transfected, or separately transfected into host cells which are then fused to form a single host cell expressing both chains.

The term "respectively" means that for production of a bispecific antibody according to the invention at least the respective two antigens must be used. Therefore an antibody specifically binding to EGFR and HER2 is selected and produced if immunization is performed with at least the two antigens EGFR and HER2. An antibody specifically binding to EGFR and HER3 is selected and produced if immunization is performed with at least the two antigens EGFR and HER3. An antibody specifically binding to HER2 and HER3 is selected and produced if immunization is performed with at least the two antigens HER2 and HER3. Preferably for immunization all three antigens are used. Therefore it is not possible to produce a bispecific antibody against two antigens by immunization with only one antigen. Such an antibody would be directed to a common epitope of both antigens and is preferably excluded from the invention, especially excluded for the antigens EGFR and HER2.

One aspect of the invention is a pharmaceutical composition comprising an antibody according to the invention. Another aspect of the invention is the use of an antibody according to the invention for the manufacture of a pharmaceutical composition. A further aspect of the invention is a method for the manufacture of a pharmaceutical composition comprising an antibody according to the invention. In another aspect, the present invention provides a composition, e.g. a pharmaceutical composition, containing an antibody according to the present invention, formulated together with a pharmaceutical carrier.

Furthermore the antibodies according to the invention are especially useful for the treatment of diseases associated with a dysregulation of HER pathways, e.g. cancer. For this the antibody according to the invention can be investigated in a respective mouse tumor model e.g. according to Krupke DM; Begley DA; Sundberg JP; Bult CJ; Eppig JT, The Mouse Tumor Biology database., Nat Rev Cancer 2008 Jun;8(6):459-65. Therefore one aspect of the invention is a pharmaceutical composition for the treatment of cancer.

Another aspect of the invention is an antibody according to the invention for the treatment of cancer.

Another aspect of the invention is the use of an antibody according to the invention for the manufacture of a medicament for the treatment of cancer.

Another aspect of the invention is a method of treatment of a patient suffering from cancer by administering an antibody according to the invention to said patient in the need of such treatment.

As used herein, "pharmaceutical carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g. by injection or infusion).

A composition of the present invention can be administered by a variety of methods known in the art. As will be appreciated by the skilled artisan, the route and/or mode of administration will vary depending upon the desired results. To administer a compound of the invention by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents include saline and aqueous buffer solutions. Pharmaceutical carriers include sterile aqueous solutions or dispersions and sterile powders for the extemporaneous preparation of sterile injectable solutions or dispersion. The use of such media and agents for pharmaceutically active substances is known in the art.

The phrases "parenteral administration" and "administered parenterally" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion.

The term "cancer" as used herein may be, for example, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, lymphoma, lymphocytic leukemia, including refractory versions of any of the above cancers, or a combination of one or more of the above cancers. Preferably such cancer is a breast cancer, colon cancer, lung cancer, or pancreatic cancer. Preferably such cancers are further characterized by EGFR, HER2 and/or HER3 expression or overexpression.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin. Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art. Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The method according to the invention comprises in summary the steps of immunization, B cell isolation, enrichment of B cells, isolation of single B cells, preferably co-cultivation with feeder cells, selection of a single B cell which comprises respective mRNA, and production of the antibody according to the invention. Such methods are mentioned for the production of monospecific antibodies e.g. in WO2011147903, WO2007003041, WO2008045140, W02004106377, EP1255780, and EP1633787.

### Immunization

Immunization can be performed according to the methods known of the state of the art, e.g. by using DNA of the antigens or fragments thereof, complete protein antigens or fragments thereof, antigen expressing cells. Preferably the EGFR, HER2, and/or HER3 antigen is/are fusion polypeptides consisting of said antigen and a human Fc polypeptide. Preferably immunization in step i) is repeated at least three times and appropriately up to six times during 90 days (if an antibody according to the invention is identified already after e.g. the fourth immunization, further immunizations are not necessary). Preferably complete Freund's adjuvant (CFA) or CFA and incomplete Freund's adjuvant (IFA) is (are) used as adjuvant.

### B cell isolation

The B-cells are isolated from the rabbit, preferably from the blood of the rabbit. The B-cells are isolated up to 8 days, preferably 6 to 8 days, after 3rd to 6th immunization. Preferably PBMCs are isolated and depleted from macrophages (see e.g. EP0488470) and used as B cells in step iii). Isolation of B cells can be for example also performed by labeling non-B cells with non B cell markers, e.g. anti CD2, CD14, CD16, CD36, CD43, and CD235a antibodies and separating the labeled non B cells from non-labeled B cells.

### Enrichment of B cells

Antigen specific B cells are preferably isolated (enriched) in step iii) by treating the B cells with the EGFR, HER2 and/or HER3 antigens or a cell expressing one or more of the respective antigens. The antigens used for enrichment must not be all antigens used for immunization. It is also sufficient to use in this step only one of the antigens. Preferably the antigens and the cell expressing the antigens are used in immobilized manner, so that the antigen specific B cells can be separated easily. Such methods are e.g. described in Kodituwakko AP et al., Immunol. Cell Biol. (2003) 81, 163-170 and EP0488470.

### Isolation of single B cells

Isolation of single rabbit B cells in step iv) is preferably performed by FACS. Preferably an anti-rabbit IgG is used for FACS selection. Such selected single B cells are antibody producing B cells.

### Co-cultivation with feeder cells

Preferably the antigen producing B cells are co-cultivated with feeder cells after step iv) and before the selection step v) is performed. This increases the amount of antibody in the cell supernant and facilitates analysis and selection of secreted rabbit antibodies specifically binding to human EGFR and HER2, to EGFR and HER3 or to HER2 and HER3 (see e.g. Zubler, R.H., et al., Eur. J. Immunol. 14 (1984) 357-63, Wen L. et al., Eur. J. Immunol. 17 (1987) 887-92, Hoffmann P et al., J Immunol. Methods 1996;196(1):85-91, Roy A. et al., J Hematother. Stem Cell Res. 2001; 10(6):873-80, Dlu A. et al., Proc. Nati. Acad. Sci. USA Vol. 84, pp. 9140-9144, 1987, and EP0488470).

### Selection of a single B cell which comprises m RNA

Selection of a single B cell which comprises mRNA encoding polypeptides comprising a heavy and light chain variable region of an antibody according to the invention can be performed, preferably after co-cultivated with feeder cells, by analyzing the cell supernatant for secreted rabbit antibodies binds specifically to EGFR and HER2, to EGFR and HER3 or to HER2 and HER3. Analysis is preferably performed by ELISA. Immunoglobulin sequences can be then recovered from the selected single human B cell e.g. according to de Wildt RM, Hoet RM. Methods Mol. Biol. 2002; 178:121-31 and analyzed e.g. by RT PCR.

The production of an antibody according to the invention, expressed by a single B cell, can be performed by recombinant means.Techniques and procedures described or referenced herein are for example, the widely utilized methodologies described in Sambrook et al., Molecular Cloning: A Laboratory Manual 3rd. edition (2001) Cold Spring Harbor Laboratory Press, Cold Spring Harbor, N.Y. CURRENT PROTOCOLS IN MOLECULAR BIOLOGY (F. M. Ausubel, et al. eds., (2003)); the series METHODS IN ENZYMOLOGY (Academic Press, Inc.): PCR 2: A PRACTICAL APPROACH (M. J. MacPherson, B. D. Hames and G. R. Taylor eds. (1995)), Harlow and Lane, eds. (1988) ANTIBODIES, A LABORATORY MANUAL, and ANIMAL CELL CULTURE (R. I. Freshney, ed. ( 1987)).

The following examples and sequences are provided to aid the understanding of the present invention. It is understood that modifications can be made in the procedures set forth without departing from the spirit of the invention.

### Examples

### Example 1

### Immunization of rabbits (scheme 1)

Three recombinant human Fc-chimera proteins fused with the extracellular domains of the tyrosine kinase receptors EGFR, Her2 and Her3 were used as immunogen mixture. Two different immunization schemes, scheme 1 and scheme 2, were explored.

For the immunization according to scheme 1, three New Zealand White (NZW) rabbits were immunized by injecting 1ml of immunogen mixture in each of the animals at day day 0, 7, 14, 28, 56 and 84. Proteins were diluted in PBS), pooled in equimolar amounts and mixed 1:1 (v/v) with complete Freund's adjuvant (CFA) before use. A final concentration of 100µg of each immunogen (300µg in total) was used per animal for the 1st immunization and for the 2nd, 3rd, 4th, 5th and 6th immunization 50µg of each immunogen (150µg in total) and per animal was used. Blood samples were collected in tubes, coated with EDTA, four, five and six days post-immunization after the 3rd, 4th, 5th and 6th immunization. Seven anti EGFR/HER2 antibodies according to the invention were identified from the blood sample taken after the third immunization. Ten anti EGFR/HER3 antibodies according to the invention were identified from blood samples taken after the 3rd, 4th, 5th and 6th immunization. One anti Her2/HER3 antibody according to the invention was identified from blood samples taken after the 3^{rd} immunization.

### Example 2

### Immunization of rabbits (scheme 2)

For the immunization according to scheme 2, each of the three NZW rabbits were immunized subcutaneously with 1ml of immunogen mixture at day 0, 7, 14, 28, 42 and 56. For the first injection, proteins were diluted in PBS, pooled in equimolar amounts and mixed 1:1 (v/v) with CFA before use. A final concentration of 100µg of each Immunogen (300µg in total) per animal was used for the 1st immunization. For the 2nd ,3rd, 4th, 5th and 6th immunization, proteins were diluted in PBS, pooled in equimolar amounts and mixed 1:1 (v/v) with incomplete Freund's adjuvant (IFA) before use. 50µg of each Immunogen (150µg in total) was used per animal. Blood samples were collected in tubes, coated with EDTA, four, five and six days post-immunization after the 3rd, 4th, 5th and 6th Immunization at intervals of 2 weeks. Nine anti EGFR/HER2 antibodies according to the invention were identified from the blood sample taken after the 3rd, 5th and 6th immunization. Six anti EGFR/HER3 antibodies according to the invention were identified from blood samples taken after the 4th, 5th and 6th immunization. One anti Her2/HER3 antibody according to the invention was identified from blood samples taken after the 6th immunization.

### Example 3

### 3.1 Immunogen Coating/Cell Preparation

The three fusion-proteins used for immunization were coated onto a surface of a cell-culture 6-well plate with a concentration of 8µg in PBS/10cm² and incubated. Alternatively plates were seeded with a cell line BT-474 (DSMZ ACC 64) expressing the three tyrosine kinase receptors EGFR, Her2 and Her3 on their cell surface. One day before use cells were seeded in DMEM+5%FCS at a density leading to about 90% confluence after 24h.

### 3.2 Isolation of peripheral blood mononuclear cells from rabbits

PBMCs were isolated from whole blood of immunized rabbits. The blood was diluted 1:1 with PBS and layered on Lympholyte^{®} according to the manufacturer's instructions (Cedarlane, CL5120). Peripheral blood mononuclear cells (PBMC) were separated from erythrocytes by density gradient centrifugation (800xg, 20min, RT). Cells were removed from the interface, washed twice with PBS (800xg, 10min) and suspended in RPMI 1640 based cell culture medium.

### 3.3 Monocyte depletion

PBMCs were incubated in cell culture medium on plastic. Unbound lymphocytes were collected after incubation time.

### 3.4 Enrichment of antigen specific cells

Antigen specific lymphocytes were enriched on Immunogen coated plates or directly on BT-474 cells (see 3.1.). Lymphocytes were washed twice with PBS to remove unspecific cells and subsequently incubated with 750µl Trypsin per 10cm² culture surface for 7-10min. Detached cells were collected in cell culture medium for further steps.

### 3.5 Single-cell sorting of Immunoglobulin G-secreting lymphocytes

PBMCs/lymphocytes were stained with a FITC (Fluorescein Isothiocyanate Isomer 1) conjugated goat anti-rabbit IgG antibody, Abd Serotec, STAR121F). A flow cytometric analysis and single-cell sorting was performed with a FACS cytometer. Single positive lymphocytes were sorted directly to 200µl cell culture medium covering 3,0x10⁶ irradiated EL-4 B5 feeder cells (L. Wen et al. Eur. J. Immunol. 17 (1987) 887-892). The cell culture medium described above was supplemented with 5% activated T-cell macrophage supernatant from rabbits (MicroCoat). Co-cultivation medium was supplemented with 2x10⁻⁰⁶g/ml SAC (Staphylococcus Aureus Cowan) solution. After co-cultivation of B-cells and feeder cells for 7 days supernatants were transferred for antibody detection and cells were harvested in 100µl RNA isolation buffer (Qiagen, RLT).

### 3.6 Screening for Immunoglobulin's via enzyme-linked immunosorbent assay

Secreted rabbit antibodies were detected by analyzing the supernatant via a biotinylated capturing antibody (anti-rabbit IgG antibody produced in goat) with a final concentration of 1 µg/ml PBS+0,5%BSA+0,05%Tween^{®}20, coated on streptavidin microtiter plates and a horse radish peroxidase coupled anti-rabbit IgG detection antibody with a final concentration of 1:7500. Washing steps were performed by using PBS+0.1%Tween^{®}20. 3,3',5,5'-Tetramethylbenzidine (TMB) was used as substrate and HCl to stop the enzymatic reaction.

### 3.7 Determination of HER specific antibodies in B-cell Supernatants

Microtiter plates were coated with EGFR, HER2, HER3 protein (recombinant Fc chimeric conjugates of human EGFR, HER2, HER3 or IL12 Rß1). After a blocking process, specific antibodies from B-cell supernatants bind to the targets and are then detected by a POD-labeled anti-rabbit IgG antibody. The IL12R binding was used as a counterscreen. The HER proteins were tagged with a linker, huFc and His (EGFR does not have a His-tag) like the IL12R protein. Antibodies which bind to the tag were positive in both assays, whereas antigen specific antibodies just bound to the HER proteins and not to IL12R.

12.5µL 0.5µg/mL HER protein in PBS was transferred to a microtiter plate, incubated and washed 3x with Wash Buffer. 90µL Block Buffer was added to each well, incubated and washed. 12.5µL Standard Antibody (Anti-EGF-Receptor, ERBB2 Rabbit anti-Human Monoclonal (extracellular domain) (SP3) Antibody; ERBB3/HER3 (N-terminal) antibody; start concentration 2µg/ml; IL-12Rbeta1 antibody start concentration 500ng/ml) in 1:2 dilutions or sample diluted in ELISA buffer was added, incubated and washed. 12.5µl 1:5000 POD-Antibody (Anti-rabbit IgG, peroxidase-linked species-specific Fab2 fragment (from donkey) (ECL); assay dilution: 1:5000) in Elisa Buffer was added, incubated and washed. 15µl TMB was added and 15µl HCl was added after sufficient development. Absorbance (Optical Density O.D.) was read at 450nm/620nm. Results are shown in tables 4 to 6.
- ELISA Buffer:: PBS, 0.5% BSA, 0.05% Tween^{®}20
- Wash Buffer:: PBS, 0.1% Tween^{®}20
- Block Buffer:: PBS, 2% BSA, 0.05% Tween^{®}20

**Table 4**

| **Antibody HER2/HER3** | **Optical density (OD) measured for antigen** | | | | **IgG concentration [ng/ml]** |
|---|---|---|---|---|---|
| | EGFR | HER2 | HER3 | IL12R | |
| 47 | 0.0 | 1.8 | 0.4 | 0.0 | 207.3 |
| 87 | 0.0 | 0.9 | 1.6 | 0.0 | 1500.0 |

**Table 5**

| **Antibody EGFR/HER3** | **Optical density (OD) measured for antigen** | | | | **IgG concentration [ng/ml]** |
|---|---|---|---|---|---|
| | EGFR | HER2 | HER3 | IL12R | |
| 11 | 0,6 | 0,0 | 3,5 | 0,0 | 1502.9 |
| 22 | 1,9 | 0,0 | 2,9 | 0,0 | 196,7 |
| 23 | 3,0 | 0,0 | 3,2 | 0,0 | 233,7 |
| 24 | 2,6 | 0,0 | 2,6 | 0,0 | 1432,5 |
| 28 | 2,0 | 0,0 | 3,1 | 0,0 | 212,5 |
| 32 | 0,3 | 0,0 | 2,3 | 0,0 | 1170,2 |
| 52 | 2,7 | 0,0 | 2,1 | 0,0 | 146,1 |
| 53 | 2,4 | 0,0 | 0,3 | 0,0 | 81,2 |
| 55 | 2,7 | 0,0 | 3,3 | 0,0 | 250,4 |
| 56 | 3,4 | 0,0 | 3,1 | 0,0 | 565,3 |
| 60 | 2,8 | -0,1 | 1,0 | 0,0 | 59,9 |
| 62 | 2,4 | 0,0 | 2,1 | 0,0 | 102,9 |
| 64 | 2,9 | 0,0 | 0,3 | 0,0 | >max |
| 69 | 1,9 | 0,0 | 2,4 | 0,1 | 341,6 |
| 71 | 2,4 | 0,0 | 1,6 | 0,0 | 953,0 |
| 75 | 2,6 | 0,0 | 0,3 | 0,0 | 445.5 |

| **Table 6 Antibody EGFR/HER2** | **Optical density (OD) measured for antigen** | | | | **IgG concentration [ng/ml]** |
|---|---|---|---|---|---|
| | EGFR | HER2 | HER3 | IL12R | |
| 1 | 0,3 | 2,4 | 0,0 | 0,0 | 1596,5 |
| 6 | 0,4 | 3,2 | 0,0 | 0,0 | 1912,4 |
| 9 | 2,2 | 0,3 | 0,0 | 0,0 | 750,1 |
| 15 | 0,4 | 2,9 | 0,0 | 0,0 | 460,6 |
| 18 | 0,4 | 2,2 | 0,0 | 0,0 | 1501,7 |
| 20 | 1,7 | 2,5 | 0,0 | 0,0 | 563,0 |
| 21 | 2,9 | 0,4 | 0,0 | 0,0 | 1506,2 |
| 34 | 0,3 | 3,1 | 0,0 | 0,0 | 1143,3 |
| 45 | 2,6 | 0,3 | 0,0 | 0,0 | 1511,2 |
| 68 | 1,8 | 2,7 | 0,0 | 0,0 | 1500,0 |
| 72 | 1,8 | 2,7 | 0,0 | 0,0 | 425,8 |
| 74 | 2,7 | 0,5 | 0,0 | 0,0 | 486,6 |
| 77 | 1,5 | 2,5 | 0,0 | 0,0 | 1500,0 |
| 78 | 0,3 | 2,5 | 0,0 | 0,1 | 581,5 |
| 79 | 2,8 | 0,4 | 0,0 | 0,0 | 1294,4 |
| 88 | 1,2 | 0,4 | 0,0 | 0,0 | 1122,0 |

### Example 4

### Immunization of rats (comparison)

Immunization was performed according to example1 (immunization scheme 1) with Wistar rats.

### Result:

Overall 441 anti-huHer1, 774 anti-huHer2 and 299 anti-huHer3 were isolated after four blood sample collection steps from each rat (10weeks after immunization start) by a multiple immunization strategy. No bispecific antibody was identified.

## Claims

1. A method for the production of a bispecific antibody specifically binding to EGFR and HER2, to EGFR and HER3 or to HER2 and HER3, **characterized in** performing in the order specified the steps of
i) immunizing a rabbit with the three antigens EGFR, HER2 and HER3 or the two antigens EGFR and HER2, EGFR and HER3, or HER2 and HER3, respectively,
ii) isolating B cells from said rabbit,
iii) isolating B cells from said B cells isolated in step ii) which bind to one or two antigens used in step i) for immunization,
iv) isolating single B cells from said B cells isolated in step iii),
v) selecting one of said single B cells which comprises mRNA encoding a polypeptide comprising a VH region and/or a polypeptide comprising a VL region of an antibody which binds specifically to EGFR and HER2, or
selecting one of said single B cells which comprises mRNA encoding a polypeptide comprising a VH region and/or a polypeptide comprising a VL region of an antibody which binds specifically to EGFR and HER3 , or
selecting one of said single B cells which comprises mRNA encoding a polypeptide comprising a VH region and/or a polypeptide comprising a VL region of an antibody which binds specifically to HER2 and HER3, and
vi) producing the antibody expressed by said selected B cell.

2. A method according to claim 1, **characterized in** selecting in step v) a single B cell which comprises mRNA encoding an antibody specifically binding to HER2 and HER3, comprising a heavy chain variable (VH) region which is at least 90% identical to a VH region of SEQ ID NO:1+n and a light chain variable (VL) region which is at least 90% identical to a VL region of SEQ ID NO:3+n, wherein n is a number selected from the group of 0 and 1.

3. A method according to claim 1, **characterized in** selecting in step v) a single B cell which comprises mRNA encoding an antibody specifically binding to EGFR and HER2, comprising a VH region which is at least 90% identical to a VH region of SEQ ID NO:17+n and a VL region which is at least 90% identical to a VL region of SEQ ID NO:33+n, wherein n is a number selected from the group of 0, 1, 2, 3, 4, 5, 6, 7, 8, 9, 10, 11, 12, 13, 14, and 15.

4. A method according to claim 1, **characterized in** selecting in step v) a single B cell which comprises mRNA encoding an antibody specifically binding to EGFR and HER3 comprising a VH region which is at least 90% identical to a VH region of SEQ ID NO:145+n and a VL region which is at least 90% identical to a VL region of SEQ ID NO:161+n, wherein n is a number selected from the group of 0, 1, 2, 3, 4, 5, 6, 7, 8, 9, 10, 11, 12, 13, 14, and 15.

5. A monoclonal antibody, **characterized in** specifically binding to human HER2 and HER3.

6. An antibody according to claim 5 **characterized in that** the antibody comprises a VH region selected from the group of VH regions comprising a CDR1H region of SEQ ID NO:5+n, a CDR2H region of SEQ ID NO:7+n and a CDR3H region of SEQ ID NO:9+n and **in that** the antibody comprises a VL region selected from the group of VL regions comprising a CDR1L region of SEQ ID NO:11+n, a CDR2L region of SEQ ID NO:13+n and aCDR3L region of SEQ ID NO:15+n, wherein n is a number selected from the group consisting of 0 and 1.

7. A monoclonal rabbit antibody, **characterized in** specifically binding to human EGFR and HER2.

8. An antibody according to claim 7, **characterized in that** the antibody comprises a VH region selected from the group of VH regions comprising a CDR1H region of SEQ ID NO:49+n, a CDR2H region of SEQ ID NO:65+n and aCDR3H region of SEQ ID NO:81+n and **in that** the antibody comprises a VL region selected from the group of VL regions comprising a CDR1L region of SEQ ID NO:97+n, a CDR2L region of SEQ ID N0:113+n and aCDR3L region of SEQ ID NO:129+n, wherein n is a number selected from the group of 0, 1, 2, 3, 4, 5, 6, 7, 8, 9, 10, 11, 12, 13, 14, and 15.

9. A monoclonal rabbit antibody, **characterized in** specifically binding to human EGFR and HER3.

10. An antibody according to claim 9, **characterized in that** the antibody comprises a VH region selected from the group of VH regions comprising a CDR1H region of SEQ ID NO:177+n, a CDR2H region of SEQ ID NO:193+n and aCDR3H region of SEQ ID NO:209+n and **in that** the antibody comprises a VL region selected from the group of VL regions comprising a CDR1L region of SEQ ID NO:225+n, a CDR2L region of SEQ ID NO:241+n and aCDR3L region of SEQ ID NO:257+n, wherein n is a number selected from the group of 0, 1, 2, 3, 4, 5, 6, 7, 8, 9, 10, 11, 12, 13, 14, and 15.

11. An antibody according to any one of claims 5 to 10, **characterized in that** its CDRL2 region consists of the first three amino acids of SEQ ID NO: 13, 14, 113 to 128 or 241 to 256 respectively.

12. Pharmaceutical composition **characterized by** comprising an antibody according to any one of claims 5 to 11.

13. An antibody according to any one of claims 5 to 11 for use in the treatment of cancer.

14. A rabbit B cell, **characterized in** comprising mRNA encoding a polypeptide comprising a VH region and/or mRNA encoding a polypeptide comprising a VL region of an antibody according to any one of claims 5 to 11.

15. A method for the production of an antibody according to any one of claims 5 to 11, **characterized in** isolating from a rabbit B cell comprising mRNA encoding a polypeptide comprising a VH region and/or mRNA encoding a polypeptide comprising a VL region of said antibody, expressing recombinantly said antibody and selecting said antibody if it binds specifically to EGFR and HER2, to EGFR and HER3 or to HER2 and HER3.


SEQUENCE LISTING

<110> Mab Discovery GmbH

<120> Bispecific antibodys against EGFR, HER2 and HER3

<130> MD1202

<160> 280

<170> BiSSAP 1.0

<210> 1
<211> 109
<212> PRT
<213> Oryctolagus

<220>
<221> SOURCE
<222> 1..109
<223> /mol_type="protein"
/organism="Oryctolagus"

<400> 1
Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Ser Ser Tyr Ala
            20                  25                  30
Met Asn Trp Val Arg Gln Ala Pro Gly Lys Gly Leu Glu Trp Ile Gly
        35                  40                  45
Ile Ile Asp Ser Ser Gly Ile Thr Tyr Tyr Ala Thr Trp Ala Lys Gly
    50                  55                  60
Arg Phe Thr Ile Ser Lys Thr Ser Thr Thr Val Asp Leu Lys Met Thr
65                  70                  75                  80
Ser Leu Thr Thr Glu Asp Thr Ala Thr Tyr Phe Cys Ala Arg Gly Val
                85                  90                  95
Thr Leu Trp Gly Gln Gly Thr Leu Val Thr Val Ser Ser
            100                 105

<210> 2
<211> 118
<212> PRT
<213> Oryctolagus

<220>
<221> SOURCE
<222> 1..118
<223> /mol_type="protein"
/organism="Oryctolagus"

<400> 2
Gln Ser Val Glu Glu Ser Gly Gly Arg Leu Val Thr Pro Gly Thr Pro
1               5                   10                  15
Leu Thr Val Thr Cys Thr Val Ser Gly Phe Ser Leu Ser Ser Tyr Val
            20                  25                  30
Met Ser Trp Val Arg Gln Ala Pro Gly Lys Gly Leu Glu Trp Ile Gly
        35                  40                  45
Ile Ile Ala Ser Ser Gly His Thr Tyr Tyr Ala Thr Trp Ala Lys Gly
    50                  55                  60
Arg Phe Thr Ile Ser Lys Thr Ser Thr Thr Val Asp Leu Lys Ile Thr
65                  70                  75                  80
Ser Pro Thr Thr Glu Asp Thr Ala Thr Tyr Phe Cys Ala Arg Tyr Gly
                85                  90                  95
Ser Asp Ser Gly Trp Gly Tyr Tyr Phe Thr Leu Trp Gly Pro Gly Thr
            100                 105                 110
Leu Val Thr Val Ser Ser
        115

<210> 3
<211> 113
<212> PRT
<213> Oryctolagus

<220>
<221> SOURCE
<222> 1..113
<223> /mol_type="protein"
      /organism="Oryctolagus"

<400> 3
Ala Gln Val Leu Thr Gln Thr Ala Ser Pro Val Ser Ala Ala Val Gly
1               5                   10                  15
Ser Thr Val Thr Ile Asn Cys Gln Ala Ser Gln Ser Val Tyr Asn Asn
            20                  25                  30
Asn Arg Leu Ser Trp Phe Gln Gln Lys Pro Gly Gln Pro Pro Lys Leu
        35                  40                  45
Leu Ile Ser Gly Ala Ser Thr Leu Ala Ser Gly Val Ser Ser Arg Phe
    50                  55                  60
Ser Gly Ser Gly Ser Gly Thr Gln Phe Thr Leu Thr Ile Ser Asp Val
65                  70                  75                  80
Gln Cys Asp Asp Ala Ala Thr Tyr Tyr Cys Leu Gly Ser Tyr Asp Cys
                85                  90                  95
Ser Ser Ala Asp Cys Gly Ala Phe Gly Gly Gly Thr Glu Val Val Val
            100                 105                 110
Lys

<210> 4

<211> 110
<212> PRT
<213> Oryctolagus

<220>
<221> SOURCE
<222> 1..110
<223> /mol_type="protein"
      /organism="Oryctolagus"

<400> 4
Ala Gln Val Leu Thr Gln Thr Pro Ser Ser Val Ser Glu Pro Val Gly
1               5                   10                  15
Gly Thr Val Thr Ile Asn Cys Gln Ala Ser Glu Asn Ile Tyr Ser Tyr
            20                  25                  30
Leu Ser Trp Tyr Gln Gln Lys Arg Gly Gln Pro Pro Lys Leu Leu Ile
        35                  40                  45
Tyr Gly Ala Ser Thr Leu Ala Ser Gly Val Pro Ser Arg Phe Lys Gly
    50                  55                  60
Ser Gly Ser Gly Thr Glu Tyr Thr Leu Thr Ile Ser Asp Leu Glu Cys
65                  70                  75                  80
Asp Asp Ala Ala Thr Tyr Tyr Cys Gln Ser Gly Tyr Tyr Thr Asn Ser
                85                  90                  95
Gly Asp Leu Ala Phe Gly Gly Gly Thr Glu Val Val Val Lys
            100                 105                 110

<210> 5
<211> 8
<212> PRT
<213> Oryctolagus

<220>
<221> SOURCE
<222> 1..8
<223> /mol_type="protein"
      /organism="Oryctolagus"

<400> 5
Gly Phe Ser Leu Ser Ser Tyr Ala
1               5

<210> 6
<211> 8
<212> PRT
<213> Oryctolagus

<220>
<221> SOURCE
<222> 1..8
<223> /mol_type="protein"

/organism="Oryctolagus"

<400> 6
Gly Phe Ser Leu Ser Ser Tyr Val
1               5

<210> 7
<211> 7
<212> PRT
<213> Oryctolagus

<220>
<221> SOURCE
<222> 1..7
<223> /mol_type="protein"
      /organism="Oryctolagus"

<400> 7
Ile Asp Ser Ser Gly Ile Thr
1               5

<210> 8
<211> 7
<212> PRT
<213> Oryctolagus

<220>
<221> SOURCE
<222> 1..7
<223> /mol_type="protein"
      /organism="Oryctolagus"

<400> 8
Ile Ala Ser Ser Gly His Thr
1               5

<210> 9
<211> 6
<212> PRT
<213> Oryctolagus

<220>
<221> SOURCE
<222> 1..6
<223> /mol_type="protein"
      /organism="Oryctolagus"

<400> 9
Ala Arg Gly Val Thr Leu
1               5

<210> 10
<211> 15
<212> PRT
<213> Oryctolagus

<220>
<221> SOURCE
<222> 1..15
<223> /mol_type="protein"
      /organism="Oryctolagus"

<400> 10
Ala Arg Tyr Gly Ser Asp Ser Gly Trp Gly Tyr Tyr Phe Thr Leu
1               5                   10                  15

<210> 11
<211> 8
<212> PRT
<213> Oryctolagus

<220>
<221> SOURCE
<222> 1..8
<223> /mol_type="protein"
      /organism="Oryctolagus"

<400> 11
Gln Ser Val Tyr Asn Asn Asn Arg
1               5

<210> 12
<211> 6
<212> PRT
<213> Oryctolagus

<220>
<221> SOURCE
<222> 1..6
<223> /mol_type="protein"
      /organism="Oryctolagus"

<400> 12
Glu Asn Ile Tyr Ser Tyr
1               5

<210> 13
<211> 4
<212> PRT
<213> Oryctolagus

<220>
<221> SOURCE
<222> 1..4
<223> /mol_type="protein"
      /organism="Oryctolagus"

<400> 13
Gly Ala Ser Thr
1

<210> 14
<211> 4
<212> PRT
<213> Oryctolagus

<220>
<221> SOURCE
<222> 1..4
<223> /mol_type="protein"
      /organism="Oryctolagus"

<400> 14
Gly Ala Ser Thr
1

<210> 15
<211> 13
<212> PRT
<213> Oryctolagus

<220>
<221> SOURCE
<222> 1..13
<223> /mol_type="protein"
      /organism="Oryctolagus"

<400> 15
Leu Gly Ser Tyr Asp Cys Ser Ser Ala Asp Cys Gly Ala
1               5                   10

<210> 16
<211> 12
<212> PRT
<213> Oryctolagus

<220>
<221> SOURCE
<222> 1..12
<223> /mol_type="protein"

/organism="Oryctolagus"

<400> 16
Gln Ser Gly Tyr Tyr Thr Asn Ser Gly Asp Leu Ala
1               5                   10

<210> 17
<211> 114
<212> PRT
<213> Oryctolagus

<220>
<221> SOURCE
<222> 1..114
<223> /mol_type="protein"
      /organism="Oryctolagus"

<400> 17
Gln Ser Leu Glu Glu Ser Gly Gly Arg Leu Val Thr Pro Gly Thr Pro
1               5                   10                  15
Leu Thr Leu Thr Cys Thr Ala Ser Gly Phe Ser Leu Ser Ser Tyr Ala
            20                  25                  30
Met Ile Trp Val Arg Gln Ala Pro Gly Glu Gly Leu Glu Tyr Ile Gly
        35                  40                  45
Ile Ile Ile Thr Gly Gly Ser Ala Tyr Tyr Ala Asn Trp Ala Lys Gly
    50                  55                  60
Arg Phe Thr Ile Ser Arg Thr Ser Thr Thr Val Asp Leu Lys Met Thr
65                  70                  75                  80
Ser Leu Thr Thr Glu Asp Thr Ala Thr Tyr Phe Cys Ala Arg Gly Tyr
                85                  90                  95
Ser Gly Ser Ser Gly Asp Leu Trp Gly Gln Gly Thr Leu Val Thr Val
            100                 105                 110
Ser Ser

<210> 18
<211> 114
<212> PRT
<213> Oryctolagus

<220>
<221> SOURCE
<222> 1..114
<223> /mol_type="protein"
      /organism="Oryctolagus"

<400> 18
Gln Ser Val Glu Glu Ser Gly Gly Arg Leu Val Thr Pro Gly Thr Pro
1               5                   10                  15

Leu Thr Leu Thr Cys Thr Val Ser Gly Phe Ser Leu Ser Ser Tyr Thr
            20                  25                  30
Met Gly Trp Val Arg Gln Ala Pro Gly Lys Gly Leu Glu Tyr Ile Gly
        35                  40                  45
Ile Ile Asn Thr Gly Gly Tyr Thr Tyr Tyr Ala Ser Trp Ala Lys Gly
    50                  55                  60
Arg Phe Thr Ile Ser Arg Thr Ser Thr Thr Val Asp Leu Ser Met Thr
65                  70                  75                  80
Ser Leu Thr Thr Glu Asp Thr Ala Thr Tyr Phe Cys Ala Arg Val Tyr
                85                  90                  95
Pro Gly Ser Ser Cys Asp Leu Trp Gly Gln Gly Thr Leu Val Thr Val
            100                 105                 110
Ser Ser

<210> 19
<211> 116
<212> PRT
<213> Oryctolagus

<220>
<221> SOURCE
<222> 1..116
<223> /mol_type="protein"
/organism="Oryctolagus"

<400> 19
Gln Glu Gln Leu Glu Glu Ser Gly Glu Gly Leu Val Lys Pro Gly Thr
1               5                   10                  15
Pro Leu Thr Leu Thr Cys Thr Ala Ser Gly Phe Ser Leu Ser Ser Gly
            20                  25                  30
Asp Met Ser Trp Val Arg Gln Ala Pro Gly Lys Gly Leu Glu Trp Ile
        35                  40                  45
Gly Thr Ile Gly Val Asp Gly Ser Thr Asp Tyr Ala Asn Trp Ala Glu
    50                  55                  60
Gly Arg Phe Thr Ile Ser Arg Thr Ser Thr Thr Val Asp Leu Lys Met
65                  70                  75                  80
Thr Ser Pro Thr Thr Glu Asp Thr Ala Thr Tyr Phe Cys Ala Arg Gly
                85                  90                  95
Leu Phe Asp Asp Tyr Thr Phe Asn Leu Trp Gly Gln Gly Thr Leu Val
            100                 105                 110
Thr Val Ser Ser
        115

<210> 20
<211> 116
<212> PRT
<213> Oryctolagus

<220>
<221> SOURCE
<222> 1..116
<223> /mol_type="protein"
      /organism="Oryctolagus"

<400> 20
Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Asn Ser His Ala
            20                  25                  30
Met Ser Trp Val Arg Gln Ala Pro Gly Lys Gly Leu Glu Trp Ile Gly
        35                  40                  45
Ile Ile Ser Ser Ser Gly Ser Ala Trp Tyr Ala Thr Trp Val Lys Gly
    50                  55                  60
Arg Phe Thr Ile Ser Lys Thr Ser Thr Thr Val Asp Leu Lys Met Thr
65                  70                  75                  80
Ser Leu Thr Thr Glu Asp Thr Ala Thr Tyr Phe Cys Ala Arg Asp Ser
                85                  90                  95
Ala Met Val Pro Ser Asn Phe Asn Leu Trp Gly Gln Gly Thr Leu Val
            100                 105                 110
Thr Val Ser Ser
        115

<210> 21
<211> 119
<212> PRT
<213> Oryctolagus

<220>
<221> SOURCE
<222> 1..119
<223> /mol_type="protein"
      /organism="Oryctolagus"

<400> 21
Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Ser Thr Tyr Trp
            20                  25                  30
Met Ser Trp Val Arg Gln Ala Pro Gly Lys Gly Leu Glu Tyr Ile Gly
        35                  40                  45
Phe Ile Asn Thr Gly Gly Asn Ala Tyr Tyr Ala Ser Trp Ala Lys Gly
    50                  55                  60
Arg Phe Thr Ile Ser Lys Thr Ser Thr Ser Val Asp Leu Lys Ile Thr
65                  70                  75                  80
Ser Pro Thr Thr Glu Asp Thr Ala Thr Tyr Phe Cys Ala Gly Ala Tyr
                85                  90                  95
Val Gly Asp Val Gly Asp Gly Tyr Ala Leu Thr Leu Trp Gly Gln Gly
            100                 105                 110

Thr Leu Val Thr Val Ser Ser
        115

<210> 22
<211> 119
<212> PRT
<213> Oryctolagus

<220>
<221> SOURCE
<222> 1..119
<223> /mol_type="protein"
      /organism="Oryctolagus"

<400> 22
Gln Ser Leu Glu Glu Ser Gly Gly Gly Leu Val Gln Pro Glu Gly Ser
1               5                   10                  15
Leu Thr Leu Thr Cys Thr Ala Ser Gly Leu Asp Ala Ser Ser Asn Cys
            20                  25                  30
Trp Val Cys Trp Val Arg Gln Ala Pro Gly Lys Gly Leu Glu Trp Ile
        35                  40                  45
Ala Cys Ile Tyr Ala Ile Gly Ser Gly Val Thr Tyr Tyr Ala Ser Trp
    50                  55                  60
Val Asn Gly Arg Phe Ser Ile Ser Lys Thr Ser Ser Thr Thr Val Thr
65                  70                  75                  80
Leu Gln Met Thr Ser Leu Thr Val Ala Asp Thr Ala Thr Tyr Leu Cys
                85                  90                  95
Ala Arg Val Leu Asn Gly Val Gly Tyr Ile Asp Leu Trp Gly Pro Gly
            100                 105                 110
Thr Leu Val Thr Val Ser Ser
        115

<210> 23
<211> 120
<212> PRT
<213> Oryctolagus

<220>
<221> SOURCE
<222> 1..120
<223> /mol_type="protein"
      /organism="Oryctolagus"

<400> 23
Gln Glu His Leu Glu Glu Ser Gly Gly Gly Leu Val Lys Pro Pro Gly
1               5                   10                  15
Ser Leu Thr Leu Thr Cys Thr Ala Ser Gly Phe Ser Phe Ser Ser Thr
            20                  25                  30
Tyr Tyr Ile Cys Trp Val Arg Gln Ala Pro Gly Lys Gly Leu Glu Trp
        35                  40                  45

Ile Gly Cys Ile Tyr Ala Gly Ser Gly Thr Thr Tyr Tyr Ala Ser Trp
    50                  55                  60
Ala Lys Gly Arg Phe Thr Ile Ser Lys Thr Ser Ser Thr Thr Val Thr
65                  70                  75                  80
Leu Gln Met Ala Ser Leu Thr Ala Ala Asp Thr Ala Thr Tyr Phe Cys
                85                  90                  95
Ala Arg Ser Ser Asp Ser Trp Thr Tyr Gly Met Asp Leu Trp Gly Pro
            100                 105                 110
Gly Thr Leu Val Thr Val Ser Ser
        115                 120

<210> 24
<211> 122
<212> PRT
<213> Oryctolagus

<220>
<221> SOURCE
<222> 1..122
<223> /mol_type="protein"
      /organism="Oryctolagus"

<400> 24
Gln Glu Gln Leu Val Glu Ser Gly Gly Gly Leu Val Gln Pro Glu Gly
1               5                   10                  15
Ser Leu Thr Leu Thr Cys Lys Ala Ser Gly Phe Ser Phe Ser Ser Gly
            20                  25                  30
Tyr Tyr Met Cys Trp Val Arg Gln Ala Pro Gly Lys Gly Leu Glu Trp
        35                  40                  45
Ile Ala Cys Ile Tyr Ala Asp Ser Ser Glu Ser Thr Tyr Tyr Ala Ser
    50                  55                  60
Trp Ala Lys Gly Arg Phe Thr Ile Ser Lys Thr Ser Ser Thr Thr Val
65                  70                  75                  80
Thr Leu Gln Val Thr Ser Leu Thr Ala Ala Asp Thr Ala Thr Tyr Phe
                85                  90                  95
Cys Ala Arg Trp Gly Thr Val Ala Gly Tyr Ser Thr Phe Asn Leu Trp
            100                 105                 110
Gly Pro Gly Thr Leu Val Thr Val Ser Ser
        115                 120

<210> 25
<211> 118
<212> PRT
<213> Oryctolagus

<220>
<221> SOURCE
<222> 1..118
<223> /mol_type="protein"
      /organism="Oryctolagus"

<400> 25
Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Ser Ser Tyr Ala
            20                  25                  30
Met Thr Trp Val Arg Gln Ala Pro Gly Lys Gly Leu Glu Trp Ile Gly
        35                  40                  45
Ile Ile Asp His Ser Arg Ala Thr Tyr Tyr Ala Asn Trp Ala Lys Gly
    50                  55                  60
Arg Phe Thr Ile Ser Lys Thr Ser Ser Thr Thr Val Asp Leu Lys Val
65                  70                  75                  80
Thr Ser Leu Thr Thr Glu Asp Thr Ala Thr Tyr Phe Cys Ala Arg Phe
                85                  90                  95
Phe Asn Pro Gly Gly Ser Gly Tyr Leu Arg Leu Trp Gly Gln Gly Thr
            100                 105                 110
Leu Val Thr Val Ser Ser
        115

<210> 26
<211> 115
<212> PRT
<213> Oryctolagus

<220>
<221> SOURCE
<222> 1..115
<223> /mol_type="protein"
      /organism="Oryctolagus"

<400> 26
Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Ser Thr Tyr Ser
            20                  25                  30
Met Ser Trp Val Arg Gln Ala Pro Gly Lys Gly Leu Glu Trp Ile Gly
        35                  40                  45
Thr Ile Ala Ile Ser Gly Thr Thr Tyr Tyr Ala Ser Trp Ala Lys Gly
    50                  55                  60
Arg Phe Thr Ile Ser Lys Ser Ser Thr Thr Val Asp Leu Lys Ile Ser
65                  70                  75                  80
Ser Pro Thr Thr Glu Asp Thr Ala Thr Tyr Phe Cys Ala Arg Tyr Val
                85                  90                  95
Ser Asp Leu Ile Gly Thr Asn Leu Trp Gly Pro Gly Thr Leu Val Thr
            100                 105                 110
Val Ser Ser
        115

<210> 27
<211> 116

<212> PRT
<213> Oryctolagus

<220>
<221> SOURCE
<222> 1..116
<223> /mol_type="protein"
      /organism="Oryctolagus"

<400> 27
Gln Ser Leu Glu Glu Ser Gly Gly Arg Leu Val Thr Pro Gly Thr Leu
1               5                   10                  15
Leu Thr Leu Thr Cys Thr Ala Ser Gly Ile Asp Leu Ser Gly His Ala
            20                  25                  30
Met Ala Trp Val Arg Gln Ala Pro Gly Lys Gly Leu Glu Trp Ile Gly
        35                  40                  45
Ile Ile Tyr Asp Asn Gly Ile Thr Asn Tyr Pro Thr Trp Ala Lys Gly
    50                  55                  60
Arg Phe Thr Ile Ser Lys Thr Ser Thr Thr Val Glu Leu Lys Phe Thr
65                  70                  75                  80
Ser Pro Thr Thr Glu Asp Thr Ala Thr Tyr Phe Cys Ala Arg Asp Thr
                85                  90                  95
Gly Asn Gly Ala Leu Arg Leu Thr Leu Trp Gly Pro Gly Thr Leu Val
            100                 105                 110
Thr Val Ser Ser
        115

<210> 28
<211> 119
<212> PRT
<213> Oryctolagus

<220>
<221> SOURCE
<222> 1..119
<223> /mol_type="protein"
      /organism="Oryctolagus"

<400> 28
Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Ser Ser Gly Tyr
            20                  25                  30
Met Asn Trp Val Arg Gln Ala Pro Gly Lys Gly Leu Glu Trp Ile Gly
        35                  40                  45
Arg Ile His Thr Leu Ser Gly Ser Ile Trp Tyr Ala Ser Trp Val Lys
    50                  55                  60
Gly Arg Phe Thr Ile Ser Lys Thr Ser Thr Thr Val Asp Leu Lys Met
65                  70                  75                  80
Thr Ser Leu Thr Thr Lys Asp Thr Ala Thr Tyr Phe Cys Ala Arg Gly

85                  90                  95
Asn Ala Gly Gly Ser Ile Glu Tyr Gly Met Asp Leu Trp Gly Pro Gly
            100                 105                 110
Thr Leu Val Thr Val Ser Ala
        115

<210> 29
<211> 117
<212> PRT
<213> Oryctolagus

<220>
<221> SOURCE
<222> 1..117
<223> /mol_type="protein"
      /organism="Oryctolagus"

<400> 29
Gln Ser Val Glu Glu Ser Gly Gly Arg Leu Val Thr Pro Gly Thr Pro
1               5                   10                  15
Leu Thr Leu Thr Cys Thr Ile Ser Gly Phe Ser Leu Asn Asn Tyr Trp
            20                  25                  30
Met Ser Trp Val Arg Gln Ala Pro Gly Lys Gly Leu Glu Trp Ile Gly
        35                  40                  45
Ile Ile Ser Ser Ser Asp Thr Thr Trp Tyr Ala Ser Trp Ala Lys Gly
    50                  55                  60
Arg Phe Thr Ile Ser Arg Thr Ser Thr Thr Val Asp Leu Lys Met Thr
65                  70                  75                  80
Ser Leu Thr Thr Glu Asp Thr Ala Thr Tyr Phe Cys Ala Arg Asp Arg
                85                  90                  95
Gly Asp Val Gly Tyr Ser Asp Leu Asp Leu Trp Gly Pro Gly Thr Leu
            100                 105                 110
Val Thr Val Ser Ser
        115

<210> 30
<211> 117
<212> PRT
<213> Oryctolagus

<220>
<221> SOURCE
<222> 1..117
<223> /mol_type="protein"
      /organism="Oryctolagus"

<400> 30
Gln Ser Val Glu Glu Ser Gly Gly Arg Leu Val Thr Pro Gly Thr Pro
1               5                   10                  15
Leu Thr Leu Thr Cys Thr Val Ser Gly Ile Asp Leu Ser Ser Tyr Trp

                20                  25                  30
Met Ile Trp Val Arg Gln Ala Pro Gly Glu Gly Leu Glu Tyr Ile Gly
        35                  40                  45
Phe Ile Ser Asn Gly Gly Gly Ala Tyr Tyr Ala Asn Trp Thr Lys Gly
    50                  55                  60
Arg Phe Thr Ile Ser Arg Thr Ser Thr Thr Val Asp Leu Lys Met Thr
65                  70                  75                  80
Ser Leu Thr Thr Glu Asp Thr Ala Thr Tyr Phe Cys Ala Arg Ala Tyr
                85                  90                  95
Thr Thr Val Thr Ile Gly Gly Phe Asp Leu Trp Gly Pro Gly Thr Leu
            100                 105                 110
Val Thr Val Ser Ser
        115

<210> 31
<211> 118
<212> PRT
<213> Oryctolagus

<220>
<221> SOURCE
<222> 1..118
<223> /mol_type="protein"
      /organism="Oryctolagus"

<400> 31
Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Ser Ser Asn Ala
            20                  25                  30
Met Ser Trp Val Arg Gln Ala Pro Gly Lys Gly Leu Glu Trp Ile Gly
        35                  40                  45
Ile Ile Asn Ile Tyr Gly Ser Thr Asn Tyr Ala Ser Trp Ala Lys Gly
    50                  55                  60
Arg Phe Thr Ile Ser Lys Thr Ser Ser Thr Thr Val Asp Leu Lys Ile
65                  70                  75                  80
Thr Ser Pro Thr Thr Glu Asp Thr Ala Thr Tyr Phe Cys Ala Arg Asp
                85                  90                  95
Tyr Gly Gly Val Ser Ala Thr Arg Leu Asp Leu Trp Gly Gln Gly Thr
            100                 105                 110
Leu Val Thr Val Ser Ser
        115

<210> 32
<211> 117
<212> PRT
<213> Oryctolagus

<220>
<221> SOURCE

<222> 1..117
<223> /mol_type="protein"
      /organism="Oryctolagus"

<400> 32
Gln Ser Val Glu Glu Ser Gly Gly Arg Leu Val Thr Pro Gly Thr Pro
1               5                   10                  15
Leu Thr Leu Thr Cys Thr Val Ser Gly Phe Pro Leu Ser Ser Tyr Val
            20                  25                  30
Met Ser Trp Val Arg Gln Ala Pro Gly Thr Gly Leu Glu Tyr Ile Gly
        35                  40                  45
Ile Val Gly Ile Gly Gly Ser Ala Tyr Tyr Ala Ser Trp Ala Lys Gly
    50                  55                  60
Arg Phe Thr Ile Ser Arg Thr Ser Thr Thr Val Asp Leu Lys Met Thr
65                  70                  75                  80
Ser Leu Thr Pro Glu Asp Thr Ala Thr Tyr Phe Cys Ala Arg Gly Ala
                85                  90                  95
Gly Gly Asn Phe Tyr Ser Pro Leu Asp Leu Trp Gly Gln Gly Thr Leu
            100                 105                 110
Val Thr Val Ser Ser
        115

<210> 33
<211> 110
<212> PRT
<213> Oryctolagus

<220>
<221> SOURCE
<222> 1..110
<223> /mol_type="protein"
      /organism="Oryctolagus"

<400> 33
Asp Val Val Met Thr Gln Thr Pro Ala Ser Val Ser Glu Pro Val Gly
1               5                   10                  15
Gly Thr Val Thr Ile Lys Cys Gln Ala Ser Gln Ser Ile Ser Ser Trp
            20                  25                  30
Leu Ser Trp Tyr Gln Gln Lys Pro Gly Gln Pro Pro Lys Leu Leu Ile
        35                  40                  45
Tyr Gly Ala Ser Asn Leu Ala Ser Gly Val Ser Ser Arg Phe Lys Gly
    50                  55                  60
Ser Arg Ser Gly Thr Glu Tyr Thr Leu Thr Ile Ser Asp Leu Glu Cys
65                  70                  75                  80
Ala Asp Ala Ala Thr Tyr Tyr Cys Gln Cys Thr Asp Tyr Thr Ser Lys
                85                  90                  95
Tyr Val Gly Ala Phe Gly Gly Gly Thr Glu Val Leu Val Arg
            100                 105                 110

<210> 34

<211> 112
<212> PRT
<213> Oryctolagus

<220>
<221> SOURCE
<222> 1..112
<223> /mol_type="protein"
      /organism="Oryctolagus"

<400> 34
Asp Val Val Met Thr Gln Thr Pro Ala Ser Val Ser Glu Pro Val Gly
1               5                   10                  15
Gly Thr Val Thr Ile Lys Cys Gln Ala Ser Gln Ser Ile Ser Ser Ser
            20                  25                  30
Tyr Leu Ser Trp Tyr Gln Gln Lys Pro Gly Gln Pro Pro Lys Leu Leu
        35                  40                  45
Ile Tyr Arg Ala Ser Thr Leu Glu Ser Gly Val Pro Ser Arg Phe Lys
    50                  55                  60
Gly Ser Gly Ser Gly Thr Glu Phe Thr Leu Thr Ile Ser Asp Leu Glu
65                  70                  75                  80
Cys Ala Asp Ala Ala Thr Tyr Tyr Cys Gln Cys Thr Asp Tyr Ala Gly
                85                  90                  95
Ser Tyr Val Gly Gly Ala Phe Gly Gly Gly Thr Glu Val Val Val Lys
            100                 105                 110

<210> 35
<211> 111
<212> PRT
<213> Oryctolagus

<220>
<221> SOURCE
<222> 1..111
<223> /mol_type="protein"
      /organism="Oryctolagus"

<400> 35
Ala Tyr Asp Met Thr Gln Thr Pro Ala Ser Val Glu Val Ala Val Gly
1               5                   10                  15
Gly Thr Val Thr Ile Lys Cys Gln Ala Ser Gln Asn Ile Tyr Thr Tyr
            20                  25                  30
Leu Ser Trp Tyr Gln Gln Lys Pro Gly Gln Pro Pro Lys Leu Leu Ile
        35                  40                  45
Tyr Lys Thr Ser Thr Leu Ala Ser Gly Val Pro Ser Arg Phe Ser Gly
    50                  55                  60
Ser Gly Ser Gly Thr Glu Tyr Thr Leu Thr Val Ser Gly Val Gln Cys
65                  70                  75                  80
Asp Asp Ala Ala Thr Tyr Tyr Cys Gln Gln Gly Tyr Thr Thr Ser Ser
                85                  90                  95

Asn Val Ala Thr Pro Phe Gly Gly Gly Thr Glu Val Val Val Lys
            100                 105                 110

<210> 36
<211> 109
<212> PRT
<213> Oryctolagus

<220>
<221> SOURCE
<222> 1..109
<223> /mol_type="protein"
      /organism="Oryctolagus"

<400> 36
Ala Ile Glu Met Thr Gln Ser Pro Pro Ser Leu Ser Ala Ser Val Gly
1               5                   10                  15
Glu Thr Val Arg Ile Arg Cys Leu Ala Ser Glu Phe Leu Phe Asn Ala
            20                  25                  30
Val Ser Trp Tyr Gln Gln Lys Pro Gly Lys Pro Pro Thr Leu Leu Ile
        35                  40                  45
Ser Gly Ala Ser Asn Leu Glu Ser Gly Val Pro Pro Arg Phe Ser Gly
    50                  55                  60
Ser Gly Ser Gly Thr Asp Tyr Thr Leu Thr Ile Gly Gly Val Gln Ala
65                  70                  75                  80
Glu Asp Val Ala Thr Tyr Tyr Cys Leu Gly Asp Phe Ser Gly Gly Ala
                85                  90                  95
Asp Leu Thr Phe Gly Ala Gly Thr Lys Val Glu Ile Lys
            100                 105

<210> 37
<211> 109
<212> PRT
<213> Oryctolagus

<220>
<221> SOURCE
<222> 1..109
<223> /mol_type="protein"
      /organism="Oryctolagus"

<400> 37
Asp Val Val Met Thr Gln Thr Pro Ala Ser Val Ser Glu Pro Val Gly
1               5                   10                  15
Gly Thr Val Thr Ile Lys Cys Gln Ala Ser Gln Ser Ile Gly Asn Lys
            20                  25                  30
Leu Ala Trp Tyr Gln Gln Val Pro Gly Gln Pro Pro Lys Leu Leu Ile
        35                  40                  45
Tyr Ser Ala Ser Pro Leu Ala Ser Gly Val Ser Ser Arg Phe Lys Gly
    50                  55                  60

Ser Arg Ser Gly Thr Glu Tyr Thr Leu Thr Ile Ser Asp Leu Glu Cys
65                  70                  75                  80
Ala Asp Ala Ala Thr Tyr Tyr Cys Gln Cys Thr Tyr Tyr Gly Ser Ser
                85                  90                  95
Gly Asn Ala Phe Gly Gly Gly Thr Glu Val Val Val Lys
            100                 105

<210> 38
<211> 112
<212> PRT
<213> Oryctolagus

<220>
<221> SOURCE
<222> 1..112
<223> /mol_type="protein"
      /organism="Oryctolagus"

<400> 38
Asp Ile Val Met Thr Gln Thr Pro Ser Ser Val Glu Ala Ala Val Gly
1               5                   10                  15
Gly Thr Val Thr Ile Lys Cys Gln Ala Ser Gln Ser Val Tyr Asn Asn
            20                  25                  30
Asn Trp Leu Ala Trp Tyr Gln Gln Ile Pro Gly Gln Pro Pro Lys Leu
        35                  40                  45
Leu Ile Tyr Glu Ser Ser Thr Leu Ala Ser Gly Val Pro Ser Arg Phe
    50                  55                  60
Lys Gly Ser Gly Ser Gly Thr Gln Phe Thr Leu Thr Ile Ser Asp Val
65                  70                  75                  80
Gln Cys Asp Asp Ala Ala Met Tyr Tyr Cys Gln Thr Tyr Tyr Tyr Ser
                85                  90                  95
Ser Ser Arg Thr Tyr Ala Phe Gly Gly Gly Thr Glu Val Val Val Lys
            100                 105                 110

<210> 39
<211> 112
<212> PRT
<213> Oryctolagus

<220>
<221> SOURCE
<222> 1..112
<223> /mol_type="protein"
      /organism="Oryctolagus"

<400> 39
Asp Ile Val Met Thr Gln Thr Pro Ala Ser Val Ser Glu Pro Val Gly
1               5                   10                  15
Gly Thr Val Thr Ile Lys Cys Gln Ala Ser Gln Ser Ile Ser Ser Tyr
            20                  25                  30

Leu Ala Trp Tyr Gln Gln Lys Pro Gly Gln Pro Pro Lys Arg Leu Ile
        35                  40                  45
Tyr Lys Ala Ser Thr Leu Ala Ser Gly Val Pro Ser Arg Phe Lys Gly
    50                  55                  60
Ser Gly Ser Gly Thr Asp Phe Thr Leu Thr Ile Ser Asp Leu Glu Cys
65                  70                  75                  80
Ala Asp Ala Ala Thr Tyr Tyr Cys Gln Cys Thr Tyr Gly Asn Thr Gly
                85                  90                  95
Ser Asn Phe Phe Tyr Gly Phe Gly Gly Gly Thr Glu Val Val Val Lys
            100                 105                 110

<210> 40
<211> 108
<212> PRT
<213> Oryctolagus

<220>
<221> SOURCE
<222> 1..108
<223> /mol_type="protein"
      /organism="Oryctolagus"

<400> 40
Asp Val Val Met Thr Gln Thr Pro Ala Ser Val Ser Glu Pro Val Gly
1               5                   10                  15
Gly Thr Val Thr Ile Lys Cys Gln Ala Ser Gln Ser Ile Ser Thr Ala
            20                  25                  30
Leu Ala Trp Tyr Gln Gln Lys Pro Gly Gln Pro Pro Lys Leu Leu Ile
        35                  40                  45
Tyr Phe Thr Ser Thr Leu Ala Ser Gly Val Ser Ser Arg Phe Lys Gly
    50                  55                  60
Ser Gly Ser Gly Ala Glu Phe Thr Leu Thr Ile Ser Asp Leu Glu Cys
65                  70                  75                  80
Ala Asp Ala Ala Thr Tyr Tyr Cys Gln Gly Tyr Tyr Tyr Val Thr Asn
                85                  90                  95
Phe Pro Phe Gly Gly Gly Thr Glu Val Val Val Lys
            100                 105

<210> 41
<211> 110
<212> PRT
<213> Oryctolagus

<220>
<221> SOURCE
<222> 1..110
<223> /mol_type="protein"
      /organism="Oryctolagus"

<400> 41

Asp Val Val Met Thr Gln Thr Pro Ala Ser Val Ser Glu Pro Val Gly
1               5                   10                  15
Gly Thr Val Thr Ile Lys Cys Gln Ala Ser Gln Ile Ile Ser Asn Trp
            20                  25                  30
Leu Ala Trp Tyr Gln Gln Lys Pro Gly Gln Pro Pro Lys Leu Leu Ile
        35                  40                  45
Tyr Gly Ala Ser Ile Leu Ala Ser Gly Val Pro Ser Arg Phe Lys Ala
    50                  55                  60
Ser Arg Ser Gly Thr Asp Tyr Thr Leu Thr Ile Gly Asp Leu Glu Cys
65                  70                  75                  80
Ala Asp Ala Ala Thr Tyr Tyr Cys Gln Cys Pro Asp Tyr Ser Asp Thr
                85                  90                  95
Ser Leu Gly Ser Phe Gly Gly Gly Thr Glu Val Val Val Arg
            100                 105                 110

<210> 42
<211> 111
<212> PRT
<213> Oryctolagus

<220>
<221> SOURCE
<222> 1..111
<223> /mol_type="protein"
      /organism="Oryctolagus"

<400> 42
Asp Val Val Met Thr Gln Thr Pro Ala Ser Val Ser Glu Pro Val Gly
1               5                   10                  15
Gly Thr Val Thr Ile Lys Cys Gln Ala Ser Gln Ser Ile Gly Ser Asn
            20                  25                  30
Leu Ala Trp His Gln Gln Lys Pro Gly Gln Pro Pro Lys Leu Leu Ile
        35                  40                  45
Tyr Ala Ala Ser Asn Leu Ala Ser Gly Val Ser Ser Arg Phe Lys Gly
    50                  55                  60
Ser Arg Ser Gly Thr Glu Tyr Thr Leu Thr Ile Ser Ala Val Gln Cys
65                  70                  75                  80
Asp Asp Ala Ala Thr Tyr Tyr Cys Gln Tyr Thr Tyr Tyr Gly Ser Ser
                85                  90                  95
Val Val Ile Gly Thr Phe Gly Gly Gly Thr Glu Val Val Val Lys
            100                 105                 110

<210> 43
<211> 109
<212> PRT
<213> Oryctolagus

<220>
<221> SOURCE
<222> 1..109

<223> /mol_type="protein"
      /organism="Oryctolagus"

<400> 43
Asp Ile Val Met Thr Gln Thr Pro Ser Pro Val Ser Ala Ala Val Gly
1               5                   10                  15
Gly Thr Val Thr Ile Lys Cys Gln Ala Ser Gln Ser Ile Gly Ser Trp
            20                  25                  30
Leu Ser Trp Tyr Gln Gln Lys Pro Gly Gln Arg Pro Lys Leu Leu Ile
        35                  40                  45
Tyr Gly Ala Ser Thr Leu Glu Ser Gly Val Pro Ser Arg Phe Lys Gly
    50                  55                  60
Ser Gly Ser Gly Thr Glu Phe Thr Leu Thr Ile Ser Asp Leu Glu Cys
65                  70                  75                  80
Ala Asp Ala Ala Thr Tyr Tyr Cys His Cys Thr Tyr Gly Ser Val Ser
                85                  90                  95
Gly Asn Ala Phe Gly Gly Gly Thr Glu Val Val Val Lys
            100                 105

<210> 44
<211> 110
<212> PRT
<213> Oryctolagus

<220>
<221> SOURCE
<222> 1..110
<223> /mol_type="protein"
      /organism="Oryctolagus"

<400> 44
Ala Tyr Asp Met Thr Gln Thr Pro Ala Ser Val Glu Ala Ala Val Gly
1               5                   10                  15
Gly Thr Val Thr Ile Lys Cys Gln Ala Ser Glu Ser Ile Thr Asn Tyr
            20                  25                  30
Leu Asn Trp Tyr Gln Gln Lys Pro Gly Gln Pro Pro Lys Leu Leu Ile
        35                  40                  45
Tyr Ser Ala Ser Thr Leu Ala Ser Gly Val Pro Ser Arg Phe Lys Gly
    50                  55                  60
Ser Gly Ser Gly Thr Gln Phe Thr Leu Thr Ile Ser Gly Val Glu Cys
65                  70                  75                  80
Ala Asp Ala Gly Thr Tyr Tyr Cys Gln Gln Gly Tyr Ser Leu Ile Asn
                85                  90                  95
Val Gly Asp Ile Phe Gly Gly Gly Thr Glu Val Val Val Arg
            100                 105                 110

<210> 45
<211> 110
<212> PRT
<213> Oryctolagus

<220>
<221> SOURCE
<222> 1..110
<223> /mol_type="protein"
      /organism="Oryctolagus"

<400> 45
Ala Tyr Asp Met Thr Gln Thr Pro Ala Ser Val Glu Val Ala Val Gly
1               5                   10                  15
Gly Thr Val Thr Ile Lys Cys Gln Ala Ser Gln Ser Ile Ser Ser Tyr
            20                  25                  30
Leu Ser Trp Tyr Gln Gln Lys Pro Gly Gln Pro Pro Lys Leu Leu Ile
        35                  40                  45
Tyr Lys Ala Ser Thr Leu Ala Ser Gly Val Ser Ser Arg Phe Ser Gly
    50                  55                  60
Ser Gly Ser Gly Thr Glu Phe Thr Leu Thr Ile Ser Gly Val Gln Cys
65                  70                  75                  80
Asp Asp Ala Ala Thr Tyr Tyr Cys Gln Gln Gly Tyr Ser Glu Ser Asn
                85                  90                  95
Leu Asp Asn Ala Phe Gly Gly Gly Thr Glu Val Val Val Lys
            100                 105                 110

<210> 46
<211> 110
<212> PRT
<213> Oryctolagus

<220>
<221> SOURCE
<222> 1..110
<223> /mol_type="protein"
      /organism="Oryctolagus"

<400> 46
Ala Tyr Asp Met Thr Gln Thr Pro Ala Ser Val Glu Val Ala Val Gly
1               5                   10                  15
Gly Thr Val Thr Ile Asn Cys Gln Ala Ser Gln Asn Ile Tyr Asn Gly
            20                  25                  30
Leu Ala Trp Tyr Gln Gln Lys Pro Gly Gln Pro Pro Lys Leu Leu Ile
        35                  40                  45
Tyr Leu Ala Ser Thr Leu Ala Ser Gly Val Pro Ser Arg Phe Lys Gly
    50                  55                  60
Ser Gly Ser Gly Thr Glu Phe Thr Leu Thr Ile Ser Asp Leu Glu Cys
65                  70                  75                  80
Ala Asp Ala Ala Thr Tyr Tyr Cys Gln Gln Gly Phe Ser Val Asn Asn
                85                  90                  95
Val Gly Asn Val Phe Gly Gly Gly Thr Glu Met Val Val Lys
            100                 105                 110

<210> 47
<211> 113
<212> PRT
<213> Oryctolagus

<220>
<221> SOURCE
<222> 1..113
<223> /mol_type="protein"
      /organism="Oryctolagus"

<400> 47
Asp Val Val Met Thr Gln Thr Pro Ala Ser Val Glu Ala Ala Val Gly
1               5                   10                  15
Gly Thr Val Thr Ile Lys Cys Gln Ala Ser Gln Ser Ile Gly Ser Tyr
            20                  25                  30
Leu Ser Trp Tyr Gln Gln Lys Pro Gly Gln Pro Pro Lys Leu Leu Ile
        35                  40                  45
Tyr Asp Ala Ser Asn Leu Glu Asp Gly Val Pro Ser Arg Phe Lys Gly
    50                  55                  60
Ser Gly Ser Gly Thr Glu Tyr Thr Leu Thr Ile Ser Asp Leu Glu Cys
65                  70                  75                  80
Ala Asp Ala Val Thr Tyr Tyr Cys Gln Cys Thr Tyr Gly Tyr Gly Ile
                85                  90                  95
Ser Ser Ser Tyr Gly Asn Ala Phe Gly Gly Gly Thr Glu Val Val Val
            100                 105                 110
Lys

<210> 48
<211> 112
<212> PRT
<213> Oryctolagus

<220>
<221> SOURCE
<222> 1..112
<223> /mol_type="protein"
      /organism="Oryctolagus"

<400> 48
Asp Ile Val Met Thr Gln Thr Ala Ser Pro Leu Ser Gly Ala Val Gly
1               5                   10                  15
Gly Thr Val Thr Ile Lys Cys Gln Ala Ser Gln Ser Ile Ser Asn Glu
            20                  25                  30
Leu Ala Trp Tyr Gln Gln Lys Pro Gly Gln Arg Pro Lys Leu Leu Ile
        35                  40                  45
Tyr Leu Ala Ser Thr Leu Ala Ser Gly Val Pro Ser Arg Phe Lys Gly
    50                  55                  60

Ser Gly Ser Gly Thr Asp Phe Thr Leu Thr Ile Ser Asp Leu Glu Cys
65                  70                  75                  80
Ala Asp Ala Ala Thr Tyr Tyr Cys Gln Ser Tyr Ser Asp Thr Ser Ser
                85                  90                  95
Ile Ser Tyr Gly Tyr Gly Phe Gly Gly Gly Thr Glu Val Val Val Lys
            100                 105                 110

<210> 49
<211> 8
<212> PRT
<213> Oryctolagus

<220>
<221> SOURCE
<222> 1..8
<223> /mol_type="protein"
      /organism="Oryctolagus"

<400> 49
Gly Phe Ser Leu Ser Ser Tyr Ala
1               5

<210> 50
<211> 8
<212> PRT
<213> Oryctolagus

<220>
<221> SOURCE
<222> 1..8
<223> /mol_type="protein"
      /organism="Oryctolagus"

<400> 50
Gly Phe Ser Leu Ser Ser Tyr Thr
1               5

<210> 51
<211> 8
<212> PRT
<213> Oryctolagus

<220>
<221> SOURCE
<222> 1..8
<223> /mol_type="protein"
      /organism="Oryctolagus"

<400> 51
Gly Phe Ser Leu Ser Ser Gly Asp

1                   5

<210> 52
<211> 8
<212> PRT
<213> Oryctolagus

<220>
<221> SOURCE
<222> 1..8
<223> /mol_type="protein"
      /organism="Oryctolagus"

<400> 52
Gly Phe Ser Leu Asn Ser His Ala
1                   5

<210> 53
<211> 8
<212> PRT
<213> Oryctolagus

<220>
<221> SOURCE
<222> 1..8
<223> /mol_type="protein"
      /organism="Oryctolagus"

<400> 53
Gly Phe Ser Leu Ser Thr Tyr Trp
1                   5

<210> 54
<211> 9
<212> PRT
<213> Oryctolagus

<220>
<221> SOURCE
<222> 1..9
<223> /mol_type="protein"
      /organism="Oryctolagus"

<400> 54
Gly Leu Asp Ala Ser Ser Asn Cys Trp
1                   5

<210> 55
<211> 9
<212> PRT

<213> Oryctolagus

<220>
<221> SOURCE
<222> 1..9
<223> /mol_type="protein"
      /organism="Oryctolagus"

<400> 55
Gly Phe Ser Phe Ser Ser Thr Tyr Tyr
1               5

<210> 56
<211> 9
<212> PRT
<213> Oryctolagus

<220>
<221> SOURCE
<222> 1..9
<223> /mol_type="protein"
      /organism="Oryctolagus"

<400> 56
Gly Phe Ser Phe Ser Ser Gly Tyr Tyr
1               5

<210> 57
<211> 8
<212> PRT
<213> Oryctolagus

<220>
<221> SOURCE
<222> 1..8
<223> /mol_type="protein"
      /organism="Oryctolagus"

<400> 57
Gly Phe Ser Leu Ser Ser Tyr Ala
1               5

<210> 58
<211> 8
<212> PRT
<213> Oryctolagus

<220>
<221> SOURCE
<222> 1..8

<223> /mol_type="protein"
      /organism="Oryctolagus"

<400> 58
Gly Phe Ser Leu Ser Thr Tyr Ser
1               5

<210> 59
<211> 8
<212> PRT
<213> Oryctolagus

<220>
<221> SOURCE
<222> 1..8
<223> /mol_type="protein"
      /organism="Oryctolagus"

<400> 59
Gly Ile Asp Leu Ser Gly His Ala
1               5

<210> 60
<211> 8
<212> PRT
<213> Oryctolagus

<220>
<221> SOURCE
<222> 1..8
<223> /mol_type="protein"
      /organism="Oryctolagus"

<400> 60
Gly Phe Ser Leu Ser Ser Gly Tyr
1               5

<210> 61
<211> 8
<212> PRT
<213> Oryctolagus

<220>
<221> SOURCE
<222> 1..8
<223> /mol_type="protein"
      /organism="Oryctolagus"

<400> 61
Gly Phe Ser Leu Asn Asn Tyr Trp

1                   5

<210> 62
<211> 8
<212> PRT
<213> Oryctolagus

<220>
<221> SOURCE
<222> 1..8
<223> /mol_type="protein"
      /organism="Oryctolagus"

<400> 62
Gly Ile Asp Leu Ser Ser Tyr Trp
1                   5

<210> 63
<211> 8
<212> PRT
<213> Oryctolagus

<220>
<221> SOURCE
<222> 1..8
<223> /mol_type="protein"
      /organism="Oryctolagus"

<400> 63
Gly Phe Ser Leu Ser Ser Asn Ala
1                   5

<210> 64
<211> 8
<212> PRT
<213> Oryctolagus

<220>
<221> SOURCE
<222> 1..8
<223> /mol_type="protein"
      /organism="Oryctolagus"

<400> 64
Gly Phe Pro Leu Ser Ser Tyr Val
1                   5

<210> 65
<211> 7
<212> PRT

<213> Oryctolagus

<220>
<221> SOURCE
<222> 1..7
<223> /mol_type="protein"
      /organism="Oryctolagus"

<400> 65
Ile Ile Thr Gly Gly Ser Ala
1               5

<210> 66
<211> 7
<212> PRT
<213> Oryctolagus

<220>
<221> SOURCE
<222> 1..7
<223> /mol_type="protein"
      /organism="Oryctolagus"

<400> 66
Ile Asn Thr Gly Gly Tyr Thr
1               5

<210> 67
<211> 7
<212> PRT
<213> Oryctolagus

<220>
<221> SOURCE
<222> 1..7
<223> /mol_type="protein"
      /organism="Oryctolagus"

<400> 67
Ile Gly Val Asp Gly Ser Thr
1               5

<210> 68
<211> 7
<212> PRT
<213> Oryctolagus

<220>
<221> SOURCE
<222> 1..7

<223> /mol_type="protein"
      /organism="Oryctolagus"

<400> 68
Ile Ser Ser Ser Gly Ser Ala
1               5

<210> 69
<211> 7
<212> PRT
<213> Oryctolagus

<220>
<221> SOURCE
<222> 1..7
<223> /mol_type="protein"
      /organism="Oryctolagus"

<400> 69
Ile Asn Thr Gly Gly Asn Ala
1               5

<210> 70
<211> 9
<212> PRT
<213> Oryctolagus

<220>
<221> SOURCE
<222> 1..9
<223> /mol_type="protein"
      /organism="Oryctolagus"

<400> 70
Ile Tyr Ala Ile Gly Ser Gly Val Thr
1               5

<210> 71
<211> 8
<212> PRT
<213> Oryctolagus

<220>
<221> SOURCE
<222> 1..8
<223> /mol_type="protein"
      /organism="Oryctolagus"

<400> 71
Ile Tyr Ala Gly Ser Gly Thr Thr

1                   5

<210> 72
<211> 9
<212> PRT
<213> Oryctolagus

<220>
<221> SOURCE
<222> 1..9
<223> /mol_type="protein"
      /organism="Oryctolagus"

<400> 72
Ile Tyr Ala Asp Ser Ser Glu Ser Thr
1               5

<210> 73
<211> 7
<212> PRT
<213> Oryctolagus

<220>
<221> SOURCE
<222> 1..7
<223> /mol_type="protein"
      /organism="Oryctolagus"

<400> 73
Ile Asp His Ser Arg Ala Thr
1               5

<210> 74
<211> 7
<212> PRT
<213> Oryctolagus

<220>
<221> SOURCE
<222> 1..7
<223> /mol_type="protein"
      /organism="Oryctolagus"

<400> 74
Ile Ala Ile Ser Gly Thr Thr
1               5

<210> 75
<211> 7
<212> PRT

<213> Oryctolagus

<220>
<221> SOURCE
<222> 1..7
<223> /mol_type="protein"
      /organism="Oryctolagus"

<400> 75
Ile Tyr Asp Asn Gly Ile Thr
1               5

<210> 76
<211> 8
<212> PRT
<213> Oryctolagus

<220>
<221> SOURCE
<222> 1..8
<223> /mol_type="protein"
      /organism="Oryctolagus"

<400> 76
Ile His Thr Leu Ser Gly Ser Ile
1               5

<210> 77
<211> 7
<212> PRT
<213> Oryctolagus

<220>
<221> SOURCE
<222> 1..7
<223> /mol_type="protein"
      /organism="Oryctolagus"

<400> 77
Ile Ser Ser Ser Asp Thr Thr
1               5

<210> 78
<211> 7
<212> PRT
<213> Oryctolagus

<220>
<221> SOURCE
<222> 1..7

<223> /mol_type="protein"
      /organism="Oryctolagus"

<400> 78
Ile Ser Asn Gly Gly Gly Ala
1               5

<210> 79
<211> 7
<212> PRT
<213> Oryctolagus

<220>
<221> SOURCE
<222> 1..7
<223> /mol_type="protein"
      /organism="Oryctolagus"

<400> 79
Ile Asn Ile Tyr Gly Ser Thr
1               5

<210> 80
<211> 7
<212> PRT
<213> Oryctolagus

<220>
<221> SOURCE
<222> 1..7
<223> /mol_type="protein"
      /organism="Oryctolagus"

<400> 80
Val Gly Ile Gly Gly Ser Ala
1               5

<210> 81
<211> 11
<212> PRT
<213> Oryctolagus

<220>
<221> SOURCE
<222> 1..11
<223> /mol_type="protein"
      /organism="Oryctolagus"

<400> 81
Ala Arg Gly Tyr Ser Gly Ser Ser Gly Asp Leu

1                   5                        10

<210> 82
<211> 11
<212> PRT
<213> Oryctolagus

<220>
<221> SOURCE
<222> 1..11
<223> /mol_type="protein"
      /organism="Oryctolagus"

<400> 82
Ala Arg Val Tyr Pro Gly Ser Ser Cys Asp Leu
1                   5                        10

<210> 83
<211> 12
<212> PRT
<213> Oryctolagus

<220>
<221> SOURCE
<222> 1..12
<223> /mol_type="protein"
      /organism="Oryctolagus"

<400> 83
Ala Arg Gly Leu Phe Asp Asp Tyr Thr Phe Asn Leu
1                   5                        10

<210> 84
<211> 13
<212> PRT
<213> Oryctolagus

<220>
<221> SOURCE
<222> 1..13
<223> /mol_type="protein"
      /organism="Oryctolagus"

<400> 84
Ala Arg Asp Ser Ala Met Val Pro Ser Asn Phe Asn Leu
1                   5                        10

<210> 85
<211> 16
<212> PRT

<213> Oryctolagus

<220>
<221> SOURCE
<222> 1..16
<223> /mol_type="protein"
      /organism="Oryctolagus"

<400> 85
Ala Gly Ala Tyr Val Gly Asp Val Gly Asp Gly Tyr Ala Leu Thr Leu
1               5                   10                  15

<210> 86
<211> 12
<212> PRT
<213> Oryctolagus

<220>
<221> SOURCE
<222> 1..12
<223> /mol_type="protein"
      /organism="Oryctolagus"

<400> 86
Ala Arg Val Leu Asn Gly Val Gly Tyr Ile Asp Leu
1               5                   10

<210> 87
<211> 13
<212> PRT
<213> Oryctolagus

<220>
<221> SOURCE
<222> 1..13
<223> /mol_type="protein"
      /organism="Oryctolagus"

<400> 87
Ala Arg Ser Ser Asp Ser Trp Thr Tyr Gly Met Asp Leu
1               5                   10

<210> 88
<211> 14
<212> PRT
<213> Oryctolagus

<220>
<221> SOURCE
<222> 1..14

<223> /mol_type="protein"
      /organism="Oryctolagus"

<400> 88
Ala Arg Trp Gly Thr Val Ala Gly Tyr Ser Thr Phe Asn Leu
1               5                   10

<210> 89
<211> 14
<212> PRT
<213> Oryctolagus

<220>
<221> SOURCE
<222> 1..14
<223> /mol_type="protein"
      /organism="Oryctolagus"

<400> 89
Ala Arg Phe Phe Asn Pro Gly Gly Ser Gly Tyr Leu Arg Leu
1               5                   10

<210> 90
<211> 12
<212> PRT
<213> Oryctolagus

<220>
<221> SOURCE
<222> 1..12
<223> /mol_type="protein"
      /organism="Oryctolagus"

<400> 90
Ala Arg Tyr Val Ser Asp Leu Ile Gly Thr Asn Leu
1               5                   10

<210> 91
<211> 13
<212> PRT
<213> Oryctolagus

<220>
<221> SOURCE
<222> 1..13
<223> /mol_type="protein"
      /organism="Oryctolagus"

<400> 91
Ala Arg Asp Thr Gly Asn Gly Ala Leu Arg Leu Thr Leu

1                   5                   10

<210> 92
<211> 15
<212> PRT
<213> Oryctolagus

<220>
<221> SOURCE
<222> 1..15
<223> /mol_type="protein"
      /organism="Oryctolagus"

<400> 92
Ala Arg Gly Asn Ala Gly Gly Ser Ile Glu Tyr Gly Met Asp Leu
1               5                   10                  15

<210> 93
<211> 14
<212> PRT
<213> Oryctolagus

<220>
<221> SOURCE
<222> 1..14
<223> /mol_type="protein"
      /organism="Oryctolagus"

<400> 93
Ala Arg Asp Arg Gly Asp Val Gly Tyr Ser Asp Leu Asp Leu
1               5                   10

<210> 94
<211> 14
<212> PRT
<213> Oryctolagus

<220>
<221> SOURCE
<222> 1..14
<223> /mol_type="protein"
      /organism="Oryctolagus"

<400> 94
Ala Arg Ala Tyr Thr Thr Val Thr Ile Gly Gly Phe Asp Leu
1               5                   10

<210> 95
<211> 14
<212> PRT

<213> Oryctolagus

<220>
<221> SOURCE
<222> 1..14
<223> /mol_type="protein"
      /organism="Oryctolagus"

<400> 95
Ala Arg Asp Tyr Gly Gly Val Ser Ala Thr Arg Leu Asp Leu
1               5                   10

<210> 96
<211> 14
<212> PRT
<213> Oryctolagus

<220>
<221> SOURCE
<222> 1..14
<223> /mol_type="protein"
      /organism="Oryctolagus"

<400> 96
Ala Arg Gly Ala Gly Gly Asn Phe Tyr Ser Pro Leu Asp Leu
1               5                   10

<210> 97
<211> 6
<212> PRT
<213> Oryctolagus

<220>
<221> SOURCE
<222> 1..6
<223> /mol_type="protein"
      /organism="Oryctolagus"

<400> 97
Gln Ser Ile Ser Ser Trp
1               5

<210> 98
<211> 7
<212> PRT
<213> Oryctolagus

<220>
<221> SOURCE
<222> 1..7

<223> /mol_type="protein"
      /organism="Oryctolagus"

<400> 98
Gln Ser Ile Ser Ser Ser Tyr
1               5

<210> 99
<211> 6
<212> PRT
<213> Oryctolagus

<220>
<221> SOURCE
<222> 1..6
<223> /mol_type="protein"
      /organism="Oryctolagus"

<400> 99
Gln Asn Ile Tyr Thr Tyr
1               5

<210> 100
<211> 6
<212> PRT
<213> Oryctolagus

<220>
<221> SOURCE
<222> 1..6
<223> /mol_type="protein"
      /organism="Oryctolagus"

<400> 100
Glu Phe Leu Phe Asn Ala
1               5

<210> 101
<211> 6
<212> PRT
<213> Oryctolagus

<220>
<221> SOURCE
<222> 1..6
<223> /mol_type="protein"
      /organism="Oryctolagus"

<400> 101
Gln Ser Ile Gly Asn Lys

1                   5

<210> 102
<211> 8
<212> PRT
<213> Oryctolagus

<220>
<221> SOURCE
<222> 1..8
<223> /mol_type="protein"
      /organism="Oryctolagus"

<400> 102
Gln Ser Val Tyr Asn Asn Asn Trp
1               5

<210> 103
<211> 6
<212> PRT
<213> Oryctolagus

<220>
<221> SOURCE
<222> 1..6
<223> /mol_type="protein"
      /organism="Oryctolagus"

<400> 103
Gln Ser Ile Ser Ser Tyr
1               5

<210> 104
<211> 6
<212> PRT
<213> Oryctolagus

<220>
<221> SOURCE
<222> 1..6
<223> /mol_type="protein"
      /organism="Oryctolagus"

<400> 104
Gln Ser Ile Ser Thr Ala
1               5

<210> 105
<211> 6
<212> PRT

<213> Oryctolagus

<220>
<221> SOURCE
<222> 1..6
<223> /mol_type="protein"
      /organism="Oryctolagus"

<400> 105
Gln Ile Ile Ser Asn Trp
1               5

<210> 106
<211> 6
<212> PRT
<213> Oryctolagus

<220>
<221> SOURCE
<222> 1..6
<223> /mol_type="protein"
      /organism="Oryctolagus"

<400> 106
Gln Ser Ile Gly Ser Asn
1               5

<210> 107
<211> 6
<212> PRT
<213> Oryctolagus

<220>
<221> SOURCE
<222> 1..6
<223> /mol_type="protein"
      /organism="Oryctolagus"

<400> 107
Gln Ser Ile Gly Ser Trp
1               5

<210> 108
<211> 6
<212> PRT
<213> Oryctolagus

<220>
<221> SOURCE
<222> 1..6

<223> /mol_type="protein"
      /organism="Oryctolagus"

<400> 108
Glu Ser Ile Thr Asn Tyr
1               5

<210> 109
<211> 6
<212> PRT
<213> Oryctolagus

<220>
<221> SOURCE
<222> 1..6
<223> /mol_type="protein"
      /organism="Oryctolagus"

<400> 109
Gln Ser Ile Ser Ser Tyr
1               5

<210> 110
<211> 6
<212> PRT
<213> Oryctolagus

<220>
<221> SOURCE
<222> 1..6
<223> /mol_type="protein"
      /organism="Oryctolagus"

<400> 110
Gln Asn Ile Tyr Asn Gly
1               5

<210> 111
<211> 6
<212> PRT
<213> Oryctolagus

<220>
<221> SOURCE
<222> 1..6
<223> /mol_type="protein"
      /organism="Oryctolagus"

<400> 111
Gln Ser Ile Gly Ser Tyr

1                   5

<210> 112
<211> 6
<212> PRT
<213> Oryctolagus

<220>
<221> SOURCE
<222> 1..6
<223> /mol_type="protein"
      /organism="Oryctolagus"

<400> 112
Gln Ser Ile Ser Asn Glu
1                   5

<210> 113
<211> 4
<212> PRT
<213> Oryctolagus

<220>
<221> SOURCE
<222> 1..4
<223> /mol_type="protein"
      /organism="Oryctolagus"

<400> 113
Gly Ala Ser Asn
1

<210> 114
<211> 4
<212> PRT
<213> Oryctolagus

<220>
<221> SOURCE
<222> 1..4
<223> /mol_type="protein"
      /organism="Oryctolagus"

<400> 114
Arg Ala Ser Thr
1

<210> 115
<211> 4
<212> PRT

<213> Oryctolagus

<220>
<221> SOURCE
<222> 1..4
<223> /mol_type="protein"
      /organism="Oryctolagus"

<400> 115
Lys Thr Ser Thr
1

<210> 116
<211> 4
<212> PRT
<213> Oryctolagus

<220>
<221> SOURCE
<222> 1..4
<223> /mol_type="protein"
      /organism="Oryctolagus"

<400> 116
Gly Ala Ser Asn
1

<210> 117
<211> 4
<212> PRT
<213> Oryctolagus

<220>
<221> SOURCE
<222> 1..4
<223> /mol_type="protein"
      /organism="Oryctolagus"

<400> 117
Ser Ala Ser Pro
1

<210> 118
<211> 4
<212> PRT
<213> Oryctolagus

<220>
<221> SOURCE
<222> 1..4

<223> /mol_type="protein"
      /organism="Oryctolagus"

<400> 118
Glu Ser Ser Thr
1

<210> 119
<211> 4
<212> PRT
<213> Oryctolagus

<220>
<221> SOURCE
<222> 1..4
<223> /mol_type="protein"
      /organism="Oryctolagus"

<400> 119
Lys Ala Ser Thr
1

<210> 120
<211> 4
<212> PRT
<213> Oryctolagus

<220>
<221> SOURCE
<222> 1..4
<223> /mol_type="protein"
      /organism="Oryctolagus"

<400> 120
Phe Thr Ser Thr
1

<210> 121
<211> 4
<212> PRT
<213> Oryctolagus

<220>
<221> SOURCE
<222> 1..4
<223> /mol_type="protein"
      /organism="Oryctolagus"

<400> 121
Gly Ala Ser Ile

1

<210> 122
<211> 4
<212> PRT
<213> Oryctolagus

<220>
<221> SOURCE
<222> 1..4
<223> /mol_type="protein"
      /organism="Oryctolagus"

<400> 122
Ala Ala Ser Asn
1

<210> 123
<211> 4
<212> PRT
<213> Oryctolagus

<220>
<221> SOURCE
<222> 1..4
<223> /mol_type="protein"
      /organism="Oryctolagus"

<400> 123
Gly Ala Ser Thr
1

<210> 124
<211> 4
<212> PRT
<213> Oryctolagus

<220>
<221> SOURCE
<222> 1..4
<223> /mol_type="protein"
      /organism="Oryctolagus"

<400> 124
Ser Ala Ser Thr
1

<210> 125
<211> 4
<212> PRT

<213> Oryctolagus

<220>
<221> SOURCE
<222> 1..4
<223> /mol_type="protein"
      /organism="Oryctolagus"

<400> 125
Lys Ala Ser Thr
1

<210> 126
<211> 4
<212> PRT
<213> Oryctolagus

<220>
<221> SOURCE
<222> 1..4
<223> /mol_type="protein"
      /organism="Oryctolagus"

<400> 126
Leu Ala Ser Thr
1

<210> 127
<211> 4
<212> PRT
<213> Oryctolagus

<220>
<221> SOURCE
<222> 1..4
<223> /mol_type="protein"
      /organism="Oryctolagus"

<400> 127
Asp Ala Ser Asn
1

<210> 128
<211> 4
<212> PRT
<213> Oryctolagus

<220>
<221> SOURCE
<222> 1..4

<223> /mol_type="protein"
      /organism="Oryctolagus"

<400> 128
Leu Ala Ser Thr
1

<210> 129
<211> 12
<212> PRT
<213> Oryctolagus

<220>
<221> SOURCE
<222> 1..12
<223> /mol_type="protein"
      /organism="Oryctolagus"

<400> 129
Gln Cys Thr Asp Tyr Thr Ser Lys Tyr Val Gly Ala
1               5                   10

<210> 130
<211> 13
<212> PRT
<213> Oryctolagus

<220>
<221> SOURCE
<222> 1..13
<223> /mol_type="protein"
      /organism="Oryctolagus"

<400> 130
Gln Cys Thr Asp Tyr Ala Gly Ser Tyr Val Gly Gly Ala
1               5                   10

<210> 131
<211> 13
<212> PRT
<213> Oryctolagus

<220>
<221> SOURCE
<222> 1..13
<223> /mol_type="protein"
      /organism="Oryctolagus"

<400> 131
Gln Gln Gly Tyr Thr Thr Ser Ser Asn Val Ala Thr Pro

1                   5                   10

<210> 132
<211> 11
<212> PRT
<213> Oryctolagus

<220>
<221> SOURCE
<222> 1..11
<223> /mol_type="protein"
      /organism="Oryctolagus"

<400> 132
Leu Gly Asp Phe Ser Gly Gly Ala Asp Leu Thr
1               5                   10

<210> 133
<211> 11
<212> PRT
<213> Oryctolagus

<220>
<221> SOURCE
<222> 1..11
<223> /mol_type="protein"
      /organism="Oryctolagus"

<400> 133
Gln Cys Thr Tyr Tyr Gly Ser Ser Gly Asn Ala
1               5                   10

<210> 134
<211> 12
<212> PRT
<213> Oryctolagus

<220>
<221> SOURCE
<222> 1..12
<223> /mol_type="protein"
      /organism="Oryctolagus"

<400> 134
Gln Thr Tyr Tyr Tyr Ser Ser Ser Arg Thr Tyr Ala
1               5                   10

<210> 135
<211> 14
<212> PRT

<213> Oryctolagus

<220>
<221> SOURCE
<222> 1..14
<223> /mol_type="protein"
      /organism="Oryctolagus"

<400> 135
Gln Cys Thr Tyr Gly Asn Thr Gly Ser Asn Phe Phe Tyr Gly
1               5                   10

<210> 136
<211> 10
<212> PRT
<213> Oryctolagus

<220>
<221> SOURCE
<222> 1..10
<223> /mol_type="protein"
      /organism="Oryctolagus"

<400> 136
Gln Gly Tyr Tyr Tyr Val Thr Asn Phe Pro
1               5                   10

<210> 137
<211> 12
<212> PRT
<213> Oryctolagus

<220>
<221> SOURCE
<222> 1..12
<223> /mol_type="protein"
      /organism="Oryctolagus"

<400> 137
Gln Cys Pro Asp Tyr Ser Asp Thr Ser Leu Gly Ser
1               5                   10

<210> 138
<211> 13
<212> PRT
<213> Oryctolagus

<220>
<221> SOURCE
<222> 1..13

<223> /mol_type="protein"
      /organism="Oryctolagus"

<400> 138
Gln Tyr Thr Tyr Tyr Gly Ser Ser Val Val Ile Gly Thr
1               5                   10

<210> 139
<211> 11
<212> PRT
<213> Oryctolagus

<220>
<221> SOURCE
<222> 1..11
<223> /mol_type="protein"
      /organism="Oryctolagus"

<400> 139
His Cys Thr Tyr Gly Ser Val Ser Gly Asn Ala
1               5                   10

<210> 140
<211> 12
<212> PRT
<213> Oryctolagus

<220>
<221> SOURCE
<222> 1..12
<223> /mol_type="protein"
      /organism="Oryctolagus"

<400> 140
Gln Gln Gly Tyr Ser Leu Ile Asn Val Gly Asp Ile
1               5                   10

<210> 141
<211> 12
<212> PRT
<213> Oryctolagus

<220>
<221> SOURCE
<222> 1..12
<223> /mol_type="protein"
      /organism="Oryctolagus"

<400> 141
Gln Gln Gly Tyr Ser Glu Ser Asn Leu Asp Asn Ala

1                   5                   10

<210> 142
<211> 12
<212> PRT
<213> Oryctolagus

<220>
<221> SOURCE
<222> 1..12
<223> /mol_type="protein"
      /organism="Oryctolagus"

<400> 142
Gln Gln Gly Phe Ser Val Asn Asn Val Gly Asn Val
1               5                   10

<210> 143
<211> 15
<212> PRT
<213> Oryctolagus

<220>
<221> SOURCE
<222> 1..15
<223> /mol_type="protein"
      /organism="Oryctolagus"

<400> 143
Gln Cys Thr Tyr Gly Tyr Gly Ile Ser Ser Ser Tyr Gly Asn Ala
1               5                   10                  15

<210> 144
<211> 14
<212> PRT
<213> Oryctolagus

<220>
<221> SOURCE
<222> 1..14
<223> /mol_type="protein"
      /organism="Oryctolagus"

<400> 144
Gln Ser Tyr Ser Asp Thr Ser Ser Ile Ser Tyr Gly Tyr Gly
1               5                   10

<210> 145
<211> 121
<212> PRT

<213> Oryctolagus

<220>
<221> SOURCE
<222> 1..121
<223> /mol_type="protein"
      /organism="Oryctolagus"

<400> 145
Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Arg Ser Tyr Ala
            20                  25                  30
Met Ser Trp Val Arg Gln Ala Pro Gly Lys Gly Leu Glu Trp Val Gly
        35                  40                  45
Ile Ile Ser Thr Ser Gly Asp Thr Tyr Tyr Ala Ser Trp Ala Lys Gly
    50                  55                  60
Arg Phe Thr Ile Ser Lys Thr Ser Thr Thr Val Asp Leu Lys Ile Thr
65                  70                  75                  80
Ser Pro Thr Thr Glu Asp Thr Ala Thr Tyr Phe Cys Ala Arg Tyr Ser
                85                  90                  95
Tyr Asp Asp Tyr Gly Asp Tyr Val Arg Val Gly Met Asp Leu Trp Gly
            100                 105                 110
Pro Gly Thr Leu Val Thr Val Ser Ser
        115                 120

<210> 146
<211> 108
<212> PRT
<213> Oryctolagus

<220>
<221> SOURCE
<222> 1..108
<223> /mol_type="protein"
      /organism="Oryctolagus"

<400> 146
Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Ser Asn Tyr Gly
            20                  25                  30
Met Ser Trp Val Arg Gln Ala Pro Gly Lys Gly Leu Glu Trp Ile Gly
        35                  40                  45
Ile Ile Thr Ser Arg Ser Thr Tyr Tyr Ala Ser Trp Ala Lys Gly Arg
    50                  55                  60
Phe Thr Ile Ser Lys Thr Ser Ser Thr Thr Val Asp Leu Lys Met Thr
65                  70                  75                  80
Ser Leu Thr Thr Glu Asp Thr Ala Thr Tyr Phe Cys Gly Val Asn Asn
                85                  90                  95

Leu Trp Gly Gln Gly Thr Leu Val Thr Val Ser Ser
            100                 105

<210> 147
<211> 109
<212> PRT
<213> Oryctolagus

<220>
<221> SOURCE
<222> 1..109
<223> /mol_type="protein"
      /organism="Oryctolagus"

<400> 147
Gln Ser Leu Glu Glu Ser Gly Gly Arg Leu Val Lys Pro Gly Thr Pro
1               5                   10                  15
Leu Thr Leu Thr Cys Thr Ala Ser Gly Phe Ser Leu Ser Ser Tyr Tyr
            20                  25                  30
Met Ile Trp Val Arg Gln Ala Pro Gly Lys Gly Leu Glu Tyr Ile Gly
        35                  40                  45
Ile Ile Ser Ser Ser Gly Ser Thr Tyr Tyr Ala Ser Trp Ala Lys Gly
    50                  55                  60
Arg Phe Thr Ile Ser Lys Thr Ser Ser Thr Thr Val Asp Leu Lys Met
65                  70                  75                  80
Thr Ser Leu Thr Thr Glu Asp Thr Ala Thr Tyr Phe Cys Gly Val Asn
                85                  90                  95
Asn Leu Trp Gly Gln Gly Thr Leu Val Thr Val Ser Ser
            100                 105

<210> 148
<211> 117
<212> PRT
<213> Oryctolagus

<220>
<221> SOURCE
<222> 1..117
<223> /mol_type="protein"
      /organism="Oryctolagus"

<400> 148
Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Ser Ser Asn Ala
            20                  25                  30
Ile Ser Trp Val Arg Gln Ala Pro Gly Lys Gly Leu Glu Trp Ile Gly
        35                  40                  45
Phe Ile Asn Ile Tyr Gly Ser Thr Tyr Tyr Ala Ser Trp Ala Lys Gly
    50                  55                  60

Arg Phe Thr Ile Ser Arg Thr Ser Thr Thr Val Asp Leu Lys Met Thr
65                  70                  75                  80
Ser Leu Thr Thr Glu Asp Thr Ala Thr Tyr Phe Cys Ala Arg Asp Arg
                85                  90                  95
Thr Gly Asp Ser Pro Thr Arg Leu Asp Leu Trp Gly Gln Gly Thr Leu
            100                 105                 110
Val Thr Val Ser Ser
        115

<210> 149
<211> 118
<212> PRT
<213> Oryctolagus

<220>
<221> SOURCE
<222> 1..118
<223> /mol_type="protein"
      /organism="Oryctolagus"

<400> 149
Gln Glu Gln Leu Glu Glu Ser Gly Gly Asp Leu Val Lys Pro Gly Ala
1               5                   10                  15
Ser Leu Thr Leu Thr Cys Lys Val Ser Gly Phe Ser Leu Thr Ser Gly
            20                  25                  30
Tyr Tyr Met Cys Trp Val Arg Gln Ala Pro Gly Lys Gly Leu Glu Trp
        35                  40                  45
Leu Ala Cys Ile Tyr Thr Gly Ser Ser Gly Arg Thr Tyr Tyr Ala Ser
    50                  55                  60
Trp Ala Lys Gly Arg Phe Thr Ile Ser Lys Thr Ser Ser Thr Thr Val
65                  70                  75                  80
Thr Leu Gln Met Thr Ser Leu Thr Ala Ala Asp Thr Ala Thr Phe Phe
                85                  90                  95
Cys Ala Arg Glu Leu Tyr Leu Ser Ser Val Leu Trp Gly Pro Gly Thr
            100                 105                 110
Leu Val Thr Val Ser Ser
        115

<210> 150
<211> 109
<212> PRT
<213> Oryctolagus

<220>
<221> SOURCE
<222> 1..109
<223> /mol_type="protein"
      /organism="Oryctolagus"

<400> 150

Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Ser Ser Tyr Asp
            20                  25                  30
Met Ser Trp Val Arg Gln Ala Pro Gly Lys Gly Leu Glu Trp Ile Gly
        35                  40                  45
Phe Ile Asp Gly Asp Ser Asn Thr Tyr Asp Ala Thr Trp Ala Lys Gly
    50                  55                  60
Arg Phe Thr Ile Ser Lys Thr Ser Thr Thr Val Asp Leu Lys Ile Thr
65                  70                  75                  80
Ser Pro Thr Thr Glu Asp Thr Ala Thr Tyr Phe Cys Thr Arg Gly Phe
                85                  90                  95
Asn Leu Trp Gly Gln Gly Thr Leu Val Thr Val Ser Ser
            100                 105

<210> 151
<211> 116
<212> PRT
<213> Oryctolagus

<220>
<221> SOURCE
<222> 1..116
<223> /mol_type="protein"
      /organism="Oryctolagus"

<400> 151
Gln Ser Val Glu Glu Ser Gly Gly Arg Leu Val Thr Pro Gly Thr Ala
1               5                   10                  15
Leu Thr Leu Thr Cys Thr Val Ser Gly Phe Ser Leu Ser Ser Gly Asp
            20                  25                  30
Met Ser Trp Val Arg Gln Ala Pro Gly Lys Gly Leu Glu Trp Ile Gly
        35                  40                  45
Tyr Ile Trp Ser Gly Val Ser Thr Tyr Tyr Ala Asn Trp Ala Lys Gly
    50                  55                  60
Arg Phe Thr Ile Ser Lys Thr Ser Ser Thr Thr Val Asp Leu Lys Met
65                  70                  75                  80
Thr Ser Leu Thr Ile Glu Asp Thr Ala Thr Tyr Phe Cys Ala Arg Val
                85                  90                  95
Asp Phe Tyr Ser Tyr Val Tyr Ser Leu Trp Gly Pro Gly Thr Leu Val
            100                 105                 110
Thr Val Ser Ser
        115

<210> 152
<211> 116
<212> PRT
<213> Oryctolagus

<220>

<221> SOURCE
<222> 1..116
<223> /mol_type="protein"
      /organism="Oryctolagus"

<400> 152
Gln Ser Leu Glu Glu Ser Gly Gly Arg Leu Val Thr Pro Gly Gly Ser
1               5                   10                  15
Leu Thr Leu Thr Cys Thr Val Ser Gly Ser Asp Ile Ser Thr Tyr Asp
            20                  25                  30
Ala Ser Trp Val Arg Gln Ala Pro Gly Lys Gly Leu Glu Trp Ile Gly
        35                  40                  45
Tyr Ile Trp Ser Gly Thr Lys Thr Tyr Tyr Ala Ser Trp Ala Lys Gly
    50                  55                  60
Arg Phe Thr Ile Ser Lys Thr Ser Ser Thr Thr Val Asp Leu Lys Met
65                  70                  75                  80
Thr Ser Leu Thr Thr Glu Asp Thr Ala Thr Tyr Phe Cys Ala Thr Val
                85                  90                  95
Asn Thr Tyr Thr Tyr Val Tyr Ser Leu Trp Gly Pro Gly Thr Leu Val
            100                 105                 110
Thr Val Ser Ser
        115

<210> 153
<211> 116
<212> PRT
<213> Oryctolagus

<220>
<221> SOURCE
<222> 1..116
<223> /mol_type="protein"
      /organism="Oryctolagus"

<400> 153
Gln Ser Leu Glu Glu Ser Gly Gly Arg Leu Val Thr Pro Gly Gly Ser
1               5                   10                  15
Leu Thr Leu Thr Cys Thr Val Ser Gly Ser Asp Ile Ser Ser Tyr Asp
            20                  25                  30
Met Ser Trp Val Arg Gln Ala Pro Gly Lys Gly Leu Glu Trp Ile Gly
        35                  40                  45
Tyr Ile Trp Ser Gly Thr Thr Ile Tyr Tyr Ala Ser Trp Pro Lys Gly
    50                  55                  60
Arg Phe Thr Ile Ser Lys Thr Ser Ser Thr Thr Val Asp Leu Lys Met
65                  70                  75                  80
Thr Ser Leu Thr Thr Glu Asp Thr Ala Thr Tyr Phe Cys Ala Ala Ile
                85                  90                  95
Asn Ile Tyr Thr Tyr Val Tyr Ser Leu Trp Gly Pro Gly Thr Leu Val
            100                 105                 110
Thr Val Ser Ser

        115

<210> 154
<211> 116
<212> PRT
<213> Oryctolagus

<220>
<221> SOURCE
<222> 1..116
<223> /mol_type="protein"
      /organism="Oryctolagus"

<400> 154
Gln Ser Leu Glu Glu Ser Gly Gly Arg Leu Val Thr Pro Gly Thr Pro
1               5                   10                  15
Leu Thr Leu Thr Cys Thr Ala Ser Gly Phe Ser Leu Ser Ser Tyr Asp
            20                  25                  30
Leu Ser Trp Val Arg Gln Ala Pro Gly Lys Gly Leu Glu Trp Ile Ala
        35                  40                  45
Tyr Ile Trp Ser Gly Asp Lys Thr Tyr Tyr Ala Asn Trp Ala Lys Gly
    50                  55                  60
Arg Phe Thr Ile Ser Lys Thr Ser Ser Thr Thr Val Asp Leu Lys Met
65                  70                  75                  80
Thr Ser Leu Thr Thr Glu Asp Thr Ala Thr Tyr Phe Cys Ala Thr Val
                85                  90                  95
Asn Ile Tyr Ser Tyr Val Tyr Ser Leu Trp Gly Pro Gly Thr Leu Val
            100                 105                 110
Thr Val Ser Ser
        115

<210> 155
<211> 116
<212> PRT
<213> Oryctolagus

<220>
<221> SOURCE
<222> 1..116
<223> /mol_type="protein"
      /organism="Oryctolagus"

<400> 155
Gln Ser Leu Glu Glu Ser Gly Gly Arg Leu Val Thr Pro Gly Gly Ser
1               5                   10                  15
Leu Thr Leu Thr Cys Thr Val Ser Gly Ser Asp Ile Ser Ser Tyr Asp
            20                  25                  30
Met Ser Trp Val Arg Gln Ala Pro Gly Lys Gly Leu Glu Trp Ile Gly
        35                  40                  45
Tyr Ile Trp Ser Gly Thr Thr Thr Tyr Tyr Ala Asn Trp Ala Lys Gly

50                  55                  60
Arg Phe Thr Ile Ser Lys Thr Ser Ser Thr Thr Val Asp Leu Lys Met
65                  70                  75                  80
Thr Ser Leu Thr Thr Glu Asp Thr Ala Thr Tyr Phe Cys Ala Thr Val
                85                  90                  95
Asn Ile Tyr Thr Tyr Val Tyr Ser Leu Trp Gly Pro Gly Thr Leu Val
            100                 105                 110
Thr Val Ser Ser
        115

<210> 156
<211> 116
<212> PRT
<213> Oryctolagus

<220>
<221> SOURCE
<222> 1..116
<223> /mol_type="protein"
      /organism="Oryctolagus"

<400> 156
Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Ser Thr Asn Asp
            20                  25                  30
Ile Gly Trp Val Arg Gln Ala Pro Gly Lys Gly Leu Glu Trp Ile Gly
        35                  40                  45
Tyr Ile Trp Ser Gly Val Lys Thr Tyr Tyr Val Gly Trp Ala Lys Gly
    50                  55                  60
Arg Phe Thr Ile Ser Lys Thr Ser Ser Thr Thr Val Asp Leu Lys Met
65                  70                  75                  80
Thr Ser Leu Thr Thr Glu Asp Thr Ala Thr Tyr Phe Cys Ala Arg Val
                85                  90                  95
Asn Ser Phe Ser Tyr Val Tyr Ser Leu Trp Gly Pro Gly Thr Leu Val
            100                 105                 110
Thr Val Ser Ser
        115

<210> 157
<211> 116
<212> PRT
<213> Oryctolagus

<220>
<221> SOURCE
<222> 1..116
<223> /mol_type="protein"
      /organism="Oryctolagus"

<400> 157
Gln Glu Gln Leu Lys Glu Ser Gly Gly Gly Leu Val Thr Pro Gly Thr
1               5                   10                  15
Pro Leu Thr Leu Thr Cys Thr Ala Ser Gly Phe Thr Ile Ser Ser Tyr
            20                  25                  30
Asp Met Gly Trp Val Arg Gln Ala Pro Gly Lys Gly Leu Glu Trp Ile
        35                  40                  45
Gly Tyr Ile Trp Ser Gly Gly Ser Thr Tyr Tyr Ala Asn Trp Ala Lys
    50                  55                  60
Gly Arg Phe Thr Ile Ser Lys Thr Ser Thr Thr Val Asp Leu Lys Met
65                  70                  75                  80
Thr Ser Leu Thr Thr Glu Asp Thr Ala Thr Tyr Phe Cys Ala Arg Val
                85                  90                  95
Glu Phe Tyr Ser Tyr Val Tyr Val Leu Trp Gly Pro Gly Thr Leu Val
            100                 105                 110
Thr Val Ser Ser
        115

<210> 158
<211> 117
<212> PRT
<213> Oryctolagus

<220>
<221> SOURCE
<222> 1..117
<223> /mol_type="protein"
      /organism="Oryctolagus"

<400> 158
Gln Ser Val Glu Glu Ser Gly Gly Arg Leu Val Thr Pro Gly Thr Leu
1               5                   10                  15
Leu Thr Leu Thr Cys Thr Val Ser Gly Phe Ser Leu Thr Thr Tyr Ala
            20                  25                  30
Val Ser Trp Val Arg Gln Ala Pro Gly Lys Gly Leu Glu Tyr Met Gly
        35                  40                  45
Tyr Ile Ser Ala Thr Gly Ser Thr Tyr Tyr Ala Ser Trp Ala Lys Gly
    50                  55                  60
Arg Phe Thr Ile Ser Arg Thr Ser Thr Thr Val Asp Leu Lys Ile Thr
65                  70                  75                  80
Ser Pro Thr Ile Glu Asp Thr Ala Thr Tyr Phe Cys Ala Arg Ile Asp
                85                  90                  95
Ala Ala Thr Ser Tyr Ser Ala Phe Asp Pro Trp Gly Pro Gly Thr Leu
            100                 105                 110
Val Thr Val Ser Ser
        115

<210> 159
<211> 118
<212> PRT

<213> Oryctolagus

<220>
<221> SOURCE
<222> 1..118
<223> /mol_type="protein"
      /organism="Oryctolagus"

<400> 159
Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Ser Arg Asn Ala
            20                  25                  30
Met Ser Trp Val Arg Gln Ala Pro Gly Lys Gly Leu Glu Trp Ile Gly
        35                  40                  45
Ile Ile Asn Ile Tyr Gly Thr Thr Trp His Ala Gly Trp Ala Lys Gly
    50                  55                  60
Arg Phe Thr Ile Ser Lys Thr Ser Ser Thr Thr Val Thr Leu Lys Ile
65                  70                  75                  80
Thr Ser Pro Thr Thr Glu Asp Thr Ala Thr Tyr Phe Cys Ala Arg Asp
                85                  90                  95
Tyr Gly Gly Asp Ser Ala Thr Arg Leu Asn Leu Trp Gly Gln Gly Thr
            100                 105                 110
Leu Val Thr Val Ser Ser
        115

<210> 160
<211> 118
<212> PRT
<213> Oryctolagus

<220>
<221> SOURCE
<222> 1..118
<223> /mol_type="protein"
      /organism="Oryctolagus"

<400> 160
Gln Ser Val Glu Glu Ser Gly Gly Arg Leu Val Thr Pro Gly Thr Pro
1               5                   10                  15
Leu Thr Leu Thr Cys Thr Val Ser Gly Phe Ser Leu Ser Arg Asn Ala
            20                  25                  30
Met Ser Trp Val Arg Gln Ala Pro Gly Lys Gly Leu Glu Trp Ile Gly
        35                  40                  45
Ile Ile Asn Ile Tyr Gly Thr Thr Trp Tyr Ala Ser Trp Ala Lys Gly
    50                  55                  60
Arg Phe Thr Ile Ser Lys Thr Ser Ser Thr Thr Val Thr Leu Lys Ile
65                  70                  75                  80
Thr Ser Pro Thr Thr Glu Asp Thr Ala Thr Tyr Phe Cys Ala Arg Asp
                85                  90                  95

Tyr Gly Gly Asp Ser Ala Thr Arg Leu Asn Leu Trp Gly Gln Gly Thr
            100                 105                 110
Leu Val Thr Val Ser Ser
        115

<210> 161
<211> 112
<212> PRT
<213> Oryctolagus

<220>
<221> SOURCE
<222> 1..112
<223> /mol_type="protein"
      /organism="Oryctolagus"

<400> 161
Ala Ala Val Leu Thr Gln Thr Pro Ser Pro Val Ser Ala Ala Val Gly
1               5                   10                  15
Gly Thr Val Ser Ile Ser Cys Gln Ser Ser Lys Ser Val Tyr Tyr Asn
            20                  25                  30
Asn Trp Leu Ser Trp Phe Gln Gln Lys Pro Gly Gln Arg Pro Lys Leu
        35                  40                  45
Leu Ile Tyr Gly Ala Ser Thr Leu Ala Ser Gly Val Pro Ser Arg Phe
    50                  55                  60
Lys Gly Ser Gly Ser Glu Thr Gln Phe Thr Leu Thr Ile Ser Asp Val
65                  70                  75                  80
Gln Cys Asp Asp Ala Ala Thr Tyr Tyr Cys Ala Gly Gly Tyr Thr Ser
                85                  90                  95
Ser Ser Asp Thr Val Ser Phe Gly Gly Gly Thr Glu Val Val Val Lys
            100                 105                 110

<210> 162
<211> 111
<212> PRT
<213> Oryctolagus

<220>
<221> SOURCE
<222> 1..111
<223> /mol_type="protein"
      /organism="Oryctolagus"

<400> 162
Asp Pro Val Leu Thr Gln Thr Pro Ser Ser Val Ser Ala Ala Val Gly
1               5                   10                  15
Gly Thr Val Thr Ile Ser Cys Gln Ser Ser Gln Thr Ile Asn Asn Asn
            20                  25                  30
Asp Leu Ser Trp Phe Gln Gln Lys Pro Gly Gln Pro Pro Lys Arg Leu
        35                  40                  45

Ile Tyr Glu Ala Ser Thr Leu Thr Ser Gly Val Pro Ser Arg Phe Lys
    50                  55                  60
Gly Ser Gly Ser Gly Thr Gln Phe Thr Leu Thr Ile Ser Asp Val Gln
65                  70                  75                  80
Cys Asp Asp Ala Ala Thr Tyr Tyr Cys Leu Gly Gly Tyr Tyr Ser Tyr
                85                  90                  95
Gly Trp Tyr Phe Gly Phe Gly Gly Gly Thr Glu Val Val Val Lys
            100                 105                 110

<210> 163
<211> 111
<212> PRT
<213> Oryctolagus

<220>
<221> SOURCE
<222> 1..111
<223> /mol_type="protein"
      /organism="Oryctolagus"

<400> 163
Asp Pro Val Leu Thr Gln Thr Pro Ser Ser Val Ser Ala Ala Val Gly
1               5                   10                  15
Gly Thr Val Thr Ile Ser Cys Gln Ser Ser Gln Ser Ile Asn Asn Asn
            20                  25                  30
Asp Leu Ser Trp Phe Gln Lys Lys Pro Gly Gln Pro Pro Lys Arg Leu
        35                  40                  45
Ile Tyr Glu Ala Ser Thr Leu Thr Ser Gly Val Pro Ser Arg Phe Lys
    50                  55                  60
Gly Ser Gly Ser Gly Thr Gln Phe Thr Leu Thr Ile Ser Asp Val Gln
65                  70                  75                  80
Cys Asp Asp Ala Ala Thr Tyr Tyr Cys Leu Gly Gly Tyr Tyr Ser Tyr
                85                  90                  95
Gly Trp Tyr Phe Gly Phe Gly Gly Gly Thr Glu Val Val Val Lys
            100                 105                 110

<210> 164
<211> 113
<212> PRT
<213> Oryctolagus

<220>
<221> SOURCE
<222> 1..113
<223> /mol_type="protein"
      /organism="Oryctolagus"

<400> 164
Asp Val Val Met Thr Gln Thr Pro Ala Ser Val Glu Ala Ala Val Gly
1               5                   10                  15

Gly Thr Val Thr Ile Lys Cys Gln Ala Ser Gln Ser Ile Ser Ser Tyr
            20                  25                  30
Leu Ser Trp Tyr Gln Gln Lys Pro Gly Gln Pro Pro Lys Leu Leu Ile
        35                  40                  45
Tyr Asp Ala Ser Thr Leu Ala Ser Gly Val Pro Ser Arg Phe Lys Gly
    50                  55                  60
Ser Gly Ser Gly Thr Gln Phe Thr Leu Thr Ile Ser Asp Leu Glu Cys
65                  70                  75                  80
Ala Asp Ala Ala Thr Tyr Tyr Cys Gln Cys Thr Tyr Gly Tyr Gly Ala
                85                  90                  95
Ser Ser Ala Tyr Gly Asn Ala Phe Gly Gly Gly Thr Glu Met Val Val
            100                 105                 110
Lys

<210> 165
<211> 112
<212> PRT
<213> Oryctolagus

<220>
<221> SOURCE
<222> 1..112
<223> /mol_type="protein"
/organism="Oryctolagus"

<400> 165
Asp Val Val Met Thr Gln Thr Pro Ala Ser Val Ser Glu Pro Val Gly
1               5                   10                  15
Gly Thr Val Thr Ile Lys Cys Gln Ala Ser Gln Ser Ile Ser Ser Ser
            20                  25                  30
Tyr Leu Ser Trp Tyr Gln Gln Lys Pro Gly Gln Pro Pro Lys Leu Leu
        35                  40                  45
Ile Tyr Arg Ala Ser Thr Leu Glu Ser Gly Val Pro Ser Arg Phe Lys
    50                  55                  60
Gly Ser Gly Ser Gly Thr Glu Phe Thr Leu Thr Ile Ser Asp Leu Glu
65                  70                  75                  80
Cys Ala Asp Ala Ala Thr Tyr Tyr Cys Gln Cys Thr Asp Tyr Ala Gly
                85                  90                  95
Ser Tyr Val Gly Gly Ala Phe Gly Gly Gly Thr Glu Val Val Val Lys
            100                 105                 110

<210> 166
<211> 110
<212> PRT
<213> Oryctolagus

<220>
<221> SOURCE

<222> 1..110
<223> /mol_type="protein"
      /organism="Oryctolagus"

<400> 166
Asp Val Val Met Thr Gln Thr Pro Ala Ser Val Ser Glu Pro Val Gly
1               5                   10                  15
Gly Thr Val Thr Ile Lys Cys Gln Ala Ser Gln Ser Ile Ser Ser Tyr
            20                  25                  30
Leu Ala Trp Tyr Gln Gln Lys Pro Gly Gln Pro Pro Lys Leu Leu Ile
        35                  40                  45
Tyr Lys Ala Ser Thr Leu Ala Ser Gly Val Ser Ser Arg Phe Lys Gly
    50                  55                  60
Ser Gly Ser Gly Thr Glu Phe Thr Leu Thr Ile Ser Asp Leu Glu Cys
65                  70                  75                  80
Ala Asp Ala Ala Thr Tyr Tyr Cys Gln Cys Ser Ala Gly Gly Arg Ser
                85                  90                  95
Gly Ile Gly Gly Phe Gly Gly Gly Thr Glu Val Val Val Lys
            100                 105                 110

<210> 167
<211> 110
<212> PRT
<213> Oryctolagus

<220>
<221> SOURCE
<222> 1..110
<223> /mol_type="protein"
      /organism="Oryctolagus"

<400> 167
Ala Leu Val Met Thr Gln Thr Pro Ala Ser Val Glu Ala Ala Val Gly
1               5                   10                  15
Gly Thr Val Thr Ile Lys Cys Gln Ala Ser Gln Ser Ile Ser Ser Cys
            20                  25                  30
Leu Ala Trp Tyr Gln Gln Lys Pro Gly Gln Pro Pro Lys Leu Leu Ile
        35                  40                  45
Tyr Ser Ala Ser Thr Leu Ala Ser Gly Val Pro Ser Arg Phe Lys Gly
    50                  55                  60
Ser Gly Ser Gly Thr Glu Tyr Thr Leu Thr Ile Ser Gly Val Glu Cys
65                  70                  75                  80
Ala Asp Ala Ala Thr Tyr Tyr Cys Gln Gln His Tyr Thr Ser Asn Asn
                85                  90                  95
Ile Asp Asn Ala Phe Gly Gly Gly Thr Glu Val Val Val Lys
            100                 105                 110

<210> 168
<211> 110
<212> PRT

<213> Oryctolagus

<220>
<221> SOURCE
<222> 1..110
<223> /mol_type="protein"
      /organism="Oryctolagus"

<400> 168
Ala Tyr Asp Met Thr Gln Thr Pro Ala Ser Val Glu Val Ala Val Gly
1               5                   10                  15
Gly Thr Val Thr Ile Lys Cys Gln Ala Ser Gln Ser Ile Ser Ser Ser
            20                  25                  30
Leu Ala Trp Tyr Gln Gln Lys Ser Gly Gln Pro Pro Lys Leu Leu Ile
        35                  40                  45
Tyr Leu Ala Ser Thr Leu Ala Ser Gly Val Pro Ser Arg Phe Lys Gly
    50                  55                  60
Ser Gly Ser Gly Thr Glu Tyr Thr Leu Thr Ile Ser Gly Val Glu Cys
65                  70                  75                  80
Ala Asp Ala Ala Thr Tyr Tyr Cys Gln Gln His Tyr Thr Ala Leu Asn
                85                  90                  95
Val Asp Asn Ala Phe Gly Gly Gly Thr Glu Val Val Val Lys
            100                 105                 110

<210> 169
<211> 110
<212> PRT
<213> Oryctolagus

<220>
<221> SOURCE
<222> 1..110
<223> /mol_type="protein"
      /organism="Oryctolagus"

<400> 169
Ala Tyr Asp Met Thr Gln Thr Pro Ala Ser Val Glu Ala Ala Val Gly
1               5                   10                  15
Gly Thr Val Thr Ile Lys Cys Gln Ala Ser Glu Ser Ile Tyr Ser Ser
            20                  25                  30
Leu Ala Trp Tyr Gln Gln Lys Ser Gly Gln Pro Pro Lys Leu Leu Ile
        35                  40                  45
Tyr Leu Ala Ser Thr Leu Ala Ser Gly Val Pro Ser Arg Phe Lys Gly
    50                  55                  60
Ser Gly Ser Gly Thr Asp Tyr Thr Leu Thr Ile Ser Gly Val Glu Cys
65                  70                  75                  80
Ala Asp Ala Ala Thr Tyr Tyr Cys Gln Gln His Tyr Ser Gly Ser Asn
                85                  90                  95
Val Asp Asn Ala Phe Gly Gly Gly Thr Glu Val Val Val Lys
            100                 105                 110

<210> 170
<211> 110
<212> PRT
<213> Oryctolagus

<220>
<221> SOURCE
<222> 1..110
<223> /mol_type="protein"
      /organism="Oryctolagus"

<400> 170
Ala Tyr Asp Met Thr Gln Thr Pro Ala Ser Val Glu Val Ala Val Gly
1               5                   10                  15
Gly Thr Val Thr Ile Lys Cys Gln Ala Ser Gln Ser Ile Ser Thr Ala
            20                  25                  30
Leu Ala Trp Tyr Gln Gln Lys Ser Gly Gln Pro Pro Lys Leu Leu Ile
        35                  40                  45
Tyr Leu Ala Ser Thr Leu Ala Ser Gly Val Pro Ser Arg Phe Lys Gly
    50                  55                  60
Ser Gly Ser Gly Thr Glu Tyr Thr Leu Thr Ile Ser Gly Val Glu Cys
65                  70                  75                  80
Ala Asp Ala Ala Thr Tyr Tyr Cys Gln Gln His Tyr Ser Ser Asn Asn
                85                  90                  95
Val Asp Asn Ala Phe Gly Gly Gly Thr Glu Val Val Val Lys
            100                 105                 110

<210> 171
<211> 110
<212> PRT
<213> Oryctolagus

<220>
<221> SOURCE
<222> 1..110
<223> /mol_type="protein"
      /organism="Oryctolagus"

<400> 171
Ala Tyr Asp Met Thr Gln Thr Pro Ala Ser Val Glu Val Ala Val Gly
1               5                   10                  15
Gly Thr Val Thr Ile Lys Cys Gln Ala Ser Gln Ser Ile Ser Thr Ala
            20                  25                  30
Leu Ala Trp Tyr Gln Gln Lys Ser Gly Gln Pro Pro Lys Leu Leu Ile
        35                  40                  45
Tyr Leu Ala Ser Thr Leu Ala Ser Gly Val Pro Ser Arg Phe Lys Gly
    50                  55                  60
Ser Gly Ser Gly Thr Glu Tyr Thr Leu Thr Ile Ser Gly Val Glu Cys
65                  70                  75                  80

Ala Asp Ala Ala Thr Tyr Tyr Cys Gln Gln His Tyr Ser Gly Asn Asn
                85                  90                  95
Val Asp Asn Ala Phe Gly Gly Gly Thr Glu Val Val Val Lys
            100                 105                 110

<210> 172
<211> 110
<212> PRT
<213> Oryctolagus

<220>
<221> SOURCE
<222> 1..110
<223> /mol_type="protein"
      /organism="Oryctolagus"

<400> 172
Ala Tyr Asp Met Thr Gln Thr Pro Gly Ser Val Glu Val Ala Val Gly
1               5                   10                  15
Gly Thr Val Thr Ile Lys Cys Gln Ala Ser Glu Asp Ile His Ser Ser
            20                  25                  30
Leu Ala Trp Tyr Gln Gln Lys Pro Gly Gln Pro Pro Lys Leu Leu Ile
        35                  40                  45
Tyr Leu Ala Ser Thr Leu Glu Ser Gly Val Pro Ser Arg Phe Lys Gly
    50                  55                  60
Ser Gly Ser Gly Thr Glu Tyr Thr Leu Thr Ile Ser Gly Val Glu Cys
65                  70                  75                  80
Ala Asp Ala Ala Thr Tyr Tyr Cys Gln Gln His His Ser Thr Ser Gly
                85                  90                  95
Val Asp Asn Ala Phe Gly Gly Gly Thr Glu Val Val Val Lys
            100                 105                 110

<210> 173
<211> 110
<212> PRT
<213> Oryctolagus

<220>
<221> SOURCE
<222> 1..110
<223> /mol_type="protein"
      /organism="Oryctolagus"

<400> 173
Ala Phe Glu Leu Thr Gln Thr Pro Ser Ser Val Glu Ala Ala Val Gly
1               5                   10                  15
Gly Thr Val Thr Ile Lys Cys Gln Ala Ser Gln Ser Ile Ser Gly Cys
            20                  25                  30
Leu Ala Trp Tyr Gln Gln Lys Pro Gly Gln Pro Pro Lys Leu Leu Ile
        35                  40                  45

Tyr Leu Ala Ser Thr Leu Ala Ser Gly Val Ser Ser Arg Phe Arg Gly
    50                  55                  60
Ser Gly Ser Gly Thr Glu Tyr Thr Leu Thr Ile Ser Gly Val Glu Cys
65                  70                  75                  80
Asp Asp Ala Ala Thr Tyr Tyr Cys Gln Gln His Tyr Ser Ser Asn Asn
                85                  90                  95
Val Asp Asn Ala Phe Gly Gly Gly Thr Glu Val Val Val Lys
            100                 105                 110

<210> 174
<211> 112
<212> PRT
<213> Oryctolagus

<220>
<221> SOURCE
<222> 1..112
<223> /mol_type="protein"
      /organism="Oryctolagus"

<400> 174
Ala Gln Val Leu Thr Gln Thr Pro Ser Ser Val Ser Ala Ala Val Gly
1               5                   10                  15
Gly Thr Val Thr Ile Asn Cys Gln Ser Ser Glu Ser Val Tyr Lys Asn
            20                  25                  30
Asn Tyr Leu Ala Trp Tyr Gln Gln Lys Pro Gly Gln Pro Pro Arg Leu
        35                  40                  45
Leu Ile Tyr Leu Ala Ser Thr Leu Ala Ser Gly Val Pro Ser Arg Phe
    50                  55                  60
Ser Gly Ser Gly Ser Gly Thr Gln Phe Thr Leu Thr Ile Ser Gly Val
65                  70                  75                  80
Gln Cys Asp Asp Ala Ala Thr Tyr Tyr Cys Glu Ala Thr Tyr Val Gly
                85                  90                  95
Val Asp Trp Tyr Trp Ala Phe Gly Gly Gly Thr Glu Val Val Val Lys
            100                 105                 110

<210> 175
<211> 113
<212> PRT
<213> Oryctolagus

<220>
<221> SOURCE
<222> 1..113
<223> /mol_type="protein"
      /organism="Oryctolagus"

<400> 175
Asp Val Val Met Thr Gln Thr Pro Ala Ser Val Glu Ala Ala Val Gly
1               5                   10                  15

Gly Thr Val Thr Ile Lys Cys Gln Ala Ser Gln Ser Ile Gly Gly Tyr
            20                  25                  30
Leu Ser Trp Tyr Gln Gln Lys Pro Gly Gln Pro Pro Lys Leu Leu Ile
        35                  40                  45
Tyr Asp Ala Ser Asn Leu Glu Ile Gly Val Pro Ser Arg Phe Lys Gly
    50                  55                  60
Ser Gly Ser Gly Thr Glu Tyr Thr Leu Thr Ile Ser Asp Leu Glu Cys
65                  70                  75                  80
Ala Asp Ala Val Thr Tyr Tyr Cys Gln Cys Thr Tyr Gly Tyr Gly Ile
                85                  90                  95
Ser Ser Ser Tyr Gly Asn Ala Phe Gly Gly Gly Thr Glu Val Val Val
            100                 105                 110
Lys

<210> 176
<211> 113
<212> PRT
<213> Oryctolagus

<220>
<221> SOURCE
<222> 1..113
<223> /mol_type="protein"
/organism="Oryctolagus"

<400> 176
Asp Val Val Met Thr Gln Thr Pro Ala Ser Val Glu Ala Ala Val Gly
1               5                   10                  15
Gly Thr Val Thr Ile Lys Cys Gln Ala Ser Gln Ser Ile Gly Gly Tyr
            20                  25                  30
Leu Ser Trp Tyr Gln Gln Lys Pro Gly Gln Pro Pro Lys Leu Leu Ile
        35                  40                  45
Tyr Asp Ala Ser Asn Leu Glu Ile Gly Val Pro Ser Arg Phe Lys Gly
    50                  55                  60
Ser Gly Ser Gly Thr Glu Tyr Thr Leu Thr Ile Ser Asp Leu Glu Cys
65                  70                  75                  80
Ala Asp Ala Val Thr Tyr Tyr Cys Gln Cys Thr Tyr Gly Tyr Gly Ile
                85                  90                  95
Ser Ser Ser Tyr Gly Asn Ala Phe Gly Gly Gly Thr Glu Val Val Val
            100                 105                 110
Lys

<210> 177
<211> 8
<212> PRT
<213> Oryctolagus

<220>
<221> SOURCE
<222> 1..8
<223> /mol_type="protein"
      /organism="Oryctolagus"

<400> 177
Gly Phe Ser Leu Arg Ser Tyr Ala
1               5

<210> 178
<211> 8
<212> PRT
<213> Oryctolagus

<220>
<221> SOURCE
<222> 1..8
<223> /mol_type="protein"
      /organism="Oryctolagus"

<400> 178
Gly Phe Ser Leu Ser Asn Tyr Gly
1               5

<210> 179
<211> 8
<212> PRT
<213> Oryctolagus

<220>
<221> SOURCE
<222> 1..8
<223> /mol_type="protein"
      /organism="Oryctolagus"

<400> 179
Gly Phe Ser Leu Ser Ser Tyr Tyr
1               5

<210> 180
<211> 8
<212> PRT
<213> Oryctolagus

<220>
<221> SOURCE
<222> 1..8
<223> /mol_type="protein"

/organism="Oryctolagus"

<400> 180
Gly Phe Ser Leu Ser Ser Asn Ala
1               5

<210> 181
<211> 9
<212> PRT
<213> Oryctolagus

<220>
<221> SOURCE
<222> 1..9
<223> /mol_type="protein"
      /organism="Oryctolagus"

<400> 181
Gly Phe Ser Leu Thr Ser Gly Tyr Tyr
1               5

<210> 182
<211> 8
<212> PRT
<213> Oryctolagus

<220>
<221> SOURCE
<222> 1..8
<223> /mol_type="protein"
      /organism="Oryctolagus"

<400> 182
Gly Phe Ser Leu Ser Ser Tyr Asp
1               5

<210> 183
<211> 8
<212> PRT
<213> Oryctolagus

<220>
<221> SOURCE
<222> 1..8
<223> /mol_type="protein"
      /organism="Oryctolagus"

<400> 183
Gly Phe Ser Leu Ser Ser Gly Asp
1               5

<210> 184
<211> 8
<212> PRT
<213> Oryctolagus

<220>
<221> SOURCE
<222> 1..8
<223> /mol_type="protein"
      /organism="Oryctolagus"

<400> 184
Gly Ser Asp Ile Ser Thr Tyr Asp
1               5

<210> 185
<211> 8
<212> PRT
<213> Oryctolagus

<220>
<221> SOURCE
<222> 1..8
<223> /mol_type="protein"
      /organism="Oryctolagus"

<400> 185
Gly Ser Asp Ile Ser Ser Tyr Asp
1               5

<210> 186
<211> 8
<212> PRT
<213> Oryctolagus

<220>
<221> SOURCE
<222> 1..8
<223> /mol_type="protein"
      /organism="Oryctolagus"

<400> 186
Gly Phe Ser Leu Ser Ser Tyr Asp
1               5

<210> 187
<211> 8
<212> PRT
<213> Oryctolagus

<220>
<221> SOURCE
<222> 1..8
<223> /mol_type="protein"
      /organism="Oryctolagus"

<400> 187
Gly Ser Asp Ile Ser Ser Tyr Asp
1               5

<210> 188
<211> 8
<212> PRT
<213> Oryctolagus

<220>
<221> SOURCE
<222> 1..8
<223> /mol_type="protein"
      /organism="Oryctolagus"

<400> 188
Gly Phe Ser Leu Ser Thr Asn Asp
1               5

<210> 189
<211> 8
<212> PRT
<213> Oryctolagus

<220>
<221> SOURCE
<222> 1..8
<223> /mol_type="protein"
      /organism="Oryctolagus"

<400> 189
Gly Phe Thr Ile Ser Ser Tyr Asp
1               5

<210> 190
<211> 8
<212> PRT
<213> Oryctolagus

<220>
<221> SOURCE
<222> 1..8
<223> /mol_type="protein"

/organism="Oryctolagus"

<400> 190
Gly Phe Ser Leu Thr Thr Tyr Ala
1               5

<210> 191
<211> 8
<212> PRT
<213> Oryctolagus

<220>
<221> SOURCE
<222> 1..8
<223> /mol_type="protein"
      /organism="Oryctolagus"

<400> 191
Gly Phe Ser Leu Ser Arg Asn Ala
1               5

<210> 192
<211> 8
<212> PRT
<213> Oryctolagus

<220>
<221> SOURCE
<222> 1..8
<223> /mol_type="protein"
      /organism="Oryctolagus"

<400> 192
Gly Phe Ser Leu Ser Arg Asn Ala
1               5

<210> 193
<211> 7
<212> PRT
<213> Oryctolagus

<220>
<221> SOURCE
<222> 1..7
<223> /mol_type="protein"
      /organism="Oryctolagus"

<400> 193
Ile Ser Thr Ser Gly Asp Thr
1               5

<210> 194
<211> 6
<212> PRT
<213> Oryctolagus

<220>
<221> SOURCE
<222> 1..6
<223> /mol_type="protein"
      /organism="Oryctolagus"

<400> 194
Ile Thr Ser Arg Ser Thr
1               5

<210> 195
<211> 7
<212> PRT
<213> Oryctolagus

<220>
<221> SOURCE
<222> 1..7
<223> /mol_type="protein"
      /organism="Oryctolagus"

<400> 195
Ile Ser Ser Ser Gly Ser Thr
1               5

<210> 196
<211> 7
<212> PRT
<213> Oryctolagus

<220>
<221> SOURCE
<222> 1..7
<223> /mol_type="protein"
      /organism="Oryctolagus"

<400> 196
Ile Asn Ile Tyr Gly Ser Thr
1               5

<210> 197
<211> 9
<212> PRT
<213> Oryctolagus

<220>
<221> SOURCE
<222> 1..9
<223> /mol_type="protein"
      /organism="Oryctolagus"

<400> 197
Ile Tyr Thr Gly Ser Ser Gly Arg Thr
1               5

<210> 198
<211> 7
<212> PRT
<213> Oryctolagus

<220>
<221> SOURCE
<222> 1..7
<223> /mol_type="protein"
      /organism="Oryctolagus"

<400> 198
Ile Asp Gly Asp Ser Asn Thr
1               5

<210> 199
<211> 7
<212> PRT
<213> Oryctolagus

<220>
<221> SOURCE
<222> 1..7
<223> /mol_type="protein"
      /organism="Oryctolagus"

<400> 199
Ile Trp Ser Gly Val Ser Thr
1               5

<210> 200
<211> 7
<212> PRT
<213> Oryctolagus

<220>
<221> SOURCE
<222> 1..7
<223> /mol_type="protein"

/organism="Oryctolagus"

<400> 200
Ile Trp Ser Gly Thr Lys Thr
1               5

<210> 201
<211> 7
<212> PRT
<213> Oryctolagus

<220>
<221> SOURCE
<222> 1..7
<223> /mol_type="protein"
      /organism="Oryctolagus"

<400> 201
Ile Trp Ser Gly Thr Thr Ile
1               5

<210> 202
<211> 7
<212> PRT
<213> Oryctolagus

<220>
<221> SOURCE
<222> 1..7
<223> /mol_type="protein"
      /organism="Oryctolagus"

<400> 202
Ile Trp Ser Gly Asp Lys Thr
1               5

<210> 203
<211> 7
<212> PRT
<213> Oryctolagus

<220>
<221> SOURCE
<222> 1..7
<223> /mol_type="protein"
      /organism="Oryctolagus"

<400> 203
Ile Trp Ser Gly Thr Thr Thr
1               5

<210> 204
<211> 7
<212> PRT
<213> Oryctolagus

<220>
<221> SOURCE
<222> 1..7
<223> /mol_type="protein"
      /organism="Oryctolagus"

<400> 204
Ile Trp Ser Gly Val Lys Thr
1               5

<210> 205
<211> 7
<212> PRT
<213> Oryctolagus

<220>
<221> SOURCE
<222> 1..7
<223> /mol_type="protein"
      /organism="Oryctolagus"

<400> 205
Ile Trp Ser Gly Gly Ser Thr
1               5

<210> 206
<211> 7
<212> PRT
<213> Oryctolagus

<220>
<221> SOURCE
<222> 1..7
<223> /mol_type="protein"
      /organism="Oryctolagus"

<400> 206
Ile Ser Ala Thr Gly Ser Thr
1               5

<210> 207
<211> 7
<212> PRT
<213> Oryctolagus

<220>
<221> SOURCE
<222> 1..7
<223> /mol_type="protein"
      /organism="Oryctolagus"

<400> 207
Ile Asn Ile Tyr Gly Thr Thr
1               5

<210> 208
<211> 7
<212> PRT
<213> Oryctolagus

<220>
<221> SOURCE
<222> 1..7
<223> /mol_type="protein"
      /organism="Oryctolagus"

<400> 208
Ile Asn Ile Tyr Gly Thr Thr
1               5

<210> 209
<211> 18
<212> PRT
<213> Oryctolagus

<220>
<221> SOURCE
<222> 1..18
<223> /mol_type="protein"
      /organism="Oryctolagus"

<400> 209
Ala Arg Tyr Ser Tyr Asp Asp Tyr Gly Asp Tyr Val Arg Val Gly Met
1               5                   10                  15
Asp Leu

<210> 210
<211> 5
<212> PRT
<213> Oryctolagus

<220>

<221> SOURCE
<222> 1..5
<223> /mol_type="protein"
      /organism="Oryctolagus"

<400> 210
Gly Val Asn Asn Leu
1               5

<210> 211
<211> 5
<212> PRT
<213> Oryctolagus

<220>
<221> SOURCE
<222> 1..5
<223> /mol_type="protein"
      /organism="Oryctolagus"

<400> 211
Gly Val Asn Asn Leu
1               5

<210> 212
<211> 14
<212> PRT
<213> Oryctolagus

<220>
<221> SOURCE
<222> 1..14
<223> /mol_type="protein"
      /organism="Oryctolagus"

<400> 212
Ala Arg Asp Arg Thr Gly Asp Ser Pro Thr Arg Leu Asp Leu
1               5                   10

<210> 213
<211> 10
<212> PRT
<213> Oryctolagus

<220>
<221> SOURCE
<222> 1..10
<223> /mol_type="protein"
      /organism="Oryctolagus"

<400> 213
Ala Arg Glu Leu Tyr Leu Ser Ser Val Leu
1               5                   10

<210> 214
<211> 6
<212> PRT
<213> Oryctolagus

<220>
<221> SOURCE
<222> 1..6
<223> /mol_type="protein"
      /organism="Oryctolagus"

<400> 214
Thr Arg Gly Phe Asn Leu
1               5

<210> 215
<211> 12
<212> PRT
<213> Oryctolagus

<220>
<221> SOURCE
<222> 1..12
<223> /mol_type="protein"
      /organism="Oryctolagus"

<400> 215
Ala Arg Val Asp Phe Tyr Ser Tyr Val Tyr Ser Leu
1               5                   10

<210> 216
<211> 12
<212> PRT
<213> Oryctolagus

<220>
<221> SOURCE
<222> 1..12
<223> /mol_type="protein"
      /organism="Oryctolagus"

<400> 216
Ala Thr Val Asn Thr Tyr Thr Tyr Val Tyr Ser Leu
1               5                   10

<210> 217

<211> 12
<212> PRT
<213> Oryctolagus

<220>
<221> SOURCE
<222> 1..12
<223> /mol_type="protein"
      /organism="Oryctolagus"

<400> 217
Ala Ala Ile Asn Ile Tyr Thr Tyr Val Tyr Ser Leu
1               5                   10

<210> 218
<211> 12
<212> PRT
<213> Oryctolagus

<220>
<221> SOURCE
<222> 1..12
<223> /mol_type="protein"
      /organism="Oryctolagus"

<400> 218
Ala Thr Val Asn Ile Tyr Ser Tyr Val Tyr Ser Leu
1               5                   10

<210> 219
<211> 12
<212> PRT
<213> Oryctolagus

<220>
<221> SOURCE
<222> 1..12
<223> /mol_type="protein"
      /organism="Oryctolagus"

<400> 219
Ala Thr Val Asn Ile Tyr Thr Tyr Val Tyr Ser Leu
1               5                   10

<210> 220
<211> 12
<212> PRT
<213> Oryctolagus

<220>

<221> SOURCE
<222> 1..12
<223> /mol_type="protein"
      /organism="Oryctolagus"

<400> 220
Ala Arg Val Asn Ser Phe Ser Tyr Val Tyr Ser Leu
1               5                   10

<210> 221
<211> 12
<212> PRT
<213> Oryctolagus

<220>
<221> SOURCE
<222> 1..12
<223> /mol_type="protein"
      /organism="Oryctolagus"

<400> 221
Ala Arg Val Glu Phe Tyr Ser Tyr Val Tyr Val Leu
1               5                   10

<210> 222
<211> 14
<212> PRT
<213> Oryctolagus

<220>
<221> SOURCE
<222> 1..14
<223> /mol_type="protein"
      /organism="Oryctolagus"

<400> 222
Ala Arg Ile Asp Ala Ala Thr Ser Tyr Ser Ala Phe Asp Pro
1               5                   10

<210> 223
<211> 14
<212> PRT
<213> Oryctolagus

<220>
<221> SOURCE
<222> 1..14
<223> /mol_type="protein"
      /organism="Oryctolagus"

<400> 223
Ala Arg Asp Tyr Gly Gly Asp Ser Ala Thr Arg Leu Asn Leu
1               5                   10

<210> 224
<211> 14
<212> PRT
<213> Oryctolagus

<220>
<221> SOURCE
<222> 1..14
<223> /mol_type="protein"
      /organism="Oryctolagus"

<400> 224
Ala Arg Asp Tyr Gly Gly Asp Ser Ala Thr Arg Leu Asn Leu
1               5                   10

<210> 225
<211> 8
<212> PRT
<213> Oryctolagus

<220>
<221> SOURCE
<222> 1..8
<223> /mol_type="protein"
      /organism="Oryctolagus"

<400> 225
Lys Ser Val Tyr Tyr Asn Asn Trp
1               5

<210> 226
<211> 7
<212> PRT
<213> Oryctolagus

<220>
<221> SOURCE
<222> 1..7
<223> /mol_type="protein"
      /organism="Oryctolagus"

<400> 226
Gln Thr Ile Asn Asn Asn Asp
1               5

<210> 227

<211> 7
<212> PRT
<213> Oryctolagus

<220>
<221> SOURCE
<222> 1..7
<223> /mol_type="protein"
      /organism="Oryctolagus"

<400> 227
Gln Ser Ile Asn Asn Asn Asp
1               5

<210> 228
<211> 6
<212> PRT
<213> Oryctolagus

<220>
<221> SOURCE
<222> 1..6
<223> /mol_type="protein"
      /organism="Oryctolagus"

<400> 228
Gln Ser Ile Ser Ser Tyr
1               5

<210> 229
<211> 7
<212> PRT
<213> Oryctolagus

<220>
<221> SOURCE
<222> 1..7
<223> /mol_type="protein"
      /organism="Oryctolagus"

<400> 229
Gln Ser Ile Ser Ser Ser Tyr
1               5

<210> 230
<211> 6
<212> PRT
<213> Oryctolagus

<220>

<221> SOURCE
<222> 1..6
<223> /mol_type="protein"
      /organism="Oryctolagus"

<400> 230
Gln Ser Ile Ser Ser Tyr
1               5

<210> 231
<211> 6
<212> PRT
<213> Oryctolagus

<220>
<221> SOURCE
<222> 1..6
<223> /mol_type="protein"
      /organism="Oryctolagus"

<400> 231
Gln Ser Ile Ser Ser Cys
1               5

<210> 232
<211> 6
<212> PRT
<213> Oryctolagus

<220>
<221> SOURCE
<222> 1..6
<223> /mol_type="protein"
      /organism="Oryctolagus"

<400> 232
Gln Ser Ile Ser Ser Ser
1               5

<210> 233
<211> 6
<212> PRT
<213> Oryctolagus

<220>
<221> SOURCE
<222> 1..6
<223> /mol_type="protein"
      /organism="Oryctolagus"

<400> 233
Glu Ser Ile Tyr Ser Ser
1               5

<210> 234
<211> 6
<212> PRT
<213> Oryctolagus

<220>
<221> SOURCE
<222> 1..6
<223> /mol_type="protein"
      /organism="Oryctolagus"

<400> 234
Gln Ser Ile Ser Thr Ala
1               5

<210> 235
<211> 6
<212> PRT
<213> Oryctolagus

<220>
<221> SOURCE
<222> 1..6
<223> /mol_type="protein"
      /organism="Oryctolagus"

<400> 235
Gln Ser Ile Ser Thr Ala
1               5

<210> 236
<211> 6
<212> PRT
<213> Oryctolagus

<220>
<221> SOURCE
<222> 1..6
<223> /mol_type="protein"
      /organism="Oryctolagus"

<400> 236
Glu Asp Ile His Ser Ser
1               5

<210> 237

<211> 6
<212> PRT
<213> Oryctolagus

<220>
<221> SOURCE
<222> 1..6
<223> /mol_type="protein"
      /organism="Oryctolagus"

<400> 237
Gln Ser Ile Ser Gly Cys
1               5

<210> 238
<211> 8
<212> PRT
<213> Oryctolagus

<220>
<221> SOURCE
<222> 1..8
<223> /mol_type="protein"
      /organism="Oryctolagus"

<400> 238
Glu Ser Val Tyr Lys Asn Asn Tyr
1               5

<210> 239
<211> 6
<212> PRT
<213> Oryctolagus

<220>
<221> SOURCE
<222> 1..6
<223> /mol_type="protein"
      /organism="Oryctolagus"

<400> 239
Gln Ser Ile Gly Gly Tyr
1               5

<210> 240
<211> 6
<212> PRT
<213> Oryctolagus

<220>

<221> SOURCE
<222> 1..6
<223> /mol_type="protein"
      /organism="Oryctolagus"

<400> 240
Gln Ser Ile Gly Gly Tyr
1               5

<210> 241
<211> 4
<212> PRT
<213> Oryctolagus

<220>
<221> SOURCE
<222> 1..4
<223> /mol_type="protein"
      /organism="Oryctolagus"

<400> 241
Gly Ala Ser Thr
1

<210> 242
<211> 4
<212> PRT
<213> Oryctolagus

<220>
<221> SOURCE
<222> 1..4
<223> /mol_type="protein"
      /organism="Oryctolagus"

<400> 242
Glu Ala Ser Thr
1

<210> 243
<211> 4
<212> PRT
<213> Oryctolagus

<220>
<221> SOURCE
<222> 1..4
<223> /mol_type="protein"
      /organism="Oryctolagus"

<400> 243
Glu Ala Ser Thr
1

<210> 244
<211> 4
<212> PRT
<213> Oryctolagus

<220>
<221> SOURCE
<222> 1..4
<223> /mol_type="protein"
      /organism="Oryctolagus"

<400> 244
Asp Ala Ser Thr
1

<210> 245
<211> 4
<212> PRT
<213> Oryctolagus

<220>
<221> SOURCE
<222> 1..4
<223> /mol_type="protein"
      /organism="Oryctolagus"

<400> 245
Arg Ala Ser Thr
1

<210> 246
<211> 4
<212> PRT
<213> Oryctolagus

<220>
<221> SOURCE
<222> 1..4
<223> /mol_type="protein"
      /organism="Oryctolagus"

<400> 246
Lys Ala Ser Thr
1

<210> 247

<211> 4
<212> PRT
<213> Oryctolagus

<220>
<221> SOURCE
<222> 1..4
<223> /mol_type="protein"
      /organism="Oryctolagus"

<400> 247
Ser Ala Ser Thr
1

<210> 248
<211> 4
<212> PRT
<213> Oryctolagus

<220>
<221> SOURCE
<222> 1..4
<223> /mol_type="protein"
      /organism="Oryctolagus"

<400> 248
Leu Ala Ser Thr
1

<210> 249
<211> 4
<212> PRT
<213> Oryctolagus

<220>
<221> SOURCE
<222> 1..4
<223> /mol_type="protein"
      /organism="Oryctolagus"

<400> 249
Leu Ala Ser Thr
1

<210> 250
<211> 4
<212> PRT
<213> Oryctolagus

<220>

<221> SOURCE
<222> 1..4
<223> /mol_type="protein"
      /organism="Oryctolagus"

<400> 250
Leu Ala Ser Thr
1

<210> 251
<211> 4
<212> PRT
<213> Oryctolagus

<220>
<221> SOURCE
<222> 1..4
<223> /mol_type="protein"
      /organism="Oryctolagus"

<400> 251
Leu Ala Ser Thr
1

<210> 252
<211> 4
<212> PRT
<213> Oryctolagus

<220>
<221> SOURCE
<222> 1..4
<223> /mol_type="protein"
      /organism="Oryctolagus"

<400> 252
Leu Ala Ser Thr
1

<210> 253
<211> 4
<212> PRT
<213> Oryctolagus

<220>
<221> SOURCE
<222> 1..4
<223> /mol_type="protein"
      /organism="Oryctolagus"

<400> 253
Leu Ala Ser Thr
1

<210> 254
<211> 4
<212> PRT
<213> Oryctolagus

<220>
<221> SOURCE
<222> 1..4
<223> /mol_type="protein"
      /organism="Oryctolagus"

<400> 254
Leu Ala Ser Thr
1

<210> 255
<211> 4
<212> PRT
<213> Oryctolagus

<220>
<221> SOURCE
<222> 1..4
<223> /mol_type="protein"
      /organism="Oryctolagus"

<400> 255
Asp Ala Ser Asn
1

<210> 256
<211> 4
<212> PRT
<213> Oryctolagus

<220>
<221> SOURCE
<222> 1..4
<223> /mol_type="protein"
      /organism="Oryctolagus"

<400> 256
Asp Ala Ser Asn
1

<210> 257

<211> 12
<212> PRT
<213> Oryctolagus

<220>
<221> SOURCE
<222> 1..12
<223> /mol_type="protein"
      /organism="Oryctolagus"

<400> 257
Ala Gly Gly Tyr Thr Ser Ser Ser Asp Thr Val Ser
1               5                   10

<210> 258
<211> 12
<212> PRT
<213> Oryctolagus

<220>
<221> SOURCE
<222> 1..12
<223> /mol_type="protein"
      /organism="Oryctolagus"

<400> 258
Leu Gly Gly Tyr Tyr Ser Tyr Gly Trp Tyr Phe Gly
1               5                   10

<210> 259
<211> 12
<212> PRT
<213> Oryctolagus

<220>
<221> SOURCE
<222> 1..12
<223> /mol_type="protein"
      /organism="Oryctolagus"

<400> 259
Leu Gly Gly Tyr Tyr Ser Tyr Gly Trp Tyr Phe Gly
1               5                   10

<210> 260
<211> 15
<212> PRT
<213> Oryctolagus

<220>

<221> SOURCE
<222> 1..15
<223> /mol_type="protein"
      /organism="Oryctolagus"

<400> 260
Gln Cys Thr Tyr Gly Tyr Gly Ala Ser Ser Ala Tyr Gly Asn Ala
1               5                   10                  15

<210> 261
<211> 13
<212> PRT
<213> Oryctolagus

<220>
<221> SOURCE
<222> 1..13
<223> /mol_type="protein"
      /organism="Oryctolagus"

<400> 261
Gln Cys Thr Asp Tyr Ala Gly Ser Tyr Val Gly Gly Ala
1               5                   10

<210> 262
<211> 12
<212> PRT
<213> Oryctolagus

<220>
<221> SOURCE
<222> 1..12
<223> /mol_type="protein"
      /organism="Oryctolagus"

<400> 262
Gln Cys Ser Ala Gly Gly Arg Ser Gly Ile Gly Gly
1               5                   10

<210> 263
<211> 12
<212> PRT
<213> Oryctolagus

<220>
<221> SOURCE
<222> 1..12
<223> /mol_type="protein"
      /organism="Oryctolagus"

<400> 263
Gln Gln His Tyr Thr Ser Asn Asn Ile Asp Asn Ala
1               5                   10

<210> 264
<211> 12
<212> PRT
<213> Oryctolagus

<220>
<221> SOURCE
<222> 1..12
<223> /mol_type="protein"
      /organism="Oryctolagus"

<400> 264
Gln Gln His Tyr Thr Ala Leu Asn Val Asp Asn Ala
1               5                   10

<210> 265
<211> 12
<212> PRT
<213> Oryctolagus

<220>
<221> SOURCE
<222> 1..12
<223> /mol_type="protein"
      /organism="Oryctolagus"

<400> 265
Gln Gln His Tyr Ser Gly Ser Asn Val Asp Asn Ala
1               5                   10

<210> 266
<211> 12
<212> PRT
<213> Oryctolagus

<220>
<221> SOURCE
<222> 1..12
<223> /mol_type="protein"
      /organism="Oryctolagus"

<400> 266
Gln Gln His Tyr Ser Ser Asn Asn Val Asp Asn Ala
1               5                   10

<210> 267

<211> 12
<212> PRT
<213> Oryctolagus

<220>
<221> SOURCE
<222> 1..12
<223> /mol_type="protein"
      /organism="Oryctolagus"

<400> 267
Gln Gln His Tyr Ser Gly Asn Asn Val Asp Asn Ala
1               5                   10

<210> 268
<211> 12
<212> PRT
<213> Oryctolagus

<220>
<221> SOURCE
<222> 1..12
<223> /mol_type="protein"
      /organism="Oryctolagus"

<400> 268
Gln Gln His His Ser Thr Ser Gly Val Asp Asn Ala
1               5                   10

<210> 269
<211> 12
<212> PRT
<213> Oryctolagus

<220>
<221> SOURCE
<222> 1..12
<223> /mol_type="protein"
      /organism="Oryctolagus"

<400> 269
Gln Gln His Tyr Ser Ser Asn Asn Val Asp Asn Ala
1               5                   10

<210> 270
<211> 12
<212> PRT
<213> Oryctolagus

<220>

<221> SOURCE
<222> 1..12
<223> /mol_type="protein"
      /organism="Oryctolagus"

<400> 270
Glu Ala Thr Tyr Val Gly Val Asp Trp Tyr Trp Ala
1               5                   10

<210> 271
<211> 15
<212> PRT
<213> Oryctolagus

<220>
<221> SOURCE
<222> 1..15
<223> /mol_type="protein"
      /organism="Oryctolagus"

<400> 271
Gln Cys Thr Tyr Gly Tyr Gly Ile Ser Ser Ser Tyr Gly Asn Ala
1               5                   10                  15

<210> 272
<211> 15
<212> PRT
<213> Oryctolagus

<220>
<221> SOURCE
<222> 1..15
<223> /mol_type="protein"
      /organism="Oryctolagus"

<400> 272
Gln Cys Thr Tyr Gly Tyr Gly Ile Ser Ser Ser Tyr Gly Asn Ala
1               5                   10                  15

<210> 273
<211> 10
<212> PRT
<213> Oryctolagus

<220>
<221> SOURCE
<222> 1..10
<223> /mol_type="protein"
      /organism="Oryctolagus"

<400> 273
Phe Gly Gly Gly Thr Glu Val Val Val Lys
1               5                   10

<210> 274
<211> 10
<212> PRT
<213> Oryctolagus

<220>
<221> SOURCE
<222> 1..10
<223> /mol_type="protein"
      /organism="Oryctolagus"

<400> 274
Phe Gly Gly Gly Thr Glu Met Val Val Lys
1               5                   10

<210> 275
<211> 10
<212> PRT
<213> Oryctolagus

<220>
<221> SOURCE
<222> 1..10
<223> /mol_type="protein"
      /organism="Oryctolagus"

<400> 275
Phe Gly Gly Gly Thr Glu Val Leu Val Arg
1               5                   10

<210> 276
<211> 10
<212> PRT
<213> Oryctolagus

<220>
<221> SOURCE
<222> 1..10
<223> /mol_type="protein"
      /organism="Oryctolagus"

<400> 276
Phe Gly Ala Gly Thr Lys Val Glu Ile Lys
1               5                   10

<210> 277

<211> 10
<212> PRT
<213> Oryctolagus

<220>
<221> SOURCE
<222> 1..10
<223> /mol_type="protein"
      /organism="Oryctolagus"

<400> 277
Phe Gly Gly Gly Thr Glu Val Val Val Arg
1               5                   10

<210> 278
<211> 11
<212> PRT
<213> Oryctolagus

<220>
<221> SOURCE
<222> 1..11
<223> /mol_type="protein"
      /organism="Oryctolagus"

<400> 278
Trp Gly Gln Gly Thr Leu Val Thr Val Ser Ser
1               5                   10

<210> 279
<211> 11
<212> PRT
<213> Oryctolagus

<220>
<221> SOURCE
<222> 1..11
<223> /mol_type="protein"
      /organism="Oryctolagus"

<400> 279
Trp Gly Pro Gly Thr Leu Val Thr Val Ser Ser
1               5                   10

<210> 280
<211> 11
<212> PRT
<213> Oryctolagus

<220>

<221> SOURCE
<222> 1..11
<223> /mol_type="protein"
      /organism="Oryctolagus"

<400> 280
Trp Gly Pro Gly Thr Leu Val Thr Val Ser Ala
1               5                   10